(19) **Europäisches Patentamt / European Patent Office / Office européen des brevets**

(11) **EP 1 471 443 B1**

(12) # EUROPEAN PATENT SPECIFICATION

(45) Date of publication and mention
of the grant of the patent:
**23.11.2011 Bulletin 2011/47**

(51) Int Cl.:
**G06F 17/30** (2006.01)     **C07K 1/00** (2006.01)

(21) Application number: **03700474.4**

(22) Date of filing: **08.01.2003**

(86) International application number:
**PCT/JP2003/000057**

(87) International publication number:
**WO 2003/060765 (24.07.2003 Gazette 2003/30)**

(54) **METHOD OF CONSTRUCTING STEREOSTRUCTURE OF PROTEIN HAVING PLURAL NUMBER OF CHAINS**

VERFAHREN ZUM KONSTRUIEREN DER STEREOSTRUKTUR EINES PROTEINS MIT MEHREREN KETTEN

METHODE DE CONSTRUCTION DE LA STEREOSTRUCTURE D'UNE PROTEINE A PLUSIEURS CHAINES

(84) Designated Contracting States:
**AT BE BG CH CY CZ DE DK EE ES FI FR GB GR HU IE IT LI LU MC NL PT SE SI SK TR**

(30) Priority: **09.01.2002   JP 2002002859**

(43) Date of publication of application:
**27.10.2004   Bulletin 2004/44**

(73) Proprietor: **Umeyama, Hideaki
Urayasu-shi,
Chiba 279-0011 (JP)**

(72) Inventors:
• **UMEYAMA, Hideaki
Urayasu-shi, Chiba 279-0011 (JP)**
• **IWADATE, Mitsuo
Hanyu-shi, Saitama 348-0062 (JP)**
• **SUZUKI, Eiichiro
Sagamihara-shi, Kanagawa 228-0827 (JP)**

(74) Representative: **Clegg, Richard Ian
Mewburn Ellis LLP
33 Gutter Lane
London
EC2V 8AS (GB)**

(56) References cited:
**WO-A-01/35316     WO-A-99/18440
US-A- 5 884 230**

• **YONEDA TERUYO ET AL: "A computer modeling study of the interaction between tissue factor pathway inhibitor and blood coagulation factor Xa" JOURNAL OF PROTEIN CHEMISTRY, vol. 16, no. 6, 1997, pages 597-605, XP009051993 ISSN: 0277-8033**
• **KAWABATA TAKESHI ET AL: "GTOP: A database of protein structures predicted from genome sequences" NUCLEIC ACIDS RESEARCH, vol. 30, no. 1, 1 January 2002 (2002-01-01), pages 294-298, XP002339685 ISSN: 0305-1048**
• **OGATA K UMEYAMA H: "An automatic homology modeling method consisting of database searches and simulated annealing" JOURNAL OF MOLECULAR GRAPHICS AND MODELLING, ELSEVIER SCIENCE, NEW YORK, NY, US, vol. 18, 18 June 2000 (2000-06-18), pages 258-272, XP002956301 ISSN: 1093-3263**
• **KIHARA DAISUKE ET AL: "TOUCHSTONE: An ab initio protein structure prediction method that uses threading-based tertiary restraints" PROCEEDINGS OF THE NATIONAL ACADEMY OF SCIENCES OF THE UNITED STATES OF AMERICA, vol. 98, no. 18, 28 August 2001 (2001-08-28), pages 10125-10130, XP002339686 ISSN: 0027-8424**
• **KOLINSKI A ET AL: "Generalized comparative modeling (GENECOMP): A combination of sequence comparison, threading, and lattice modeling for protein structure prediction and refinement" PROTEINS, vol. 44, no. 2, 1 August 2001 (2001-08-01), pages 133-149, XP002339687 ISSN: 0887-3585**

EP 1 471 443 B1

- **LABESSE G ET AL: "Incremental threading optimization (TITO) to help alignment and modelling of remote homologues" BIOINFORMATICS (OXFORD), vol. 14, no. 2, 1998, pages 206-211, XP002339688 ISSN: 1367-4803**
- **UMEYAMA ET AL.: 'Genome ikagaku to kiso kara no bioinformatics hito genome database, homology kensaku, SNP, proteome kaiseki kara shikkan kaiseki soyaku eno oyo made dai 5 sho informatics to soyaku 4. Tanpakushitsu rittai kozo no jido modeling' EXPERIMENTAL MEDICINE vol. 19, no. 11, 05 July 2001, pages 1470 - 1474, XP002963796**

**Description**

Technical Field

The present invention relates to a method of constructing a tertiary structure of a protein composed of plural chains, more specifically, to a method of predicting a tertiary structure of a protein composed of plural chains whose tertiary structure is not known. According to this method, a protein composed of plural chains is treated as a temporary single chain for simplification, thereby the protein structure can be predicted with consideration given to the interaction between the plural peptide chains constituting the protein. As a result, as will be described in the examples mentioned below, more highly reliable prediction of a tertiary structure of a protein can be performed than a conventional method.

In addition, the present invention relates to a tertiary structure model of a protein obtained by the method, an available database for the method, a database structure, a computer software program, a computer installed therewith, an interface and the like.

For example, when a stop signal of a delimiter is added to each C terminal residue of an amino acid sequence composed of plural chains, and a protein tertiary structure composed of three-dimensional coordinates of a main-chain and a side-chain is constructed, potential calculation can be performed with consideration given to the interaction of the amino acid residues between the protein chains by obtaining the C terminal residue number of each chain based on this stop signal. According to this method, a model excellent in packing of the side-chain can be constructed, therefore, the tertiary structure of a protein composed of plural chains can be predicted with higher reliability than by a conventional method. Addition of the delimiter is performed for handling the plural chains as a single chain by binding all the plural chains one another. Therefore, it is not necessary to add the delimiter to the C terminal end of the last binding chain when making it a single chain (the last terminal end when making it a single chain).

In the present invention, a chain which constitutes or can constitute a protein such as a polypeptide chain or a ligand, namely, a chain of the protein is referred to as merely "a protein chain" in some cases.

In the case where a chain (protein chain) other than a polypeptide chain exists in a protein, for example, a component which is one protein chain constituting plural chains is a low molecular weight ligand such as a peptide, various binding states can be created by mutating arbitrarily the amino acid sequences of the ligand.

As a potential parameter, by adding an arbitrary interatomic potential parameter to an interatomic parameter constituting amino acid residues, modification into an arbitrary ligand molecule can be performed. In addition, by fixing the amino acid sequences of the ligand and creating a data set in which the amino acid sequences of an environmental protein chain has been mutated diversely, various receptor models, which can bind to a specific ligand, can be constructed. Also, since the interaction between proteins composed of plural chains can be represented accurately, a model, in which a recognition region related to a function is described, can be constructed. By mutating the amino acid residues of the interaction region, a model, in which an increase or decrease in the function can be adjusted, can be constructed. In the case of a single chain, after it is divided into a domain or a module to assume it as plural chains, they are restored as a temporary single chain, thereby a highly accurate tertiary structure model can be attempted.

In the method of constructing a tertiary structure of a protein of the present invention, the basic backbone is to make the use of an comparative modeling method of proteins, in particular, a homology modeling method or a threading method. As a protein tertiary structure, the one whose three-dimensional coordinates have been determined by an X-ray crystallographic analysis or the like is used as a template to be referred to. In practical, unless a protein whose structure has been determined as plural chains is used as a template, accurate relative configuration of respective protein chains, particularly, respective polypeptide chains cannot be determined in many cases. In other words, the present invention is an comparative modeling method using a reference protein complex whose relative configuration is known. However, homology of amino acid sequences between a target protein, which is to be a subject of modeling, and a reference protein is not necessarily high, all the proteins which meet the predetermined requirement (E-value) described later can be used as a reference protein (threading method). In addition, if modeling is performed only with respect to, for example, the interaction interface, existence of an experimental structure which can be referred to a whole complex is not necessarily required.

Background Art

WO0135316 discloses computer-based methods for generating and using three-dimensional (3-D) structural models of target molecules and databases containing the models. The targets can be protein structural variants derived from genes containing polymorphisms. The models are generated using molecular modeling techniques and are used in structure-based drug design studies for identifying drugs that bind to particular structural variants in structure-based drug design studies, for designing allele-specific drugs and population-specific drugs and for predicting clinical responses in patients. Computer-based methods for predicting drug resistance or sensitivity via computational phenotyping are also provided. Databases containing protein structural variant models are also provided.

US5884230 discloses a method in a computer system for modeling a three-dimensional structure of a model protein is provided. In one embodiment, the modeling is based upon a three-dimensional structure of a template protein and an amino acid sequence alignment of the model protein and the template protein. For each amino acid in the model protein, when the template protein has an amino acid aligned with the amino acid of the model protein, the position of the backbone atom of the amino acid of the model protein is established based on the position of a topologically equivalent backbone atom in the aligned amino acid of the template protein.

JOURNAL OF PROTEIN CHEMISTRY, (YONEDA T; KOMOOKA H; UMEYAMA H) vol. 16, #6, 1997, pp. 597-605 discloses computer models of complexes of FXa with K1 or K2, which were made using a crystal structure of FXa. A tertiary structure of FXa using CHIMERA to assess the accuracy of a homology modeling method is disclosed. The isolated model structure of FXa allegedly agreed well with the crystal structure, but analyses of complexes of this structure with K1 or K2 apparently revealed that the models of complexes could not provide clear evidence of greater binding ability to K2 because of the positional difference of a few side chains interacting with the inhibitor.

NUCLEIC ACIDS RESEARCH, 20020101 OXFORD UNIVERSITY PRESS, SURREY, (KAWABATA T; ET AL) vol.30, #1, pp.294 - 298 discloses the construction of a Genomes TO Protein structures and functions (GTOP) database, containing protein fold predictions of a huge number of sequences.

PROCEEDINGS OF THE NATIONAL ACADEMY OF SCIENCES OF USA, NATIONAL ACADEMY OF SCIENCE, WASHINGTON, DC, US, (KIHARA D; ET AL), vol. 98, #18, pp. 10125 - 10130 discloses a threading-based method of secondary and tertiary restraint prediction has been developed and applied to ab initio folding for the prediction of protein structure from amino acid sequence.

PROTEINS: STRUCTURE, FUNCTION AND GENETICS, John Wiley & Sons, Inc, US, (KOLINSKI A; ET AL), vol. 44, #2, pp.133 - 149 discloses a generalized comparative modeling method, GENECOMP, for the refinement of threading models. Ab initio folding using a lattice protein model, SICHO, near the template provided by the new threading algorithm PROSPECTOR is employed.

Determination of genome sequences has been performed worldwide, and lots of amino acid sequences encoded by more than 70 types of genomes have been determined in the same way. In particular, complete genome sequence analysis of human and many other living species has been proceeding to construct a database of the sequence information (refer to Gerardo Jimenez-Sanchez, Nature 409, 853-855 (2001)). Although the function of a gene can be specified or predicted from the genome sequence to a certain degree, there are so many genes whose functions cannot be predicted from only the sequence information. For a gene, the protein obtained by translating the DNA sequence of the gene into an amino acid sequence actually plays a function. For elucidation of the function of a protein, determination of the tertiary structure is performed by an empirical method such as an X-ray crystallographic analysis or NMR, however, it generally needs works requiring considerable time and effort. Therefore, a protein with a known tertiary structure whose amino acid sequences show a high homology with those of a target protein is searched, and prediction of the function of the target protein is performed.

As a method of predicting the tertiary structure of a protein itself from the amino acid sequences of a protein with an unknown tertiary structure, a homology modeling method is generally used (refer to T. Yoneda, H. Komooka and H. Umeyama, J. Protein Chem., 16, 597-605, 1997. The whole content is included for reference in this description as a part). This is a computational scientific method primarily including the following 4 steps.

(1) When an amino acid sequence of an arbitrary target protein with an unknown tertiary structure (target sequence) is given, a reference protein having a similar sequence to the target sequence (reference sequence) is selected from a tertiary structure database such as PDB (Protein Data Bank) by searching a protein whose sequence is statistically significantly similar (homology search) to give a sequence alignment between the target sequence and the reference sequence.

For performing the database search and the alignment, computer software such as FASTA (refer to Pearson WR, Methods Enzymol, 266, 227-258, 1996), PSI-BLAST (refer to Schaffer AA, Wolf YI, Ponting CP, Koonin EV, Aravind L and Altschul SF, Bioinformatics, 12, 1000-1011, 1999) or LIBRA (refer to Ota, M. and Nishikawa, K., Protein Engineering, 10, 339-351, 1997) can be used. FASTA is a program for performing the matching of the sequences of 20 alphabetical characters which mean 20 natural amino acids. It has been reported that if a tertiary structure is constructed to a reference protein with a high homology (similarity of amino acid residues about 30% or more, which corresponds to a FASTA's E-value of about 0.01 or less), a highly reliable model can be constructed.

On the other hand, in PSI-BLAST, the matching of character sequences is performed in the same way. However, PSI-BLAST does not have information whether characters agree or not, but has a characteristic of optimizing an alignment by calculating the similarity of characters called a profile as a substitution matrix for each character sequence region of a homologous protein and further repeating the calculation. LIBRA is a program based on the 3D-1D method (also known as the threading method), and used for examining a known tertiary structure and searching a homologous sequence to a target sequence. Therefore, its search algorithm is apparently different from that of FASTA or PSI-BLAST. Accordingly, LIBRA can sometimes detect homology between sequences widely (though including also an error), unlike the case of FASTA or PSI-BLAST.

(2) When the alignment calculated by FASTA, PSI-BLAST, LIBRA or the like is used, the correspondence relationship between the target sequence and the homologous reference sequence is determined for each character sequence region. Based on this relationship, three-dimensional coordinates of amino acid residues of the target sequence are created from the three-dimensional coordinates of the reference protein.

(3) In the case where the corresponding amino acid residue in the target sequence side to the reference sequence does not exist (amino acid residue deletion), the coordinates of the amino acid residue at the position of the reference protein side are not used. On the contrary, in the case where the corresponding amino acid residue in the reference sequence side to the target sequence does not exist (amino acid residue insertion), the coordinates of the amino acid residue at the position of the target sequence is created by searching an appropriate one from the database of protein fragment coordinates which has been prepared beforehand (refer to, for example, K. Ogata and H. Umeyama, Journal of Molecular Graphics and Modeling 18, 258-272, 2000. The whole content is included for reference in this description as a part).

(4) In the construction of the protein coordinates according to the above-mentioned (2) and (3), an inappropriate gap, collision or strain may structurally occur between amino acid residues. Therefore, these unnatural structures are normalized by an energy minimization calculation or a molecular dynamics calculation (refer to M. Takeda-Shitaka, H. Umeyama, FEBS Letters, 425, 448-452, 1998. Thewhole content is included for reference in this description as a part).

In some modeling software, for smoothly eliminating the structural strains mentioned in (4), the calculation and search procedures mentioned in (2) to (4) are performed for all atoms of the protein by, for example, the simulated annealing (SA) method gradually, instead of simultaneously.

"SA method" is a method for finding the global minimum point of energy E without getting trapped in a local minimum point, when a new state x' is obtained by applying a perturbation to a system in a state x, by performing transition to a new state x' with a high probability if the energy value E(x') in the new state is lower than the energy value E(x) in the old state, and with a low probability if it is higher. More specifically, energy minimization by the SA method is performed first for an $\alpha$ carbon atom which constitutes a protein backbone, subsequently for main-chain atoms including the $\alpha$ carbon atom, finally for all the atoms of the protein including side-chain atoms. "molecular dynamics calculation" is a method for calculating a coordinate change in which potential energy E should decrease by representing the potential energy E of a system as a coordinate function and performing an energy minimization calculation by mainly steepest descent method, the conjugate gradient method or the like. "Monte Carlo method" is a calculation method of probability dependent energy optimization based on statistical mechanics.

As mentioned above, if an alignment to the target sequence mentioned in (2) is obtained, its tertiary structure can be predicted and constructed via the creation of three-dimensional coordinates (refer to above-mentioned K. Ogata and H. Umeyama, Journal of Molecular Graphics and Modeling 18, 258-272, 2000).

However, conventionally, when an arbitrary sequence to a protein composed of plural chains is given, one or more proteins with a high homology are selected for each chain independently from a tertiary structure database such as PDB, an alignment is given, and modeling is performed independently for each of them. Therefore, as will be described in the examples mentioned below, a tertiary structure in which a protein-protein interaction mode is fully reflected could not be obtained. In particular, in the case where a ligand binding region of a drug, an enzyme substrate or the like is composed of plural chains, the above drawback is critical. In the case where for a drug or an enzyme substrate, a property such as function efficiency of a protein that accepts such a ligand is changed, the same drawback occurs. Conventionally, as a method of eliminating this drawback, there is a method of improving the drawback by presuming an interaction mode among plural chains after constructing a model by a molecular dynamics method or the like. However, an enormous amount of calculation time and computing machine resources are needed for searching a global minimum. In addition, since molecular dynamics parameters, which should originally correspond to a multibody, correspond to a two-body, it is difficult to give the native tertiary structure. Therefore, there is a problem that the method is not suitable for a genome-wide industrial application.

Disclosure of the Invention

1. Problems to be solved by the present invention

The present inventors investigated a method of constructing the tertiary structure of a protein composed of plural chains by converting the plural chains to a single chain when an arbitrary sequence for the protein is given. On an experimental basis, a method of binding with the appropriate number of glycine (G) - oligomer chains for converting natural or conceptional plural chains to a single chain is known as a spontaneously conceived one (refer to JP-A-2002-112782). In the calculation, the method can be handled in the same way, however, this method cannot always be adopted. For example, in the case where the C terminal of the initial chain and/or the N terminal of the subsequent chain is located at the complex interface, glycine-oligomer cannot be inserted. A complex can be formed in the same method as the distance geometry method based on the distance information of NMR. However, this method requires a lot of input data, thereby the preparation of calculation is complicated. Accordingly, a simple method of constructing a structure has been awaited.

It is an object that the present invention , with taking the above-mentioned situation into consideration, to provide a method of constructing a tertiary structure of an arbitrary protein composed of plural chains, which serves as a particularly important key factor in developing drugs or the like, highly accurately and much more efficiently than by a conventional method. Another object of the present invention is to provide a method of performing various modifications of a ligand molecule or a modification of a protein such as a receptor promptly and efficiently. Another object of the present invention is to specify the cause of an genetic disease and to promote development of drugs related to the disease by constructing a protein model composed of plural chains to elucidate a protein-protein interaction mode and to clarify the recognition mechanism of the interaction.

2. Means for Solving the Problems

The present invention provides a method of constructing a tertiary structure of a protein composed of plural chains as set forth in the appended claims. The present invention also provides a computer program for executing the method when run on a computer as set out in the appended claims.

The present inventors repeatedly investigated to obtain an appropriate tertiary structure model when arbitrary amino acid sequences of a protein composed of plural chains are given, and as a result, found or developed the following (1) to (10) methods and computer programs therefor.

(1) A method of fully automatically or manually constructing a tertiary structure model composed of three dimensional coordinates of main-chains and side-chains of a protein in the same way as a single chain in the case where an arbitrary amino acid sequence is given, by correcting an input file format of the plural chains in a computer program to present a form of a temporary single chain (a single chain) so as to extend an applicable range of a conventional comparative modeling method, for example, a homology modeling method from a protein composed of a single chain to a protein composed of plural chains (an extended modeling method), while taking that the protein is composed of plural chains into consideration in a potential formula, and a computer program therefor.

It is desirable that the number of chains constituting the target protein and the number of chains constituting the reference protein be the same. For example, in the case where the number of chains constituting the target protein is two, a reference protein having two chains can be adopted as a reference protein (candidate) selected for constructing the tertiary structure. Also, in the case where the number of chains constituting the target protein is three, a reference protein having three chains can be adopted as a reference protein (candidate) selected for constructing the tertiary structure. However, the numbers of chains of both (the reference protein and the target protein) are not necessarily the same, and the reference protein that includes a protein constructing the tertiary structure of the target protein or a portion (plural chains) thereof can be adopted as a reference protein.

A file format of sequences for a computer is corrected so as to regard the respective proteins composed of plural chains as a single chain (temporary single chain), however, there is no particular restriction on a method of making it a temporary single chain. For example, a method of correcting an amino acid sequence alignment between the target protein composed of plural chains and the reference protein to an alignment as a temporary single chain by inserting an identification mark at the boundary of each protein chain (e.g., polypeptide chain) is convenient and advantageous for performing the program. There is a method of being capable of converting plural chains to a temporary single chain other than this. For example, it is also feasible by a method of creating a separate file in which the boundary of each protein chain is registered sequentially with the residue number and sending it as a variable to a computer software program. Both are different only in the file format used for indicating the boundary of each protein chain, and the contents are exactly equivalent. In the same way, a method of using an identification number or the like as an internal variable indicating the boundary position or the boundary of each protein chain obtained from the result of performing sequence alignment procedure in a computer software program is also suitable. These methods can be, as a matter of course, used in the present invention. Therefore, these contents are included in the content of the above-mentioned development (content of the present invention).

In addition, by correcting the input file format of the plural chains so as to present a form of a temporary single chain, the input file format and an interaction between protein chains, which generally become more complicated with the increase in the number of chains, can be always described with the simplest input format and the clearest potential formula.

(2) In the above method, a method of correction to an sequence alignment having extensibility of the number of chains by selecting an amino acid sequence of the reference protein composed of plural chains based on a result of an alignment output preferably using FASTA, PSI-BLAST, LIBRA, FAMS, RPS-BLAST, IMPALA, ClustalW, HMMER, BIOCES or the like which is a variety of existing computer software on correction of the sequence alignment, adding a delimiter (e.g., "U" or the like) other than an amino acid symbol to next to the end (C terminal) of each amino acid sequence of the sequence alignment to take the boundary between respective chains consideration on modeling, and handling it as if it were a single chain. As mentioned above, addition of the delimiter is for enabling to handle plural chains as a single chain by binding all the chains one another. Therefore, addition of the delimiter is not needed for the C terminal end of the last binding chain when making it a single chain (the last terminal end when making it a single chain). Even if it is added, the delimiter of the last terminal end can be disregarded.

By preparing the sequence alignment having such a format, the upper limit of the chain number of a protein (currently up to 36 chains as a default) can be unlimitedly extended in theory (within the limits of the memory in a computer).

(3) In an extended modeling method such as an extended homology modeling, a method of constructing a model structure, by which construction and optimization of $C\alpha$ atomic coordinates are performed or can be performed sequentially or simultaneously for all chains by determining the C terminal residue number of each protein chain (e.g. polypeptide chain) from the sequence alignment corrected by the above-mentioned method (2) when performing potential calculation, disconnecting a chemical bond potential (potential energy arising from chemical bond length) and a chemical bond angle potential (potential energy arising from chemical bond angle) at the boundary and adding an interatomic interaction potential (energy) at the boundary, and a computer program therefor.

(4) In an extended modeling method such as an extended homology modeling, a method of constructing a model structure, by which construction and optimization of coordinates of main-chain atoms of N, $C\alpha$, C and O (carbonyl oxygen) and coordinates of side-chains of amino acid residues are performed or can be performed sequentially or simultaneously for all chains by determining the C terminal residue number of each protein chain (e.g. polypeptide chain) from the sequence alignment corrected by the above-mentioned method (2) when performing potential calculation, disconnecting a chemical bond potential, a chemical bond angle potential and a chemical bond torsional angle potential at the boundary of the main-chain of each protein and adding an interatomic interaction potential at the boundary, and a computer program therefor.

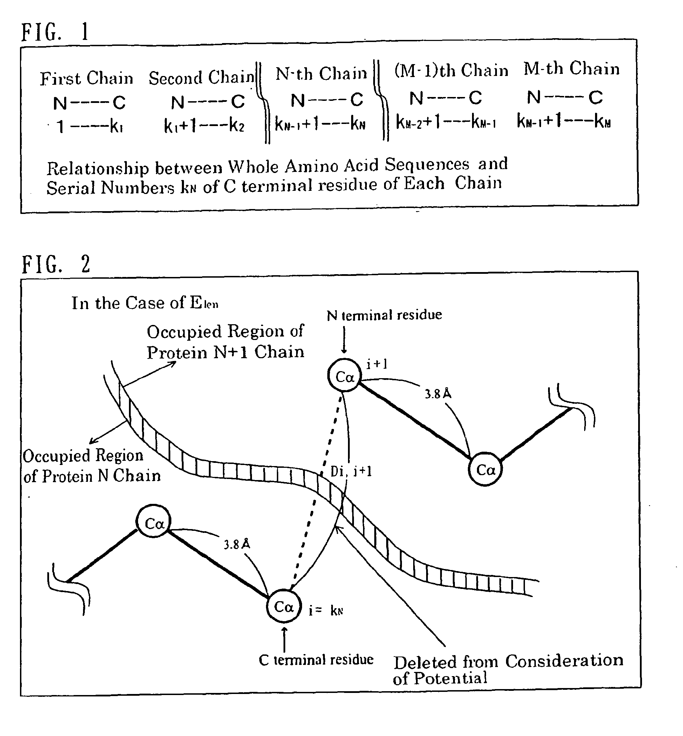

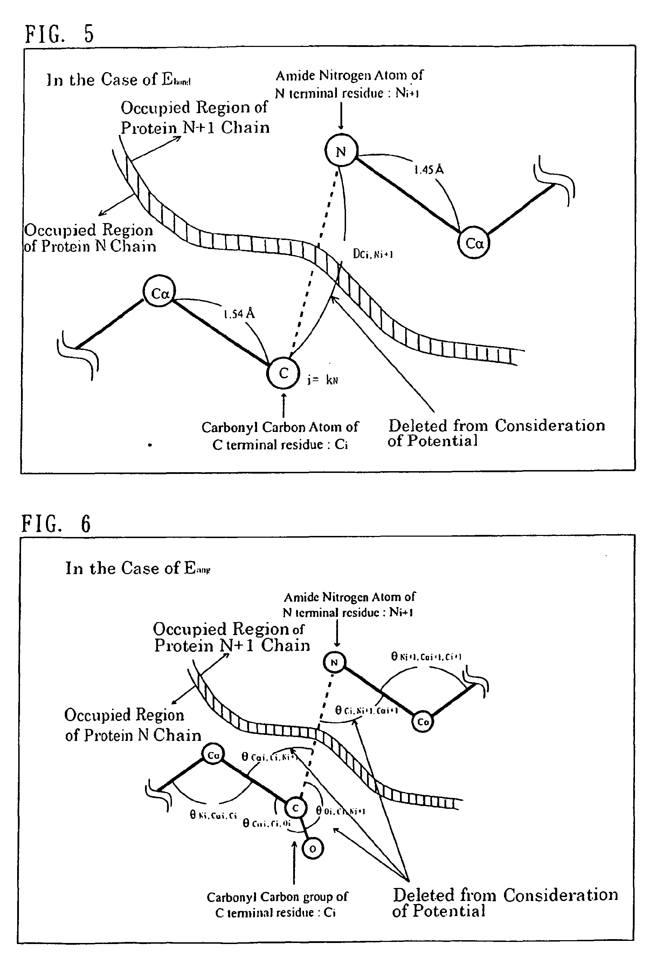

Fig. 1 shows the relationship between the whole amino acid sequences of the protein and the serial numbers (hereinafter referred to as "$k_N$". N indicates the number of protein chains) of the C terminal residues of each protein chain (e.g. polypeptide chain). Figs. 2 to 8 show specific descriptions of the above-mentioned methods (3) and (4). In each step of construction and optimization of $C\alpha$ atomic coordinates, as shown in Figs. 2 to 4, disconnection of the chemical bond potential and the chemical bond angle potential between the C terminal residue $k_N$ of the N-th protein chain and the N terminal residue $k_N+1$ of the (N+1)-th protein chain, and addition of the interatomic interaction potential are performed sequentially or simultaneously for all protein chains from N=1 to (the total number of chains)-1 (M-1). In each step of construction and optimization of main-chain atomic coordinates, as shown in Figs 5 to 8, disconnection of the chemical bond potential, the chemical bond angle potential, the chemical bond torsional angle potential between the C terminal residue $k_N$ of the N-th protein chain and the N terminal residue $k_N+1$ of the (N+1)-th protein chain, and addition of the interatomic interaction potential are performed sequentially or simultaneously for all protein chains from N=1 to (the total number of chains)-1 (M-1). Accordingly, validity of the model structure can be improved. In addition, simplification and improvement of efficiency of calculation steps can be realized.

(5) A method of constructing a protein-protein interaction model, when using an extended modeling method, in particular an extended homology modeling method or a threading method, by accurately determining atomic coordinates of an amino acid residue of a protein-protein interaction region.

(6) A method of constructing various peptide ligand models, which can bind to a target protein, when using an extended modeling method such as an extended homology modeling in the case where a certain protein component constituting plural chains includes a peptide ligand (including amino acid derivatives, peptide derivatives and the like), by creating a data set, in which an amino acid sequence of the ligand is mutated diversely.

(7) A method of constructing various ligand receptor (protein) models, which can bind to a specific ligand, when using an extended modeling method, in particular an extended homology modeling method, in the case where a component constituting plural chains includes a peptide ligand (including amino acid derivatives, peptide derivatives and the like), by fixing an amino acid sequence of the ligand and creating a data set, in which an amino acid sequence of an environmental protein chain is mutated diversely.

(8) A method of constructing a useful protein model, when using an extended modeling method, in particular, an extended homology modeling method, in the case where a component constituting plural chains is a common protein (chain), by creating a data set, in which an amino acid sequence of a recognition region of proteins is mutated diversely, and increasing or decreasing the function efficiency of the protein.

(9) A method of attempting a highly accurate tertiary structure model, in the case of a single chain, by dividing a protein into domains or modules to have it assumed as plural chains, and enabling to restore the plural chains to a temporary single chain by applying the above-mentioned (1) to (8).

(10) A computer software program, for enabling to browse and search the below-mentioned content for a database composed of the tertiary structure model of a protein, the ligand model and the ligand receptor (protein) model, which are constructed based on the above-mentioned (1) to (9) and a computer installed with the program.

The subjects to be browsed and searched are the following:

A) a gene identification code or a protein identification code of a target protein composed of plural chains, an about one-line function description, a target amino acid sequence and (coordinates of) three-dimensional structure constructed by the above-mentioned (1) to (9);

B) a gene identification code or a protein identification code of a reference protein, an about one-line function description, a reference amino acid sequence and (coordinates of) three-dimensional structure of the reference protein; and

C) a database structure in which an alignment result between a target sequence and a reference sequence, a homology value and an E-value are described and compiled.

Namely, the present invention, as an embodiment, lies in a method of constructing a tertiary structure of a protein composed of plural chains having given arbitrary amino acid sequences (target protein) by extending an comparative modeling method of constructing a tertiary structure of a protein composed of a single chain having a given arbitrary amino acid sequence (extended modeling method), the method comprising the steps of correcting an input file format of the plural chains in a computer software program so as to present a morphology as a temporary single chain (correction of sequence alignment) and constructing the tertiary structure based on the modeling method while assuming that the structure has plural chains in the calculation of a potential formula by the computer software program. The input file format as a temporary single chain and the handling it as plural chains in the potential formula of the present invention can be applied to an comparative modeling method other than FAMS, and also to a non-comparative modeling method.

As the comparative modeling methods, a homology modeling method (refer to K. Ogata and H. Umeyama, Journal of Molecular Graphics and Modeling 18, 258-272, 2000) and/or a threading method can be given.

In the above-mentioned methods, an objective tertiary structure can be constructed fully automatically or manually.

As a content of the correction in the above-mentioned method, selecting a sequence, which has a known structure and includes the same number of plural protein chains (such as polypeptide chains and ligands) as the target sequence, and handling it as a temporary single chain can be given. For example, in the case where the target protein is composed of polypeptide chains, it includes selecting an amino acid sequence of a reference protein composed of the same number of polypeptide chains, thereby handling the plural polypeptide chains (amino acid sequences) constituting the protein as a single chain (temporary single chain) in a form in which the N terminal end (head end) of one chain has bound to the C terminal end (tail end) of another chain sequentially. At this time, the plural polypeptide chains constituting the target sequence can also be handled as a single chain in a form in which they have bound sequentially in the same manner. For example, it is preferable that a protein can be handled as a protein of a single chain on, for example, a computer by adding a delimiter, preferably a delimiter other than amino acid symbols (e.g., a character "U"), to the C terminal end (tail end) of each amino acid sequence constituting the plural chains (correction of sequence alignment). In this case, the last chain among the plural chains binding sequentially, namely, when the protein is composed of N number of protein chains, the N-th protein chain does not need to bind to a chain furthermore, it is not necessary to add the delimiter to the C terminal end of the N-th protein chain (the last chain). Even if the delimiter is added to the last terminal end of the chain, it can be disregarded on a computer or the like. As mentioned above, by taking the boundaries between respective chains into consideration on modeling, and handling it as if it were a single chain, a sequence alignment having extensibility by the number of chains can be used.

Incidentally, there is no particular restriction on a protein chain constituting a target protein or a reference protein as long as it is recognized as a chain constituting a protein practically or it can constitute a protein. The typical examples include a polypeptide chain. "A polypeptide chain" may be a chain including primarily a polypeptide residue without being limited to a chain which is constituted only by a peptide bond (amide bond) with an amino acid and/or a derivative thereof (such as a salt or an ester derivative). Other than this, as a protein chain, a substance selected from amino acid derivatives (e.g., natural or non-natural amino acids and their derivatives), peptide derivatives, pharmaceutical substances, nucleic acids, saccharides, organic compounds such as organic metal compounds, metal oxides and their ions, and inorganic compounds such as metals and their ions is given. In such a case, this often exists or is selected as a ligand.

In the present invention, a protein used as a target protein or a reference protein is composed of plural chains. These plural protein chains include one or more polypeptide chains as mentioned above to form a protein. For example, a protein composed of only plural polypeptide chains, a protein including one or more polypeptide chains and a substance such as an amino acid derivative or a peptide derivative, which exists as, for example, a ligand (as mentioned above), as a chain can be exemplified.

In the case of a protein including plural polypeptide chains in the plural chains, these plural polypeptide chains may be heterochains or homochains. In other words, plural polypeptide chains whose amino acid sequences are exactly the same (homochain) or different from one another (heterochain) may be included.

The above-mentioned method includes a method of searching the reference protein from a tertiary structure database and performing sequence alignment between the amino acid sequence of the reference protein and the target sequence.

As a software which searches the reference protein and outputs an alignment, FAMS, FASTA, PSI-BLAST, LIBRA, RPS-BLAST, IMPALA, ClustalW, HMMER, BIOCES (refer to Protein Engineering, vol. 2, No. 5, pp347-351, 1989) or the like can be adopted as a preferred software.

In the above-mentioned method, it is preferable that the corrected sequence alignment be treatable to a multiple alignment, in which the amino acid sequence of the same type or a different type of the reference protein is written, by using a file having a format, in which an amino acid sequence of each protein chain (e.g., polypeptide chain) has a delimiter at the C terminal end of the amino acid sequence, and specifying a reference protein ID for each alignment section delimited by the delimiter, thereby enabling to show an average structure by superposing the sequences.

In the above-mentioned method, it is preferable that Cα atomic coordinates and main-chain atomic coordinates be constructed based on the tertiary structure database, preferably PDB or the like, and/or a database, which is obtained by modifying or sorting the tertiary structure database so as to avoid duplication of similar structures by determining the terminal residue number of each protein chain (e.g., polypeptide chain) from the temporary single chain after the correction, disconnecting a chemical bond angle potential at a border of the terminal residue, and adding an interatomic interaction potential at the border, and minimization (optimization) of an objective function representing a temporary energy value be performed by at least one of a simulated annealing method, a molecular dynamics calculation and a Monte Carlo method. As a result, construction and optimization of the Cα atomic coordinates are performed sequentially or simultaneously for all chains, thereby an objective model structure can be constructed.

For example, construction and optimization of coordinates of main-chain atoms of N, Cα, C and O (carbonyl oxygen) and coordinates of side-chains of amino acid residues can be performed sequentially or simultaneously for all chains by determining the number of the C terminal residue of each protein chain (e.g. polypeptide chain) from the sequence alignment corrected by the above-mentioned method when performing the potential calculation, disconnecting a chemical bond potential, a chemical bond angle potential and a chemical bond torsional angle potential at the boundary of the main-chain of the respective proteins and adding an interatomic interaction potential at the boundary. In addition, when using the modeling method, atomic coordinates of an amino acid residue of a protein-protein interaction region is determined accurately, and a protein-protein interaction model can be constructed.

In the above-mentioned method, it is preferable that in the case where at least two chains among the plural chains constituting the target protein are protein chains such as polypeptide chains, a data set of a modification whose similarity or the like is superior or inferior with the use of a potential energy value as an index be created based on a possible combination of 20 amino acids for each amino acid residue located at a mutual protein-protein recognition region, thereby enabling to construct a tertiary structure of the at least two protein chains whose functions as respective proteins are increased or decreased.

It is preferable that in the case where at least one chain among the plural chains constituting the target protein is an amino acid derivative, for example, a non-natural amino acid such as βAsp or γGlu or a derivative thereof, or a peptide derivative (a peptide ligand) (at least one chain of the plural chains is a polypeptide chain), and has a similar chemical structure to the corresponding ligand molecule in the reference protein, the alignment, in which the derivative of the target protein is defined as a new residue name and a one-character code and the ligand of the reference protein is defined as another new residue name and a one-character code, be created manually or automatically, and based on the possible combination of 20 amino acids and their derivatives for each residue constituting the ligand sequences, a rank be placed in the ascending order of the potential energy value, thereby enabling to construct a ligand model data set of the amino acid derivative or the peptide derivative, in which the several higher-ranked combinations are stored as modifications with superior similarity to a binding region of a receptor protein. By creating a data set in which the amino acid sequence of the ligand is mutated diversely, various peptide ligand models which can bind to the target protein can be constructed (refer to below-mentioned Example 3).

In the same way, it is preferable that in the case where the peptide ligand exists in the chain components of the plural chains constituting the target protein (at least one chain among the plural chains is a polypeptide chain), the amino acid sequence of the ligand be fixed, and based on the possible combination of 20 amino acids for each amino acid residue located at the region recognizing the ligand, a data set of a modification with superior similarity to a binding region of the plural higher-ranked receptor proteins be created with the use of the potential energy value as an index, thereby enabling to construct the tertiary structures of various ligand receptor proteins that can bind to the ligand. In this way, various receptor models that can bind to a specific ligand can be constructed.

In the above-mentioned method, the plural chains may be domains or modules into which a single chain polypeptide is divided, and can be restored to a single temporary chain. After assuming the protein as plural chains by dividing it into domains or modules, a highly accurate tertiary structure model can be attempted by restoring the protein to a temporary single chain with the use of the above-mentioned modeling method.

In the above-mentioned method, in the target protein (or the target sequence), a substance as a component of the plural chains (as a chain constituting the plural chains), which is neither a common amino acid nor a peptide, in which plural common amino acids are bound, and preferably, has been registered in the tertiary structure database (such as PDB) can be included. The examples include non-natural amino acids, pharmaceutical substances, nucleic acids, saccharides, organic compounds such as organic metal compounds, metal oxides and their ions, hetero components of inorganic compounds or the like such as metals and their ions. In the protein, even in the case where such a substance (e.g. as a ligand or the like) is included as at least one chain of the plural chains constituting the protein, one or more polypeptide chains are included to form the protein.

As the above-mentioned method, it can include steps of searching the reference protein which is appropriate for the target sequence from the tertiary structure database and performing the sequence alignment with the amino acid sequences of the searched plural reference proteins; selecting the amino acid sequence of the reference protein whose E-value is small for the target sequence; and adding the delimiter to the C terminal region (end) of the amino acid sequence of the chain included in the reference protein and also adding the delimiter to the corresponding position to the target sequence (correction of sequence alignment).

Addition of the delimiter as mentioned above is performed for binding all the plural chains one another to enable to handle the plural chains as a single chain. Therefore, it is not necessary to add the delimiter to the C terminal end of the last binding chain when making it a single chain (the last terminal end when making it a single chain). It is added in the same way, though it is not necessary, the added delimiter of the last terminal end can be disregarded.

In addition, a step of obtaining coordinates from the reference structure determined in the step of selecting the amino acid sequence of the reference protein for a $C\alpha$ atom which is one of the constitutive atoms in an amino acid of the target sequence based on the alignment information as mentioned above and optimizing the $C\alpha$ atomic coordinates used in the above-mentioned method of the invention (example: refer to claim 11); a step of adding main-chain atomic coordinates from the tertiary structure database to the obtained $C\alpha$ atomic coordinates and optimizing the main-chain atomic coordinates used in the above-mentioned method of the invention; and adding side-chain atomic coordinates from the tertiary structure database to the obtained main-chain atomic coordinates and optimizing the side-chain atomic coordinates used in the above-mentioned method of the invention can be included.

As the above-mentioned potential formula, the potential formulae shown in Table 1 (mentioned below) can be adopted. The preferred are as follows.

In a potential formula when the total chain number = M, wherein N represents the number of protein chains such as polypeptide chains and $k_N$ represents the serial number of the C terminal residue for the N-th protein chain (such as polypeptide chain), i=1, ..., M-1 is simplistically described as i=1, M-1,

(A) in the calculation for construction and optimization steps of the $C\alpha$ atomic coordinates, a case where $i=k_{N(N=1,M-1)}$ of a temporary chemical bond potential is not included and cases where $i=k_{N(N=1,M-1)}$, $i=k_{N(N=1,M-1)}+1$ of a temporary chemical bond angle potential are not included. Also, in the case of an interatomic interaction potential, j>i+1 is added if i= $k_N$-1, and j>i is added if i= $k_N$.

(B) In the calculation for construction and optimization steps of the main-chain atomic coordinates, a bond between Ci and Ni+1 when $i=k_{N(N=1,M-1)}$ is not included in a chemical bond potential, angles when $i=k_{N(N=1,M-1)}$ $C\alpha i$-Ci-$N_{i+1}$, Oi-Ci-$N_{i+1}$ and Ci-$N_{i+1}$-$C\alpha_{i+1}$ wherein C and O represent a carbon atom and an oxygen atom in a carbonyl respectively, $C\alpha$ represents an $\alpha$ carbon atom and N represents a nitrogen atom are not included in a chemical bond angle potential, further, angles Ni-$C\alpha i$-Ci-$N_{i+1}$, $C\alpha i$-Ci-$N_{i+1}$-$C\alpha_{i+1}$, and Ci-$N_{i+1}$-$C\alpha_{i+1}$-$C_{i+1}$ when $i=k_{N(N=1,M-1)}$ are not included in a chemical bond torsional potential. In addition, for an interatomic interaction potential, when the length between atoms is represented by r, a case of $r_{ij} \leq$ a specified value for $r_{ij} \in \{r_{Ni,Ni+1}; r_{C\alpha i,Ni+1}; r_{C\alpha i,C\alpha i+1}; r_{Ci,Ni+1}; r_{Ci,C\alpha i+1}; r_{Ci,C\beta i+1}; r_{Ci,Ci+1}; r_{Oi,Ni+1}; r_{Oi,C\alpha i+1}\}$ when $i=k_{N(N=1,M-1)}$ is added.

In other words, in the extended modeling method, when performing the potential calculation, by determining the C terminal residue number of each protein chain (such as polypeptide chain) from the sequence alignment corrected by the above-mentioned method, disconnecting the chemical bond potential, the chemical bond angle potential and the chemical bond torsional angle potential at the boundaries of the main-chains of the respective protein chains and adding the interatomic interaction potential at the boundaries, thereby construction and optimization of the coordinates of main-chain atoms of N, $C\alpha$, C and O (carbonyl oxygenatoms) and the coordinates of side-chains of the amino acid residues are performed sequentially or simultaneously for all chains, accordingly a model structure can be constructed.

Fig. 1 shows the relationship between the whole amino acid sequences of the protein and the serial number of the C terminal residues of each protein chain (such as polypeptide chain). The total chain number = M, each $k_N$ is distinguished by the delimiter U. N represents the number of protein chains (such as polypeptide chains).

Figs. 2 to 8 show specific descriptions of the correction method of the above-mentioned sequence alignment. As shown in Figs. 2 to 4, disconnection of the chemical bond potential and the chemical bond angle potential between the C terminal residue $k_N$ of the N-th protein chain and the N terminal residue $k_N+1$ of the (N+1)-th protein chain, and addition of the interatomic interaction potential are performed sequentially or simultaneously for all protein chains (such as polypeptide chains) from N=1 to (the total number of protein chains)-1 (M-1). In each step of construction and optimization of the main-chain atomic coordinates, as shown in Figs 5 to 8, disconnection of the chemical bond potential, the chemical bond angle potential, the chemical bond torsional angle potential between the C terminal residue $k_N$ of the N-th protein chain and the N terminal residue $k_N+1$ of the (N+1)-th protein chain, and addition of the interatomic interaction potential are performed sequentially or simultaneously for all protein chains from N=1
to (the total number of protein chains)-1 (M-1). Thereby, not only appropriateness of the model structure can be improved, but also simplification and improvement of efficiency of the calculation steps can be realized.

Although there is no particular restriction on the input file format, for example, as a general formula for the input file format in the case where modeling of the target protein from the reference protein is performed, the following content can be adopted:

The target protein ID is written after > in the first line. The amino acid sequences of the target protein having the delimiter (string) added next to the terminal residue of each protein chain (such as polypeptide chain) are written for all the protein chains in the second line without line feed. The reference protein ID is written after > in the third line. Amino acid sequences of the reference protein having the delimiter (string) added next to the terminal residue of each protein chain (such as polypeptide chain) are written for all the protein chains in the fourth line without line feed. As a way to arrange the amino acid sequences in the second line and the fourth line, it is preferable that the positions of the same order of the delimiter (string) be made to always conform in the second line and the fourth line with the use of the alignment obtained as mentioned above.

This is illustrated as follows:

> (the target protein ID);
(sequenceofthefirstchain) (delimiter) (sequence of the second chain) (delimiter) ... (delimiter) (sequence of the M-th chain);
> (the reference protein ID);
(sequenceofthefirstchain) (delimiter) (sequence of the second chain) (delimiter) ... (delimiter) (sequence of the M-th chain).

Incidentally, the above brackets are used for easily viewable, therefore, it is preferable that a consecutive character string be made without brackets in practice.

In the method of the present invention, as mentioned above, the method of correcting the amino acid sequence alignment between the target protein composed of the plural chains and the reference protein to an alignment as a dummy single chain by inserting an identification mark at the boundary of each protein chain (such as polypeptide chain) is convenient and advantageous for performing the program. However, there is a method of converting plural chains to a temporary single chain other than this. For example, it is also feasible by a method of creating a separate file in which the boundary of each protein chain is registered sequentially with the residue number and sending it as a variable to a computer software program. Both are different only in the file format used for indicating the boundary of each protein chain, and the contents are exactly equivalent. In the same way, a method of using an identification number or the like as an internal variable indicating the boundary position or the boundary of each protein chain (such as polypeptide chain) obtained from the result of performing the sequence alignment procedure in the computer software program is also exactly equivalent. These methods can be, as a matter of course, used as the present invention.

The present invention is a method of constructing a tertiary structure of a protein (target protein) composed of plural chains having given arbitrary amino acid sequences by extending an comparative modeling method of constructing a tertiary structure of a protein composed of a single chain having a given arbitrary amino acid sequence (extended modeling method) Also, the present invention lies in a method of constructing a tertiary structure of a protein composed of plural chains characterized by assuming, for the target protein and the selected reference protein, each amino acid sequence of the plural chains, which are included in the target protein and the selected reference protein respectively and correspond each other, as a single chain in a state where the N terminal ends and the C terminal ends are bound sequentially, performing sequence alignment between the reference sequence of the thus obtained temporary single chain and the target sequence of the thus obtained temporary single chain to confirm their correspondence relationship, locating a Cα atom, which is one of the constitutive atoms in an amino acid residue in the target sequence, binding the Cα atoms by amide bonds, further adding side-chains to construct coordinates for other constitutive atoms, performing optimization, and constructing the tertiary structure by the modeling method.

As the selected reference protein, the one whose amino acid sequence of the protein chain, such as a polypeptide chain is similar, in particular, statistically significantly similar to the target protein can be preferably selected.

In the method of constructing a tertiary structure of the present invention, construction of the tertiary structure of the target protein can be performed by obtaining coordinates from the tertiary structure of the reference protein selected for Cα atoms in a main-chain amino acid in the target protein based on the obtained alignment information, optimizing the Cα atomic coordinates so as to minimize an objective function, adding coordinates (including Cβ atomic coordinates) of other atoms of a main-chain to the optimized Cα atomic coordinates, optimizing the main-chain atomic coordinates so as to minimize the objective function, adding coordinates of other atoms of side-chains to the optimized main-chain atomic coordinates, and optimizing the side-chain atomic coordinates so as to minimize the objective function.

The present invention, as another embodiment, also lies in a tertiary structure model of a protein characterized in that it is constructed by the above-mentioned method of the present invention, and further lies in a program (newFAMS) characterized in that it includes any of the above-mentioned methods of the invention or a computer characterized in that it is installed with the program.

The present invention, as another embodiment, lies in a database which can be used in an extended modeling method characterized in that a data composed of the tertiary structure model of the protein constructed by the above-mentioned method of the present invention, the ligand model used in the above-mentioned method of the present invention and the tertiary structure model of the ligand receptor protein used in the same way is fixed and combined.

The present invention, as another embodiment, lies in a database characterized in that it is constructed from a data of the tertiary structure model of the protein constructed by the above-mentioned method of the present invention, the ligand model used in the above-mentioned method of the present invention and the tertiary structure model of the ligand receptor protein used in the same way so as to enable to browse or search the data by a computer.

The present invention, as another embodiment, lies in a database structure characterized in that the following content can be browsed or searched by a computer:

a gene identification code or a protein identification code of the target protein composed of plural chains, an about one-line function description, the target amino acid sequence and (coordinates of) three-dimensional tertiary structure of the target protein;

a gene identification code or a protein identification code of the reference protein, an about one-line function description, the reference aminoacid sequence and a (coordinate of) three-dimensional tertiary structure of the reference protein; and

an alignment result between the target sequence and the reference sequence, a homology value and an E-value.

The present invention, as another embodiment, lies in a computer software program characterized in that any of the above-mentioned content of the database can be browsed or searched, or the database structure can be used, or a computer installed with the program.

The present invention further lies in an interface characterized in that it is designed to enable to access an target protein, preferably to enable to simply and easily access an target protein without a preliminary knowledge, by conjunction search such as partial agreement of an arbitrary symbol specific to living species, a protein code name, a reference protein name, a character string of an about one-line function description for a protein, which is desired to be browsed, among the tertiary structure database constructed by any of the above-mentioned method of the present invention.

By the above-mentioned method, the atomic coordinates specifying the tertiary structure of the protein having the plural chains can be provided. In this description, Cα atom means a carbon atom, which is the center of the backbone of each amino acid residue. The Cα atom of each amino acid residue except for glycine has a property of optical activity. Cβ atom means a carbon atom, which binds to a side-chain side of the Cα atom. C atom means a carbon atom of a carbonyl group, which binds to the Cα atom.

In Table 1, N represents the number of protein chains such as polypeptide chains, $k_N$ represents the serial number of the C terminal residue in the N-th protein chain (such as polypeptide chain), and when the total chain number = M, i=1, ..., M-1 is represented by i=1, M-1. Fig. 1 shows the relationship between the whole amino acid sequences of the protein and $k_N$. For each potential formula in Table 1, specific illustrations for handling each protein chain (such as polypeptide chain) at the boundary are shown in Figs. 2 to 8, and will be detailed in the following paragraph.

[Table 1]

Calculation conditions of potential function at boundary of each protein chain

It is based on a conventional method (refer to K. Ogata and H. Umeyama, Journal of Molecular Graphics and Modeling 18, 258-272, 2000, or the like). In the method, a newly revised part in the extended modeling method for a protein of plural chains of the present invention with the content, which is not known nor suggested in a conventional single-chain modeling, is shown by "provided that:" (provisio) in the below-mentioned potential formulae. In other words, the conventional modeling method is used for a part other than the below-mentioned provisio. Therefore, the content of the provisio corresponds to the modified content, which can be newly adopted for a temporary single chain modeling used in the present invention. Unless otherwise explicitly stated, the meanings of the characters in the formulae all accord with those in the above-mentioned literature of the conventional method. The constants in the formulae can vary within a reasonable and appropriate range, and are not limited to the below-mentioned specific values. Here, the whole content of the literature of the conventional method is included for reference in this description as a part.

(A) Construction and optimization steps of Cα atomic coordinates
Calculation condition of chemical bond potential

$$E_{len} = K_l \sum_i (D_{i,i+1} - 3.8)^2$$

Provided that: $i \neq k_{N(N=1, M-1)}$
Calculation condition of chemical bond angle potential

$$E_{ang} = K_a \sum_i (\theta_i - \theta_0)^2$$

Provided that: $i \neq k_{N(N=1, M-1)}$, $i \neq k_{N(N=1, M-1)}+1$
Calculation condition of interatomic interaction potential

$$E_{vdw} = K_{vdw} \sum_{i,j(>i+2)} \left\{ \left( \frac{3.8}{D_{i,j}} \right)^{12} - 2\left( \frac{3.8}{D_{i,j}} \right)^6 \right\}$$

Provided that: $j>i+1$ if $i=k_N-1$, $j>i$ if $i=k_N$
Potential function in which calculation conditions of the conventional method can be used:
Calculation condition of S-S bond potential

$$E_{ss} = K_{ss} \sum_i (D_i^{SS} - 5.4)^2$$

Calculation condition of coordinate positional potential

$$E_{pos} = K_{pos} \sum_i \frac{1}{M_i} \| \mathbf{x}_i - \langle w_i \mathbf{x}_i \rangle \|^2$$

(B) Construction and optimization steps of main-chain atomic coordinates
Calculation condition of chemical bond potential

$$E_{bond} = K_b \sum_i (b_i - b_i^0)^2$$

Provided that: if $i=k_{N(N=1,M-1)}$ and $b_i=D_{Ci,Ni+1}$, $b_i-b_i^0=0$
Calculation condition of chemical bond angle potential

$$E_{ang} = K_a \sum_i (\theta_i - \theta_i^0)^2$$

Provided that:

if $i=k_{N(N=1,M-1)}$ and $\theta_i=\theta_{C\alpha i,Ci,Ni+1}$, $\theta_i-\theta_i^0=0$
If $i=k_{N(N=1,M-1)}$ and $\theta_i=\theta_{Oi,Ci,Ni+1}$, $\theta_i-\theta_i^0=0$
if $i=k_{N(N=1, M-1)}$ and $\theta_i=\theta_{Ci,Ni+1C\alpha i+1}$, $\theta_i-\theta_i^0=0$

<u>Calculation condition of chemical bond torsional angle potential</u>

$$E_{tor} = K_t \sum_i \sqrt{(\phi_i - \phi_i^0)^2 + (\psi_i - \psi_i^0)^2} + K_\omega \sum_i (\omega_i - \omega_i^0)^2$$

Provided that: if $i=k_{N(N=1,M-1)}$, $\varphi_i-\varphi_i^0=\omega_i-\omega_i^0=\phi_{i+1}-\phi_{i+1}^0=0$
<u>Calculation condition of interatomic interaction potential</u>

$$E_{non-bond} = K_{non} \sum_{i,j} \varepsilon_{i,j} \cdot \left\{ \left( \frac{r_{i,j}^*}{r_{i,j}} \right)^{12} - 2 \left( \frac{r_{i,j}^*}{r_{i,j}} \right)^6 \right\}$$

Provided that: when $i=k_{N(N=1,M-1)}$, if $r_{ij} \leq 8.0$ for $r_{ij} \in \{r_{Ni,Ni+1}; r_{C\alpha i,Ni+1}; r_{C\alpha i,C\alpha i+1}; r_{Ci,Ni+1}; r_{Ci,C\alpha i+1}; r_{Ci,C\beta i+1}; r_{Ci,Ci+1}; r_{Oi,Ni+1}; r_{Oi,C\alpha i+1}\}$, the case is included in the calculation of $E_{non-bond}$.
Potential function in which calculation conditions of the conventional method can be used:
<u>Calculation condition of S-S bond potential</u>

$$E_{SS} = \sum_i \left\{ K_{C\alpha}^{SS}(D_i^{C\alpha} - 5.4)^2 + K_{C\beta}^{SS}(D_i^{C\beta} - 3.8)^2 \right\}$$

<u>Calculation condition of coordinate positional potential</u>

$$E_{pos} = K_{pos} \sum_i \frac{1}{M_i} \| \mathbf{x}_i - \langle w_i \mathbf{x}_i \rangle \|^2$$

<u>Calculation condition of potential with respect to amino acid residue chirality</u>

$$E_{chi} = K_{chi} \sum_i \left( \tau_i - \frac{2}{3}\pi \right)^2$$

<u>Calculation condition of hydrogen bond potential between main-chains</u>

$$E_{hydr} = K_{hydr} \sum_{i,j} \left( D_{i,j}^{N-O} - 2.9 \right)^2$$

As mentioned above, in the method of the present invention, by correcting the input file format of the plural chains and the potential formulae so as to present a morphology as a temporary single chain respectively, the input file format and the interaction between protein chains (such as polypeptide chains), which generally become more complicated with the increase in the number of chains, can be always described with the simplest input format and the clearest potential formula.

Brief Description of the Drawings

[Fig. 1]

Fig. 1 shows the relationship between the whole amino acid sequences of a protein used for explaining the present invention and the serial number $k_N$ of the C terminal residues of each chain.
The total chain number = M, each $k_N$ is distinguished by a delimiter U. N represents the number of protein chains.

[Fig. 2]

Fig. 2 illustrates how to handle C and N terminals in the Elen term of the potential formula (limited to the case of $C\alpha$).

[Fig. 3]

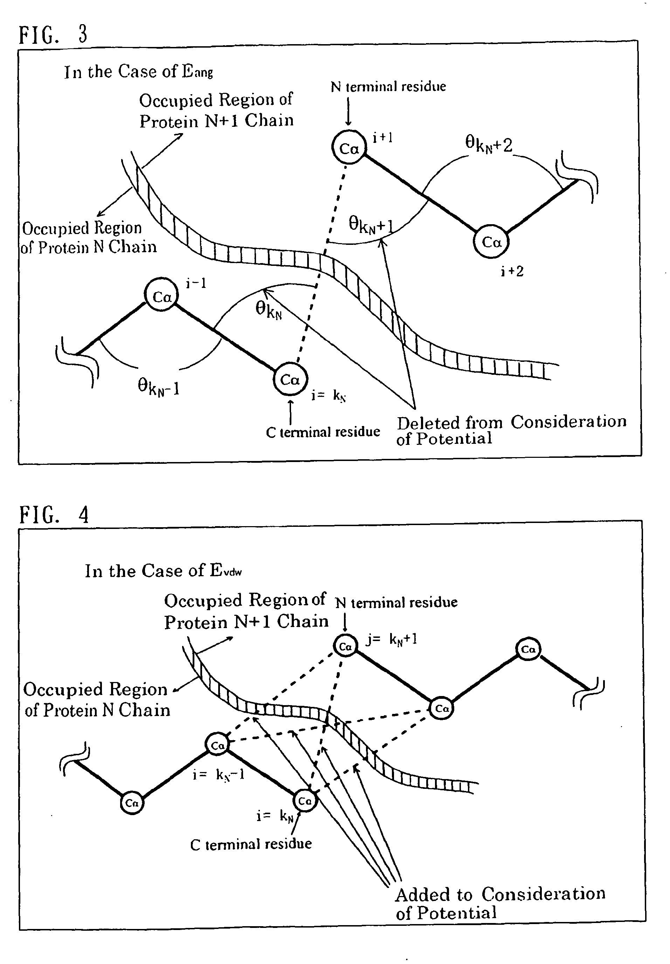

Fig. 3 illustrates how to handle C and N terminals in the Eang term of the potential formula (limited to the case of $C\alpha$).

[Fig. 4]

Fig. 4 illustrates how to handle C and N terminals in the Evdw term of the potential formula (limited to the case of $C\alpha$).

[Fig. 5]

Fig. 5 illustrates how to handle C and N terminals in the Ebond term of the potential formula.

[Fig. 6]

Fig. 6 illustrates how to handle C and N terminals in the Eang term of the potential formula.

[Fig. 7]

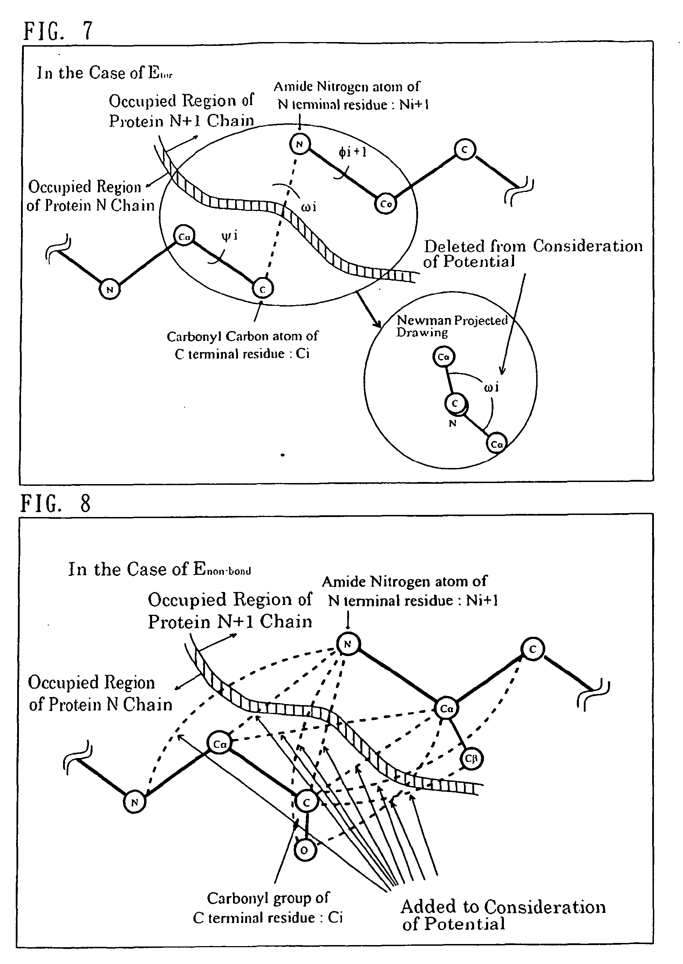

Fig. 7 illustrates how to handle C and N terminals in the Etor term of the potential formula.

[Fig. 8]

Fig. 8 illustrates how to handle C and N terminals in the Enon-bond term of the potential formula.

[Fig. 9]

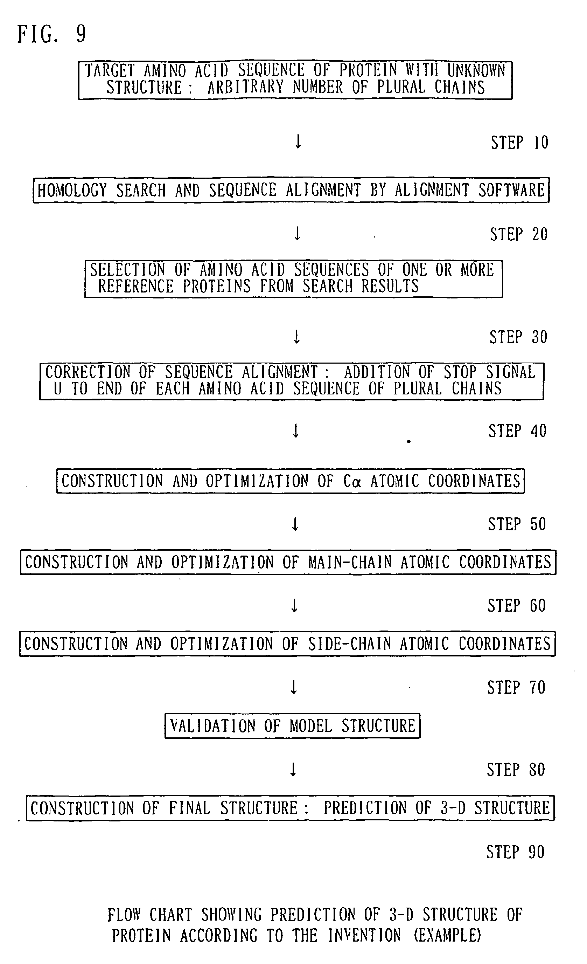

Fig. 9 is a flow chart showing an example of a method of constructing a tertiary structure of a protein composed of plural chains according to the present invention.

[Fig. 10]

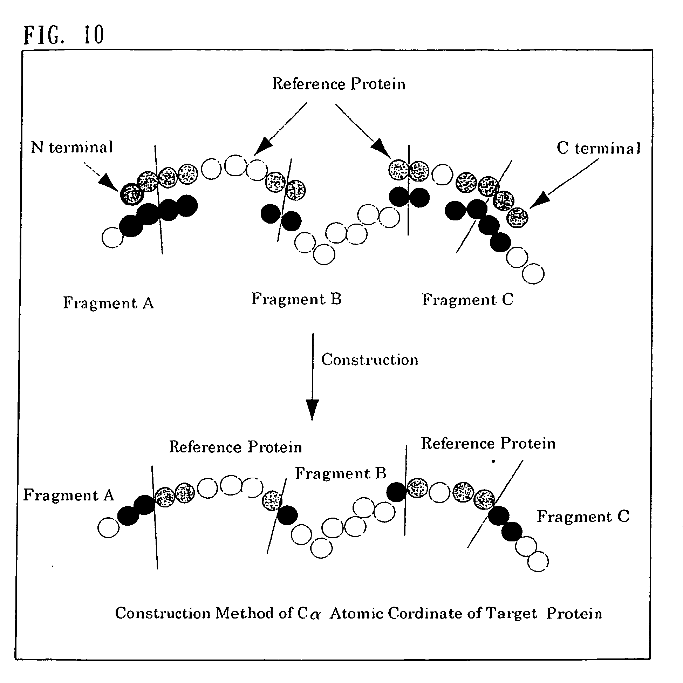

Fig. 10 illustrates an example of a method of constructing $C\alpha$ atomic coordinates of the target protein.
The matched portion of an alignment is obtained from the reference protein, and for the non-matched portion, a region having the least rmsd by superposition of two residues of N and C terminals is obtained from a database (refer to K. Ogata and H. Umeyama, Journal of Molecular Graphics and Modeling 18, 258-272, 2000).

[Fig. 11]

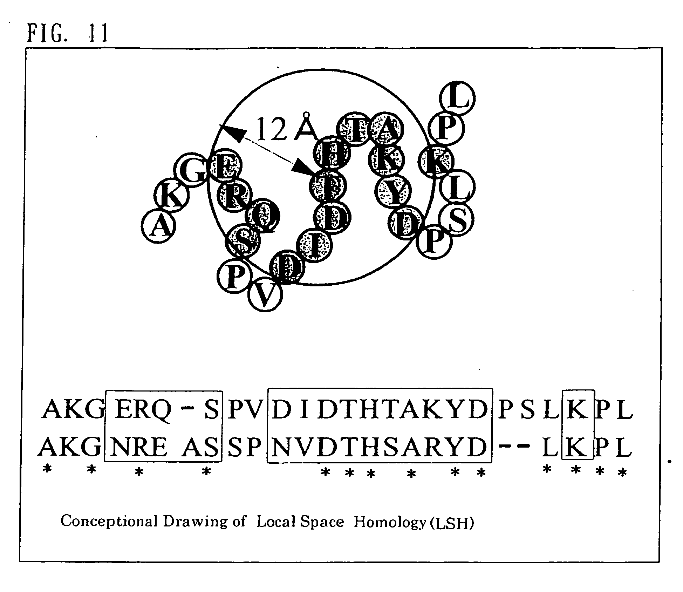

Fig. 11 illustrates the concept of local space homology (LSH).

For example, in the calculation with respect to the central T residues within the circle (sphere) of the figure, they are considered by the gray colored residues. The region enclosed by rectangles in the alignment is the residue pairs to be considered and the ratio of residues marked with asterisks is LSH (56.2% in this example) (refer to K. Ogata and H. Umeyama, Journal of Molecular Graphics and Modeling 18, 258-272, 2000).

[Fig. 12]

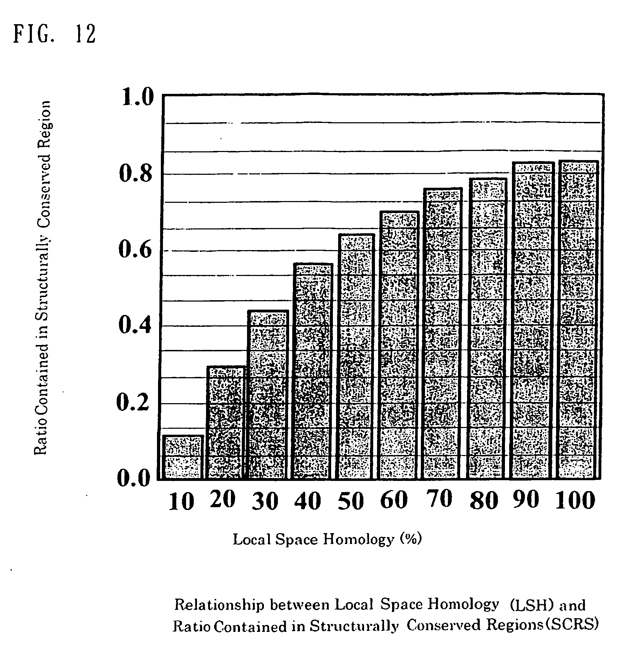

Fig. 12 illustrates the relationship between the local space homology (LSH) and the ratio contained in structurally conserved regions (SCRs).

LSH is calculated from the superposition of Cα atoms in the target protein and the reference protein, and the ratio contained in SCRs indicates the number of residues in SCRs for the total number of residues in the target protein (refer to K. Ogata and H. Umeyama, Journal of Molecular Graphics and Modeling 18, 258-272, 2000).

[Fig. 13]

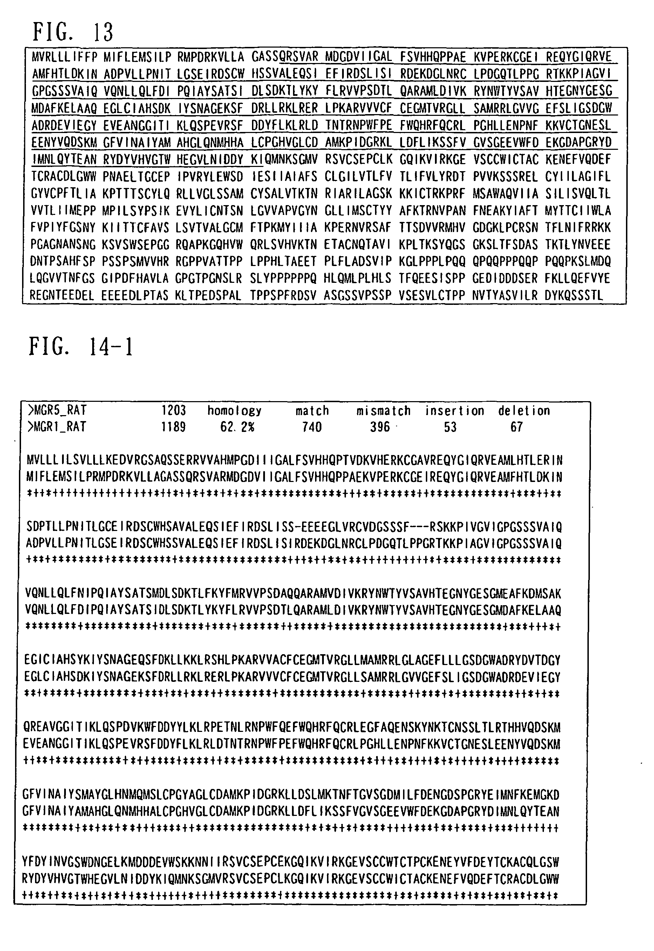

Fig. 13 shows the whole amino acid sequences of metabotropic glutamate receptor 1 derived from rat used in Example 1.

[Fig. 14]

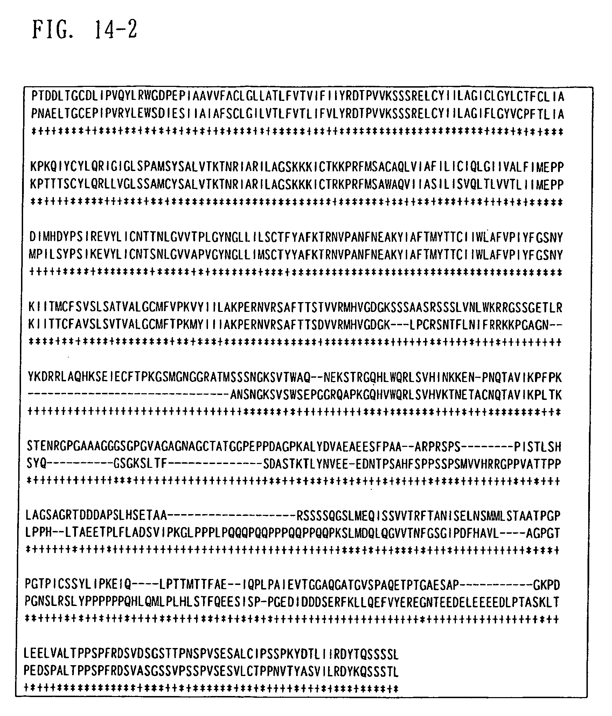

Fig. 14 is composed of Fig. 14-1 and Fig 14-2 and shows the alignment result of MGR1_RAT and MGR5_RAT in Example 1.

[Fig. 15]

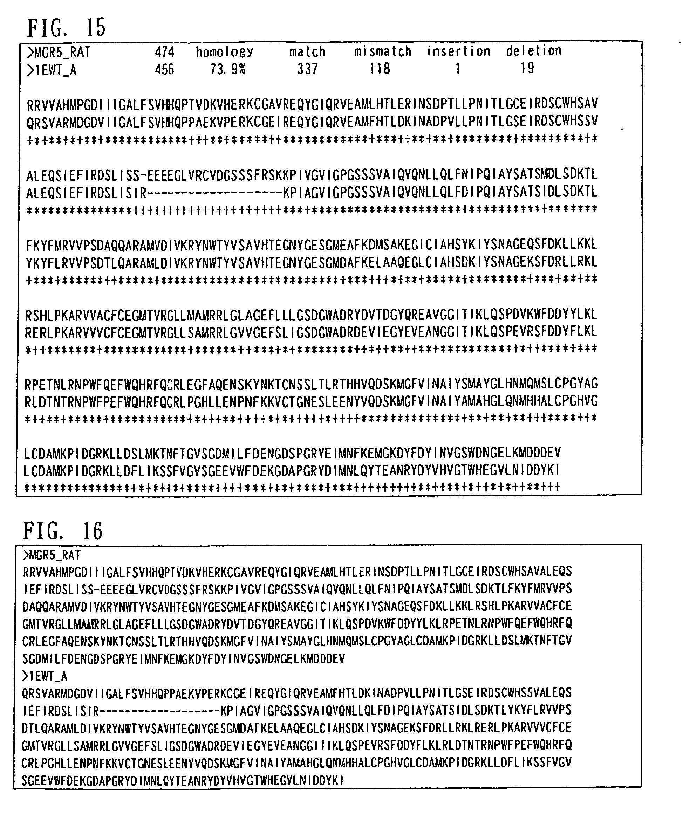

Fig. 15 shows the alignment result of MGR1_RAT and MGR5_RAT in Example 1.

Only portion, in which coordinates exist in 1EWT, is shown.

[Fig. 16]

Fig. 16 shows the input file format for the conventional FAMS in Example 1.

[Fig. 17]

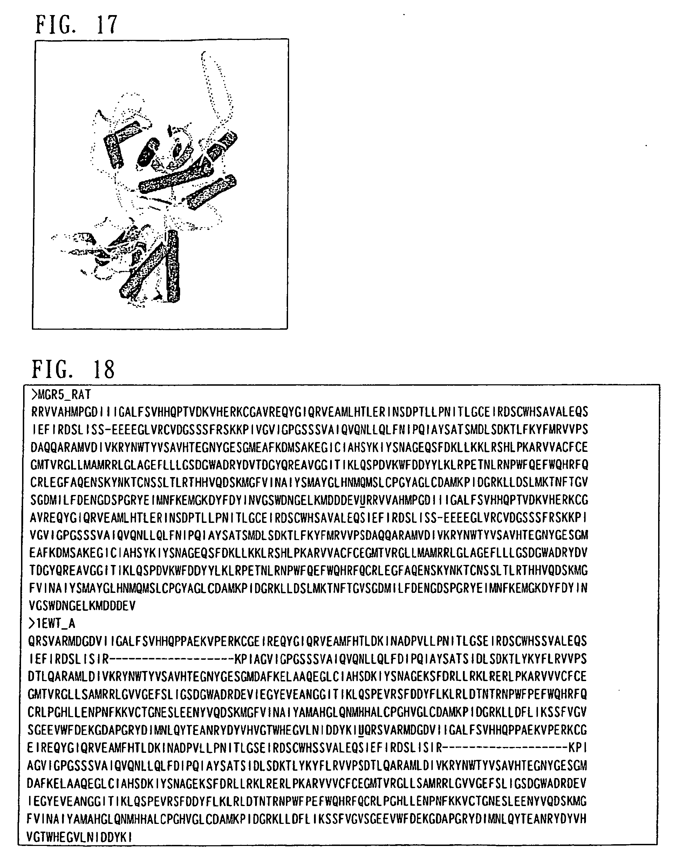

Fig. 17 shows a monomer model of mGlu receptor Swiss Prot [MGR5_RAT] in Example 1.

[Fig. 18]

Fig. 18 shows the input file format for newFAMS in Example 1.

[Fig. 19]

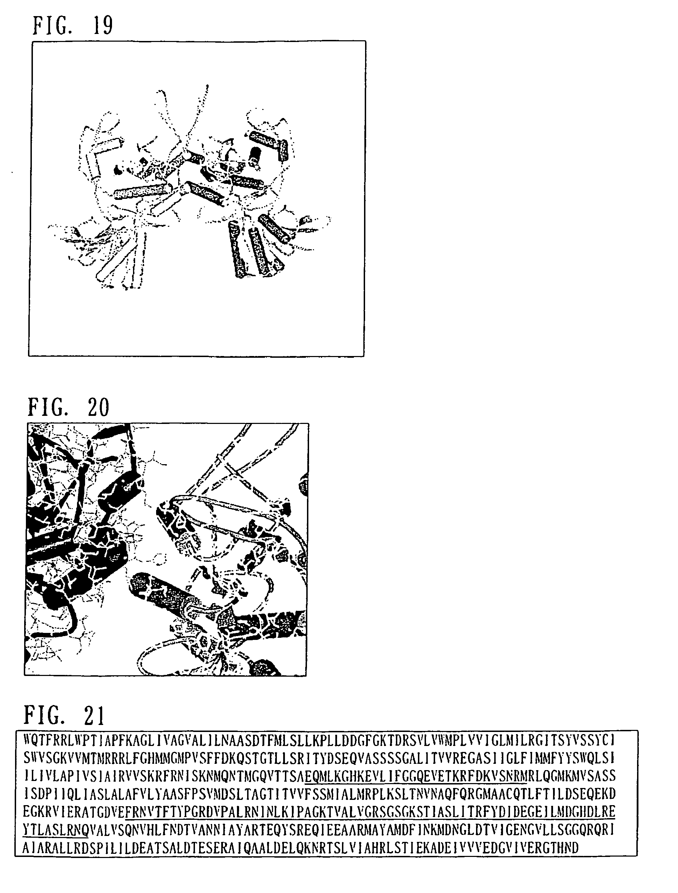

Fig. 19 shows a dimer model of mGlu receptor Swiss Prot [MGR5_RAT] in Example 1.

[Fig. 20]

Fig. 20 shows the enlarged drawing of the bound surface of the dimer model of mGlu receptor Swiss Prot [MGR5_RAT] in Example 1.

[Fig. 21]

Fig. 21 shows the amino acid sequences of 1JSQ monomer in Example 2.

[Fig. 22]

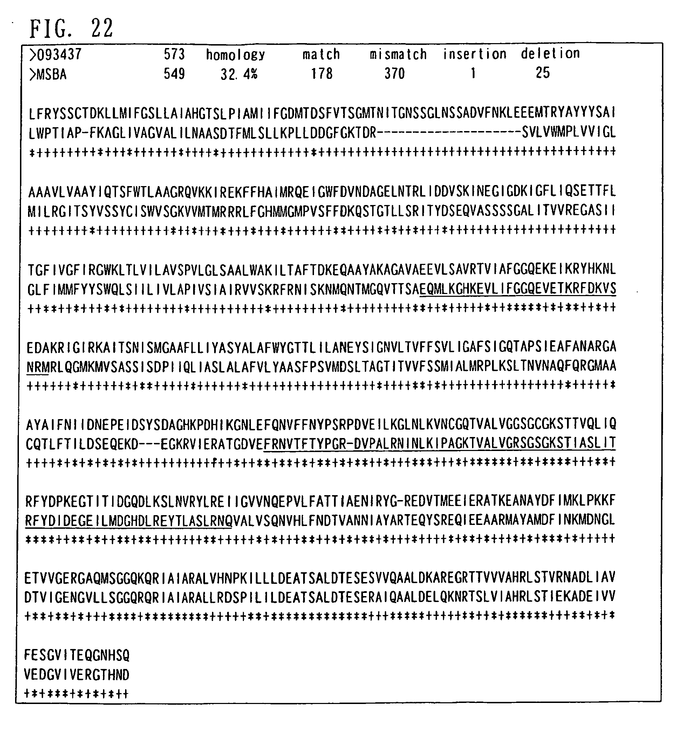

Fig. 22 shows the alignment result of [O93437] and MSBA in Example 2.

[Fig. 23]

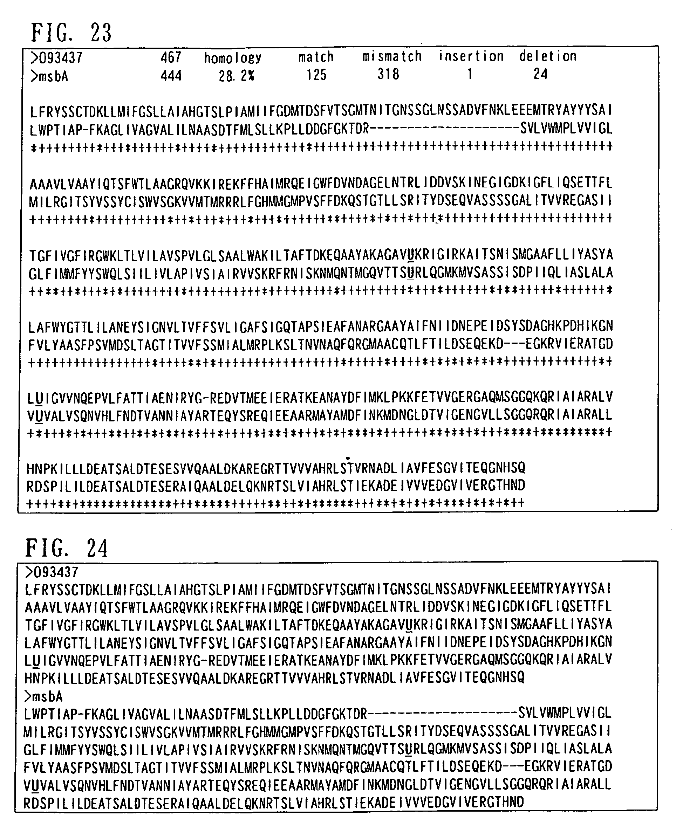

Fig. 23 shows the new alignment result of [O93437] and MSBA in Example 2.

[Fig. 24]

Fig. 24 shows the input file format of newFAMS for [O93437] in Example 2.

[Fig. 25]

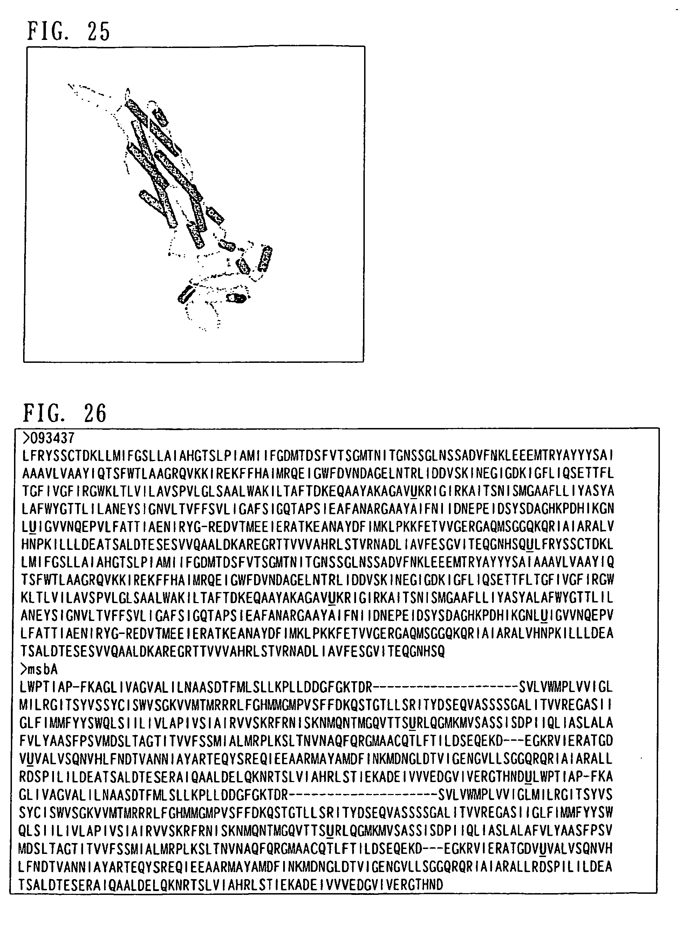

Fig. 25 shows a monomer model of ABC transporter SwissProt [O93437] in Example 2.

[Fig. 26]

Fig. 26 shows the input file format for a homodimer used in newFAMS in Example 2.

[Fig. 27]

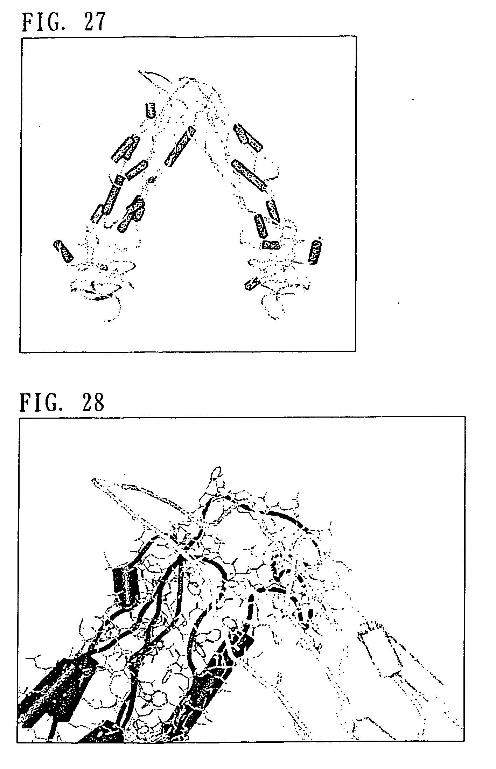

Fig. 27 shows a dimer model of ABC transporter SwissProt [O93437] in Example 2.

[Fig. 28]

Fig. 28 illustrates the bound surface of the dimer model of ABC transporter SwissProt [O93437] in Example 2.

[Fig. 29]

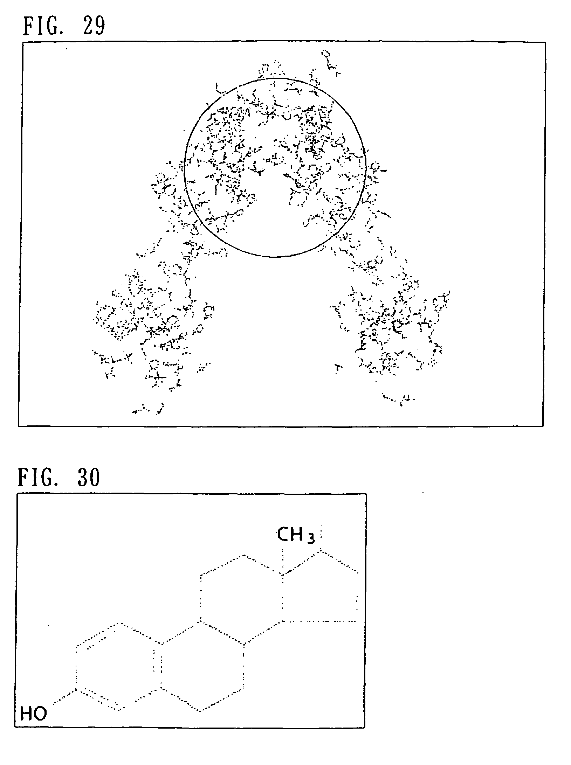

Fig. 29 illustrates the hydrophobic region of the ABC transporter model in Example 2.

[Fig. 30]

Fig. 30 shows the molecular structure of estradiol in Example 2.

[Fig. 31]

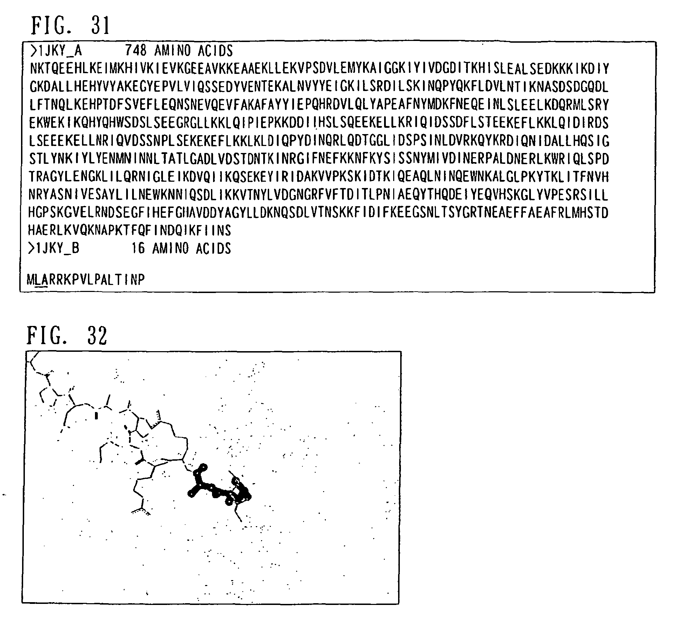

Fig. 31 shows the amino acid sequences of PDB: 1JKY in Example 3.
PDB: 1JKY contains A chain (LF) and B chain (16 residues of MAPKK).

[Fig. 32]

Fig. 32 illustrates MAPKK-2 in LF in Example 3.
In addition, ball and stick models are shown for Leu-2 and Ala-3.

[Fig. 33]

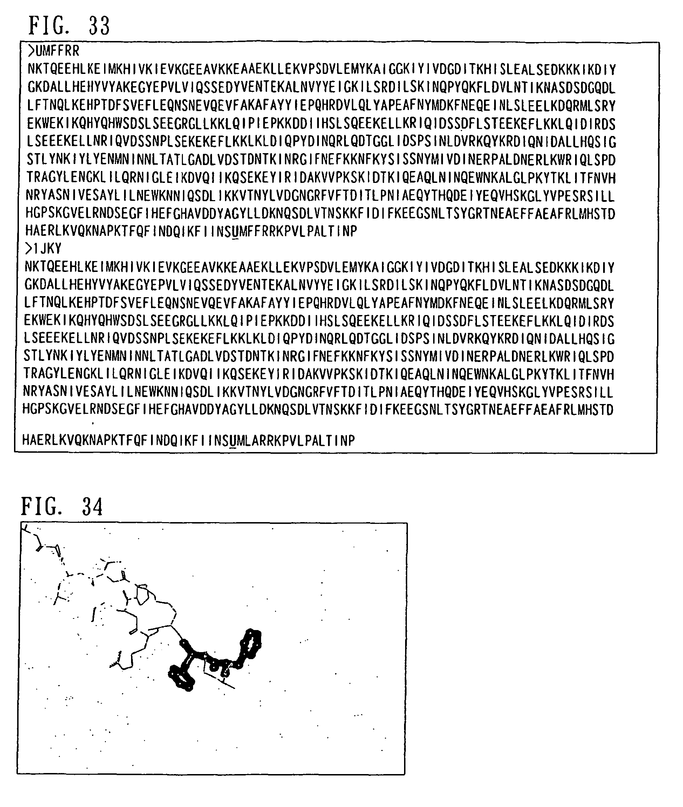

Fig. 33 shows the input file format of newFAMS in the peptide modification from [LA] to [FF] in Example 3.

[Fig. 34]

Fig. 34 illustrates a MAPKK-2 modification form in LF in Example 3.
In addition, ball and stick models are shown for Phe-2 and Phe-3.

[Fig. 35]

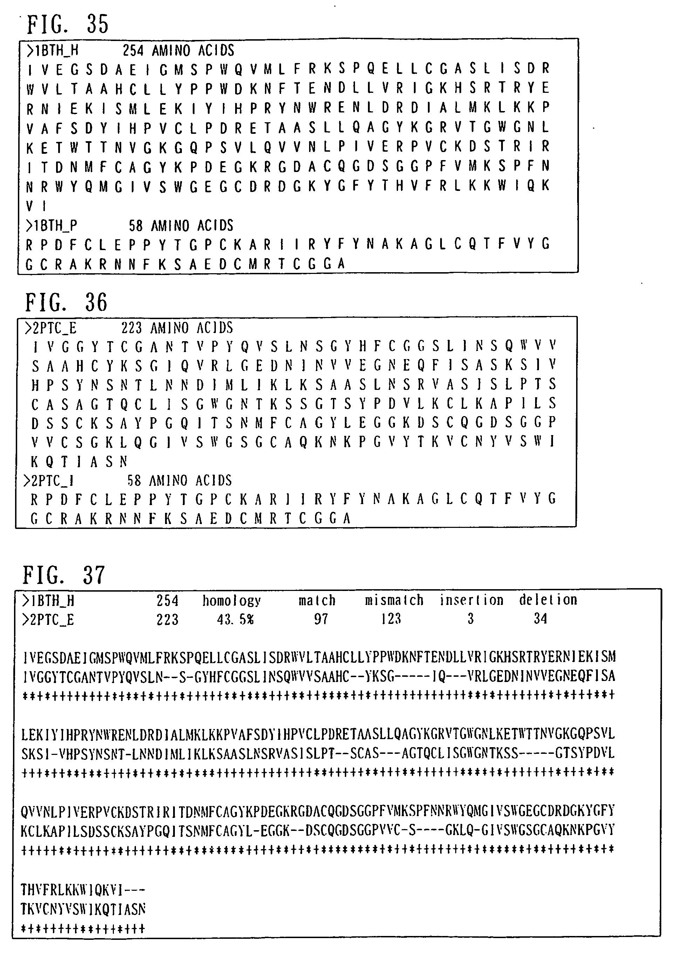

Fig. 35 shows the amino acid sequences of H chain and P chain of 1BTH in Example 4.

[Fig. 36]

Fig. 36 shows the amino acid sequences of E chain and I chain of 2PTC in Example 4.

[Fig. 37]

Fig. 37 illustrates the alignment of H chain of 1BTH and E chain of 2PTC in Example 4.

[Fig. 38]

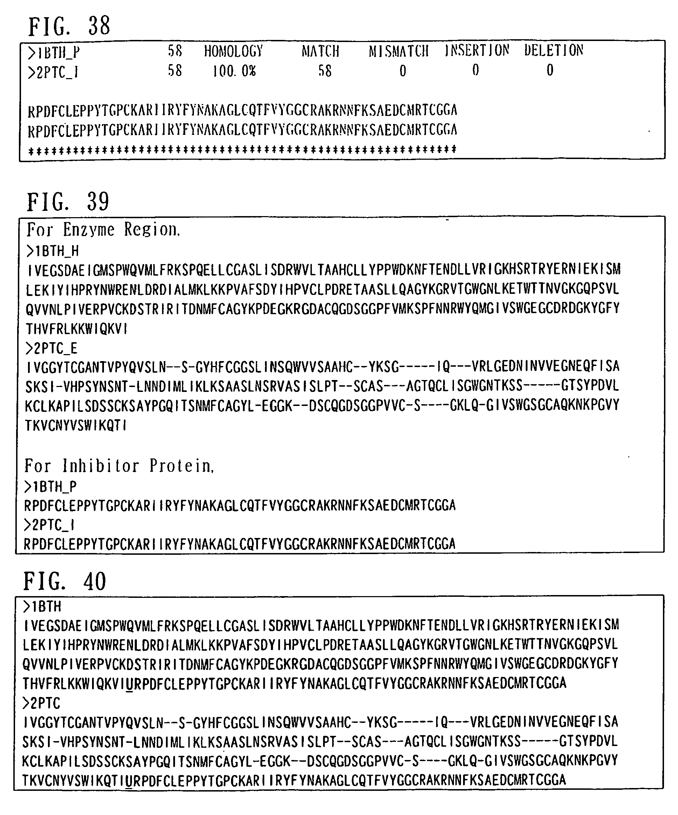

Fig. 38 illustrates the alignment of P chain of 1BTH and I chain of 2PTC in Example 4.

[Fig. 39]

Fig. 39 shows the input file format of the conventional FAMS in Example 4.

[Fig. 40]

Fig. 40 shows the input file format of newFAMS in Example 4.

[Fig. 41]

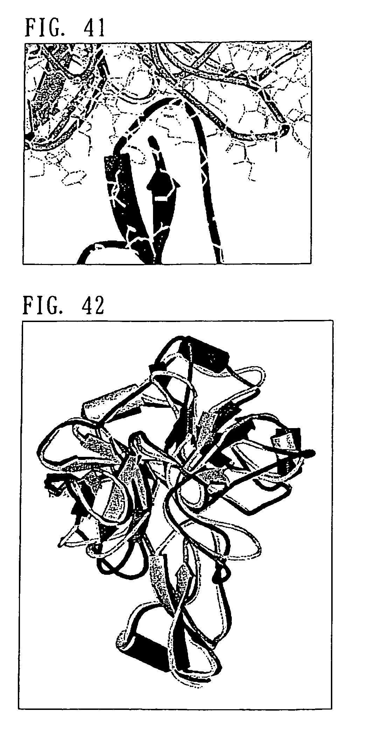

Fig. 41 illustrates the bound surface of the model of H chain and P chain of 1BTH in Example 4.

[Fig. 42]

Fig. 42 shows the superposed tertiary structures of the 1BTH (H chain and P chain) model, which is modeled from 2PTC and the 1BTH (H chain and P chain) model, which is actually registered in PDB.
The darker one was obtained by actual X-ray crystallographic analyses and the lighter one was obtained by performing the modeling this time.

[Fig. 43]

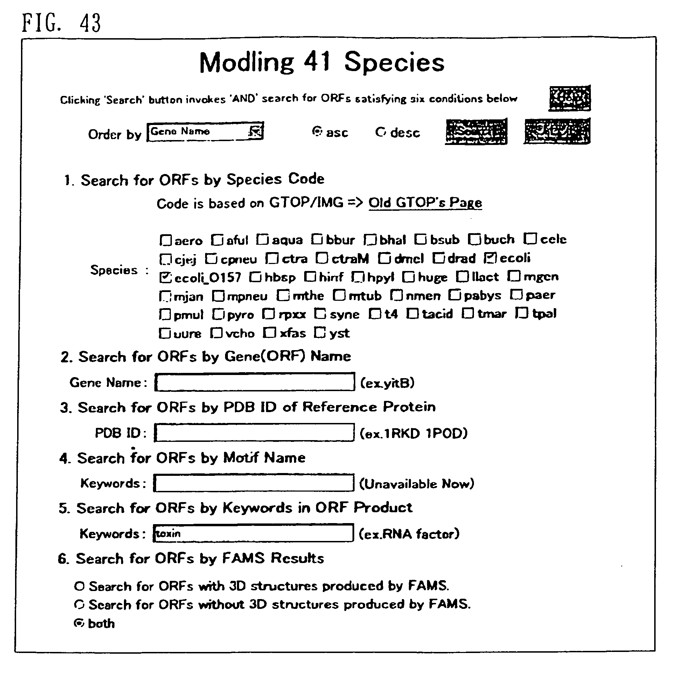

Fig. 43 illustrates an example of interface screens to a tertiary structure database in Example 5.
It is designed that a list of models whose three-dimensional coordinates can be browsed is displayed and an alignment is displayed by clicking the right button thereby three-dimensional coordinates can be obtained.

[Fig. 44]

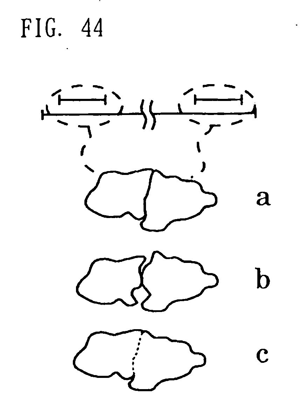

Fig. 44 conceptually illustrates and compares the case of modeling a tertiary structure by the present invention with the case of modeling by the conventional method when selecting a tertiary structure in which domains (plural chains) separated in the sequence or separated widely are contacted in the space as a reference protein.
Fig. 44a: The result of the tertiary structure of a complex in the state where domains (plural chains) separated in the sequence or separated widely are contacted in the space, is experimentally determined; Fig. 44b: The result of modeling by the conventional method in which the structure shown in Fig. 44a was used as a reference protein (a not-preferred model); Fig. 44c: The result of modeling by the present invention in which the structure shown in Fig. 44a was used as a reference protein (a preferred good model).

Embodiments of the Invention

Hereinafter, embodiments of the present invention will be described. The present invention will be detailed mainly describing a homology modeling method as a preferred and typical example. However, these are only for the purpose of illustrating typical examples, therefore the present invention is not limited thereto.
The present invention expands the applicable range of the protein Full Automatic Modeling System (FAMS: refer to K. Ogata and H. Umeyama, Journal of Molecular Graphics and Modeling 18, 258-272, 2000. The whole content is included for reference in this description as a part) developed in the Department of Biomolecular Design (Professor Hideaki Umeyama), School of Pharmaceutical Sciences, Kitasato University, and improves the system.
Several terms are used in this description and unless otherwise explicitly stated, their definitions are as follows.

"Target protein" means a protein whose complete tertiary structure has not been determined by an X-ray crystallographic analysis, an NMR analysis, or the like and which is the subject of tertiary structure construction in the present invention. The amino acid sequence of this protein may be referred to as "target sequence" or "target amino acid sequence" in some cases. This target proteinmay include a protein whose partial structure has been determined but complete tertiary structure has not been determined, a protein whose function has been specified, a protein whose function has been presumed, a protein whose amino acid sequence has been determined but function has not at all been known, and the like. "Reference protein" means a protein whose detail tertiary structure has been already determined by an X-ray crystallographic analysis or an NMR analysis, and which is referred to for alignment or optimization of atomic coordinates. The amino acid sequence of this protein may be referred to as "a reference sequence" or "a reference amino acid sequence" in some cases.

"Alignment" means, in the case where amino acid sequences of at least two types of proteins are matched, to mutually correlate them, and the method is detailed in the following explanation of each step.

"Atomic coordinates" describes a tertiary structure in three-dimensional coordinates. It is a relative distance in three directions perpendicular to each other with a certain point in the space as the origin, and is a vector quantity composed of three numbers per atom excluding hydrogen atoms existing in a protein.

Fig. 9 is a flow chart showing an example of the method of constructing a tertiary structure of a protein composed of plural chains according to the present invention.

As shown in Fig. 9, in this method (an example), first an amino acid sequence of a protein with an unknown tertiary structure (hereinafter referred to as "target sequence") is prepared in Step 10. In Step 20, a reference protein (a reference amino acid sequence) is selected from a tertiary structure database by using FASTA, PSI-BLAST or LIBRA. Using 20 types of characters, which represent amino acid residues, as an index, the alignment of the target sequence and the selected reference amino acid sequence is performed. In Step 30, amino acid sequences of one or more reference proteins are selected based on the search results. In Step 40, the character "U" is added to the C terminal region (end) of each amino acid sequence of the plural chains as a delimiter, and the sequence alignment is corrected by similarly adding "U" as a delimiter sequentially to the end of each amino acid sequence at the position corresponding to the target sequence. At this time, it is not necessary to add the above-mentioned delimiter to the terminal region of a chain corresponding to the last chain (the terminal end of a chain constituting a temporary single chain; the last terminal end of a temporary single chain) as mentioned above. Even if it is added, the delimiter added to the last terminal end can be disregarded. In Step 50, based on the alignment information, coordinates are obtained for $C\alpha$ atoms, which are one of the constitutive atoms of an amino acid residue, from the determined reference structure in Step 30, and the $C\alpha$ atomic coordinates are optimized so as to minimize the objective function ($E_{C\alpha}$) composed of the sum of various potential terms mentioned below by a simulated annealing method. In Step 60, main-chain atomic coordinates are added to the $C\alpha$ coordinates obtained in Step 50 from a database, and the main-chain atomic coordinates are optimized so as to minimize the objective function ($E_{main}$) composed of the sum of various potential terms mentioned below by the simulated annealing method. In Step 70, side-chain atomic coordinates are added to the main-chain atomic coordinates obtained in Step 60 from a database, and a tertiary structure is constructed by the same simulated annealing method as in Step 60. In Step 80, varidity of the tertiary structure of the completed model is evaluated as mentioned below and the final structure is attained in Step 90. The validity of the model structure is evaluated by superposing the main-chain atomic coordinates of the model and the reference protein for regions excluding a loop insertion or a deletion region, and the model is judged appropriate (valid) if rmsd is, for example, 1 Å or less.

The present invention has been developed, differing from the conventional FAMS, by correcting Steps 50, 60 and 70, which could only perform the structure optimization with the use of a reference structure of a single chain conventionally, thereby enabling to apply to also plural chains. Hereinafter, each step is further detailed as a preferred embodiment.

Step 10: Amino acid sequence of a protein with unknown structure

First, an amino acid sequence of a target protein with an unknown structure (target sequence) is prepared. As an amino acid sequence of a used target protein, a sequence of any origin, such as one registered in a database or one analyzed for the sequence for the first time, maybe used. In addition, a sequence of a protein whose partial structure only has been analyzed may also be the subject of construction of a tertiary structure in the present invention to obtain the information of complete tertiary structure. The database, which can be used, includes, for example, human (*H. sapiens*), drophila (*D. melanogaster*), nematode (*C. elagans*), yeast (*S. cerevisiae*), Arabidopsis thaliana (*A. thaliana*) and the like registered in a database such as GenBank: ftp://ncbi.nlm.nih.gov/genbank/genomes/, PIR: http://www-nbrf.georgetown.edu/pir/ (National Biomedical Research Foundation (NBRF)), Swiss Plot: http://www.expasy.ch/sprot/sprot-top.html (Swiss Institute of Bioinformatics (SIB), European Bioinfomatics Institute (EBI)), TrEMBL (the same URL and administrator as Swiss Plot), TrEMBLNEW (the same URL and administrator as Swiss Plot) and DAD: ftp://ftp.ddbj.nig.ac.jp (Japan DNA Data Bank).

These databases are merely examples and any databases may be used as long as they are registered with amino acid sequences of proteins.

Step 20: Database search and sequence alignment by alignment software such as FASTA, PSI-BLAST or LIBRA

Preferable softwares for the alignment of amino acid sequences prepared in Step 10 are, for example, FASTA, PSI-BLAST (Position-Specific Iterated BLSAT), LIBRA and the like.

FASTA is a program that searches a highly homologous sequence with a target sequence from a tertiary structure database and calculates the final similarity of the target sequence and the reference protein as an E-value. The details of FASTA are described in "Effective protein sequence comparison" Pearson W R, (1996) Methods Enzymol; 266: 227-58.

PSI-BLAST is programmed so as to perform profile alignment. The details of PSI-BLSAT are described in "Matching a protein sequence against a collection of PSI-BLAST-constructed position-specificscore matrices"Schaffer-AA, WolfYI, Ponting CP, Koonin EV, Aravind L and Altschul SF, Bioinformatics 1999, 12, 1000-11.

PSI-BLSAT, which performs profile alignment, is a tool provided with the highest performance at present in the search of similarity of sequences. PSI-BLAST is one of the series of programs that search similar proteins called BLAST and output alignments. As the programs showing equivalent performance recently, RPS-BLAST and IMPALA are available (refer to A.A. Schaffeetal., Bioinformatics, 15(12), 1000-1011, 1999). This program draws profile information only from statistically significant alignment relationship in the databases and generates a position specific score matrix (a matrix indicating a probability of substitution of each residue in the amino acid sequences with a certain amino acid residue statistically) of an amino acid sequence. Then inside the program, sequences having high similarity with the generated position specific score matrix instead of the target protein sequence are searched from a database, and the position specific score matrix is serially revised each time until significant alignments are not searched when the smallness of E-value is regarded as the limit. Then, the similarity of the final position specific score matrix and the reference protein is calculated as an E-value.

"E-value" quantitatively describes random background noise that exists for matches between sequences. It indicates how well two sequences are matched and it has a property of decreasing exponentially against scores representing sequence similarity and it is useful as a method for setting a threshold value when evaluating results. In PSI-BLAST, it corresponds to the case where the size of E-value has a value of normally 0.1 or less, preferably 0.001 or less (refer to A.A. Schaffe et al., Bioinformatics, 15(12), 1000-1011, 1999).

Homology search is performed for the reference protein sequence from the tertiary structure database PDB and the sequence alignment of the searched reference sequence and the target sequence is performed using such a program.

Here, "reference protein" is a data of a sequence and a three-dimensional atomic coordinates obtained from the tertiary structure database. It can be obtained from public databases registered as the protein data bank (PDB).

In PDB database, 26243 tertiary structures were registered as of November 2001. As an example, ones having sequence similarity of 95% or more are judged to be in the same category and the longest sequence in each category, and the structure having the highest X-ray resolution if the size is the same, is selected as the representative of the category. A tertiary structure database used in the present invention is a database, in which these representatives have been collected. The representative structures of 3922 were used as the PDB database when the present invention was performed.

Step 30: Selection of amino acid sequences of one or more reference proteins based on the search results

Based on the results of the homology search, amino acid sequences of one or more reference proteins that have statistically significant similarity to the target sequence are selected.

Step 40: Correction of sequence alignment by inserting delimiter U at the end of each amino acid sequence of plural chains

In the case where the target sequence of the protein composed of plural chains was aligned with each reference sequence of the plural proteins in Step 30, modeling was performed for each chain of the target sequences conventionally. However, in the present invention, a delimiter (e.g. the character "U") is inserted in the C terminal region (end) of each amino acid sequence of the plural chains, and alignment for performing simultaneously the modeling of all the target protein chains (such as polypeptide chains) as a temporary single chain is prepared. The higher limit of the number of protein chains, which can be calculated, was actually set to preferably 36 by referring to the proteins of plural chains registered in PDB, however it can be theoretically expanded to the number necessary for modeling or to the capacity limit of the computer to be used.

Step 50: Construction of initial Cα atomic coordinates

Using the alignment including the delimiter in Step 40, based on the comparison of the target sequence with the reference sequences, information is obtained for amino acid residues that have an insertion or a deletion. A region in which three or more consecutive amino acid residues are matched in the alignment is selected, and in the region, the same one as the reference proteins is applied to as the Cα atom of the target protein for these pairwise amino acid residues. In the case where a Cα atom could not be obtained, coordinates are applied to from the peptide fragment database (refer to K. Ogata and H. Umeyama, Journal of Molecular Graphics and Modeling 18, 258-272, 2000) composed of Cα atoms constructed beforehand from PDB (refer to Fig. 10).

Step 50 (1): Optimization of Cα atom by simulated annealing method

The Cα atom constructed in the above Step 50 is optimized using the objective function ($E_{C\alpha}$) obtained by referring to the reference protein coordinates using the simulated annealing step explained in the paragraph of the above-mentioned background art. This objective function is as the below-mentioned formula (1). One of the important differences between the conventional method and the present invention is that the chemical bond potential $E_{len}$, the chemical bond angle potential $E_{ang}$ and the interatomic interaction potential $E_{vdw}$ in the formula (1) are corrected as below with the $k_N$ value (equal to the serial number of the C terminal residue of the N-th protein chain) determined by referring to the delimiter U in the alignment created in Step 40.

$$E_{C\alpha} = E_{len} + E_{ang} + E_{vdw} + E_{ss} + E_{pos} \tag{1}$$

$E_{len}$ relates to the distance between Cα atoms of the residues located side by side in the sequence and is defined by the formula (2) below.

$$E_{len} = K_l \sum_i (D_{i,i+1} - 3.8)^2$$
$$(i \neq k_{N(N=1,M-1)}) \tag{2}$$

Here, $D_{i,i+1}$ is the distance between Cα atoms of the residue i and the residue i+1. $K_1$ is a constant and, for example, set to 2. However, a chemical bond does not exist between the C terminal residue $k_1$ of the first protein chain and the N terminal residue $k_1+1$ of the second protein chain, therefore the case of $i=k_1$ is not included in the calculation of $E_{len}$. In the same way, as shown in Fig. 2, a chemical bond does not exist between the C terminal residue $k_N$ of the N-th protein chain and the N terminal residue $k_N+1$ of the (N+1)-th protein chain, therefore the case of $i=k_N$ is not included in the calculation of $E_{len}$ (hereinafter, such a procedure is referred to as "disconnection of interaction"). In the case where the total number of protein chains is M, this procedure is performed from N=1 to M-1, and the residue numbers from $k_1$ to $k_{M-1}$ can be specified by the positions of U from the first delimiter $U_1$ to the (M-1)-th delimiter $U_{M-1}$ in the alignment created in Step 40.

Next, $E_{ang}$ is a function of the chemical bond angle of Cα atoms and is as the formula (3) below:

$$E_{ang} = K_a \sum_i (\theta_i - \theta_0)^2 \tag{3}$$

provided that $i \neq k_{N(N=1,M-1)}$, $i \neq k_{N(N=1,M-1)}+1$.

Here, $\theta_i$(rad) is the angle of Cα atoms of the i-th, (i+1)-th and (i+2)-th residues. $\theta_0$ is defined as $(100/180)\pi$(rad) based on the X-ray structure of PDB. $K_a$ is a constant and, for example, set to 1. However, regarding the bond angle potential $E_{ang}$, as shown in Fig. 3, disconnection of interaction procedure is performed in the same manner as $E_{len}$. In other words, the cases of $i=k_N$ and $i=k_N+1$ are not included in the calculation of $E_{ang}$. This disconnection of interaction procedure is performed in the amino acid residues from $k_1$ to $k_{M-1}$.

Next, $E_{vdw}$ is the van der Waals potential between Cα atoms and generally considered for residues separated by three or more residues and is as the formula (4) below:

$$E_{vdw} = K_{vdw} \sum_{i,j(>i+2)} \left\{ \left( \frac{3.8}{D_{i,J}} \right)^{12} - 2 \left( \frac{3.8}{D_{i,J}} \right)^{6} \right\} \qquad (4)$$

provided that j>i+1 if i= $k_N$-1 and j>i if i= $k_N$.

Here, $D_{i,j}$ is the distance between the pairwise atoms i, j that are within 6 Å from the i-th Cα atom, which is the subject, and the value of $K_{vdw}$ is set to 0.01 ($D_{i,j} \leq 3.2$ Å) or 0.001 ($D_{i,j}$>3.2 Å). However, as shown in Fig. 4, because a chemical bond does not exist between the C terminal residue $k_N$ of the N-th protein chain and the N terminal residue $k_N$+1 of the N+1-th protein chain, the calculation of $E_{vdw}$ must be performed under the conditions that j>i+1 if i= $k_N$-1 and j>i if i= $k_N$. In the case where the total number of protein chains is M, this procedure is performed from N=1 to M-1, and the residue numbers from $k_1$ to $k_{M-1}$ can be specified by the positions of the delimiter U from $U_1$ to $U_{M-1}$ in the alignment created in Step 40.

Next, $E_{ss}$ relates to the distance between the Cα atoms of Cys residues which make a pair and forms an S-S bond and is defined by the formula (5) below.

$$E_{ss} = K_{ss} \sum_{i} (D_i^{ss} - 5.4)^2 \qquad (5)$$

Here, $D_i^{ss}$ is the distance between the pairwise Cα atoms of Cys residues that form a disulfide bond within a protein chain or between protein chains. Because the serial numbers throughout all the protein chains are used for the residue number i in the present invention, $E_{ss}$ between protein chains can be handled with the conventional potential function. $K_{ss}$ is a constant and set to, for example, 5.

Next, $E_{pos}$ is a function related to the position of Cα atoms and defined by the formula (6) below. This energy term is introduced for the purpose of maintaining the positions of Cα atoms relatively stable in the protein SCRs (Structural Conserved Regions: mentioned below).

$$E_{pos} = K_{pos} \sum_{i} \frac{1}{M_i} \| \mathbf{x}_i - \langle w_i \mathbf{x}_i \rangle \|^2 \qquad (6)$$

Here, $X_i$ represents the coordinate of the i-th Cα atom and $M_i$ represents the average distance between Cα atoms that are structurally equivalent on the alignment based on the structure or, in other words, located most closely in the three-dimensional coordinate system. When $M_i$ value cannot be obtained for the residue i or, in other words, a certain amino acid residue of the target sequence cannot be correlated with the Cα of the reference sequences, $M_i$ value is set to 10. $\|\cdot\|$ means a norm (distance between coordinate vectors), and $\langle w_i x_i \rangle$ means the average coordinate of Cα atoms and is as the formula (7) below:

$$\langle w_i \mathbf{x}_i \rangle = \frac{1}{W} \sum_{j} w_i^j \mathbf{x}_i^j \qquad (7)$$

provided that j≠i.

Here, $x_i^j$ represents a Cα atomic coordinates corresponding to the i-th residue of the j-th reference protein and $w_i^j$ represents a weight for the position of the i-th Cα atom of the j-th reference protein and W is the sum of $w_i^j$ for j. This $w_i^j$ is an important parameter because it determines a rough frame of the target protein. It is determined by a local value of spatial proximity within 12 Å of a given region called local space homology (LSH) (refer to K. Ogata and H. Umeyama, Journal of Molecular Graphics and Modeling 18, 258-272, 2000) as shown in Fig. 11. The relationship between LSH and the ratio of the pairwise residues belonging to the regions where structures are well conserved (SCRs: Structural Conserved Regions) is very high as shown in Fig. 12. This means that the position of the Cα atom is statistically located within 1.0 Å compared with the reference proteins when it has a high LSH value.

Cα atoms are optimized repeatedly according to the formula (1) using the simulated annealing method. In this optimization step, the perturbation of Cα atoms is set, for example, within 1.0Å. In addition, this annealing step is repeated, for example, 100 times for all Cα atoms respectively. The parameter corresponding to the temperature is started at 25 and decreased by a factor of 0.5 at every step until 0.01 is reached and then using a constant value for the parameter.

From the result of the superposition of the tertiary structures, the acquisition of structural information from the reference sequence having the least number of insertion and deletion to the target sequence and the construction of the Cα atoms are repeated ten times, and the Cα atomic coordinates with the least value of the objective function is calculated as an optimal solution.

Step 60: Construction and optimization of main-chain atomic coordinates

Coordinates of other main-chain atoms (an N atom of amide, a C atom of carbonyl and an O atom of carbonyl) and a Cβ atom that is chemically bonded to the Cα atom are added to the Cα atomic coordinates of step 50(1) and the objective function ($E_{main}$, of formula (8) mentioned below) is minimized by the simulated annealing method. First, the Cα atoms are superposed three-dimensionally and the residues having a distance between Cα atoms of 2.5 Å or less are selected. The coordinates of main-chain atoms except the Cα are obtained from the coordinates of the reference proteins having the least distance between Cα atoms that should be superposed and are defined as the model structure.

In the case where there are no corresponding residues in the reference proteins, the main-chain atomic coordinates are constructed from a corresponding protein fragment composed of 4 residues in a database, which has been constructed beforehand (refer to, for example, K. Ogata and H. Umeyama, Journal of Molecular Graphics and Modeling 18, 258-272, 2000). In this procedure, the main-chain atom of the residue i is selected from the residues having the smallest rmsd value among Cα atoms from the (i-1)-th to (i+2)-th. At this time, the range of the superposition of the Cα atomic coordinates is from the i-th to (i+3)-th for the residues at the N terminal, and similarly from the (i-3)-th to i-th and from the (i-2)-th to (i+1)-th for the residues at the C terminal and one residue before the C terminal, respectively.

A main-chain atomic coordinates (also including a Cβ atom of a side-chain) is optimized by the simulated annealing method based on the objective function of the main-chain atom. The objective function is as the formula (8) below. One of the important differences between the conventional method and the present invention is that the chemical bond potential $E_{bond}$, the chemical bond angle potential $E_{ang}$, the chemical bond torsional angle potential $E_{tor}$ and the interatomic interaction potential $E_{non-bond}$ in the formula (8) are corrected as follows by $k_N$ value (equal to the serial number of the C terminal residue of the N-th protein chain) determined by referring to the delimiter U in the alignment created in Step 40.

$$E_{main} = E_{bond} + E_{ang} + E_{tor} + E_{non-bond} + E_{SS} + E_{pos} + E_{chi} + E_{hydr} \qquad (8)$$

$E_{bond}$ is defined as the formula (9) below:

$$E_{bond} = K_b \sum_i (b_i - b_i^0)^2 \qquad (9)$$

provided that, the case of $i=k_{N(N=1,N-1)}$ and $b_i=D_{Ci, Ni+1}$ is not added. (i+1 is the subscript of the subscript.)

Here, $b_i^0$ is a standard bond length and varies depending on the three types of chemical bonds, N-Cα, Cα-C and C-N, however it is simplistically described here. $K_b$ is a constant and set to, for example, 225. Regarding the calculation of $E_{bond}$, as shown in Fig. 5, because a chemical bond does not exist between the C terminal residue $k_N$ of the N-th protein chain and the N terminal residue $k_N+1$ of the (N+1)-th protein chain, according to the conditions that $b_i-b_i^0=0$ if $i=k_{N(N=1,M-1)}$ and $bi=D_{Ci,Ni+1}$, disconnection of interaction is performed as in the case of the calculation of $E_{len}$ for Cα by excluding from the calculation of $E_{bond}$. In the case where the total number of protein chains is M, this procedure is performed from N=1 to M-1 and the residue numbers from $k_1$ to $k_{M-1}$ can be specified according to the positions of the delimiter U from $U_1$ to $U_{M-1}$ in the alignment created in Step 40.

$E_{ang}$ is a function of chemical bond angle and as the formula (10) below:

$$E_{ang} = K_a \sum_i (\theta_i - \theta_i^0)^2$$

$$(10)$$

provided that if $i=k_{N(N=1,M-1)}$ and $\theta_i=\theta_{C\alpha i,Ci,Ni+1}$, the case is not added, if $i=k_{N(N=1,M-1)}$ and $\theta_i=\theta_{Di,Ci,Ni+1}$, the case is not added, if $i=k_{N(N=1,M-1)}$ and $\theta_i=\theta_{Ci,Ni+1,C\alpha i+1}$, the case is not added.
(explanation of three atoms that determine an angle)

Here, $\theta_i^0$ is a standard bond angle and varies depending on the types of each bond angle, however it is simplistically described here. $K_a$ is a constant and set to, for example, 45. Regarding the calculation of $E_{ang}$, as shown in Fig. 6, because a chemical bond does not exist between the C terminal residue $k_N$ of the N-th protein chain and the N terminal residue $k_N+1$ of the (N+1)-th protein chain, disconnection of interaction is performed by excluding from the calculation of $E_{ang}$. In the case where the total number of protein chains is M, this procedure is performed from N=1 to M-1 and the residue numbers from $k_1$ to $k_{M-1}$ can be specified according to the positions of the delimiter U from $U_1$ to $U_{M-1}$ in the alignment created in Step 40.

$E_{tor}$ is the chemical bond torsional angle potential of a main-chain and is as the formula (11) below:

$$E_{tor} = K_t \sum_i \sqrt{(\phi_i - \phi_i^0)^2 + (\psi_i - \psi_i^0)^2} + K_\omega \sum_i (\omega_i - \omega_i^0)^2$$

$$(11)$$

provided that if $i=k_{N(N=1,M-1)}$, $\varphi_i-\varphi_i^0=\omega_i-\omega_i^0=\phi_{i+1}-\phi_{i+1}^0 =0$.

Here, $\phi_i^0$ and $\varphi_i^0$ is set so that the torsional angle of the main-chain will satisfy Ramachandran plot. In other words, $(\phi_i^0, \varphi_i^0)$ that is closest to the coordinate $(\phi_i, \varphi_i)$ and satisfies Ramachandran plot is selected. In addition, $\omega_i^0$ is set to 0 and, is set to $\pi$(rad) only in the case of the cis-Pro residue. $K_t$ and $K_\omega$ are constants and set to for example, 10 and 50, respectively. Regarding the calculation of $E_{tor}$, as in the case of the calculation of $E_{ang}$, because a chemical bond does not exist between the C terminal residue $k_N$ of the N-th protein chain and the N terminal residue $k_N+1$ of the (N+1)-th protein chain, disconnection of interaction is performed by excluding from the calculation of $E_{tor}$. Fig. 7 shows $\omega_i$, and $\varphi_i$ and $\phi_{i+1}$ are handled in the same way. In the case where the total number of protein chains is M, this procedure is performed from N=1 to M-1 and the residue numbers from $k_1$ to $k_{M-1}$ can be specified according to the positions of the delimiter U from $U_1$ to $U_{M-1}$ in the alignment created in Step 40.

$E_{non-bond}$ is the interatomic interaction potential and as the formula (12) below:

$$E_{non-bond} = K_{non} \sum_{i,j} \varepsilon_{i,j}^* \left\{ \left( \frac{r_{i,j}^*}{r_{i,j}} \right)^{12} - 2 \left( \frac{r_{i,j}^*}{r_{i,j}} \right)^6 \right\}$$

$$(12)$$

provided that if $r_{ij} \leq 8.0$ for $r_{ij} \in \{r_{Ni,Ni+1}; r_{C\alpha i,Ni+1}; r_{C\alpha i,C\alpha i+1}; r_{Ci,Ni+1}; r_{Ci,C\alpha i+1}; r_{Ci,C\beta i+1}; r_{Ci,Ci+1}; r_{Oi,Ni+1}; r_{Oi,C\alpha i+1}\}$ when $i=k_{N(N=1,M-1)}$, the case is included in the calculation of $E_{non-bond}$.

Here, $\varepsilon_{ij}^*$ and $r_{ij}^*$ are constants that vary depending on the types of atoms (refer to Koji Ogata, doctoral dissertation, Tokyo University of Science, 1999). $K_{non}$ is a constant and is set to, for example, 0.25, and it is normally included in the calculation of $E_{non-bond}$, in the case where the pairwise atoms i,j, whose $r_{ij}$ is, for example, 8 Å or less, are separated by three or more bonds. Regarding the calculation of $E_{non-bond}$, as shown in Fig. 8, as in the case of the calculation of $E_{vdw}$ for the C$\alpha$, because a chemical bond does not exist between the C terminal residue $k_N$ of the N-th protein chain and the N terminal residue $k_N+1$ of the (N+1)-th protein chain, if $r_{ij}$ belonging to $\{r_{Ni,Ni+1}; r_{C\alpha i,Ni+1}; r_{C\alpha i,C\alpha i+1}; r_{Ci,Ni+1}; r_{Ci,C\alpha i+1}; r_{Ci,C\beta i+1}; r_{Ci,Ci+1}; r_{Oi,Ni+1}; r_{Oi,C\alpha i+1}\}$ is 8 Å or less when $i=k_{N(N=1,M-1)}$, the case must be newly included in the calculation of $E_{non-bond}$. In the case where the total number of protein chains is M, this procedure is performed from N=1 to M-1 and the residue numbers from $k_1$ to $k_{M-1}$ can be specified according to the positions of the delimiter U from $U_1$ to $U_{M-1}$ in the alignment created in Step 40.

$E_{ss}$ is a function of a disulfide bond formed by the Cys residue and is as the formula (13) below.

$$E_{ss} = \sum_i \left\{ K_{C\alpha}^{SS}(D_i^{C\alpha} - 5.4)^2 + K_{C\beta}^{SS}(D_i^{C\beta} - 3.8)^2 \right\}$$

(13)

Here, $D_i^{C\alpha}$ and $D_i^{C\beta}$ are the distances between the pairwise Cα atoms and the pairwise Cβ atoms of the Cys resides forming the disulfide bond within a protein chain or between protein chains respectively. Because the serial numbers throughout all the protein chains are used for the residue number i, in the present invention, $E_{ss}$ between protein chains can be handled with the conventional potential function. $K_{C\alpha}^{ss}$ and $K_{C\beta}^{ss}$ are constants and, for example, 7.5.

$E_{pos}$ is a function related to the positions of the main-chain atoms and is as the formula (14) below. The explanation of the formula is the same as that of the formula (6) mentioned above.

$$E_{pos} = K_{pos} \sum_i \frac{1}{M_i} \left\| \mathbf{x}_i - \langle w_i \mathbf{x}_i \rangle \right\|^2$$

(14)

Here, $\langle w_i x_i \rangle$ is given as the formula (15) below. The explanation of the formula is the same as that of the formula (7) mentioned above.

$$\langle w_i \mathbf{x}_i \rangle = \frac{1}{W} \sum_j w_i^j \mathbf{x}_i^j$$

(15)

$\langle w_i x_i \rangle$ in the above-mentioned formula (12) is obtained by superposing structures of the target protein and the reference protein. $K_{pos}$ is a constant and, for example, 0.3.

$E_{chi}$ relates to chirality of Cα and is as the formula (16) below. Here, the chirality of Cα relates to an optical isomer of an amino acid residue (L-form or D-form), and the potential of the formula (16) is used so as to generally become a Cα atom of an L-form.

$$E_{chi} = K_{chi} \sum_i \left( \tau_i - \frac{2}{3}\pi \right)^2$$

(16)

Here, $\tau_i$ is a torsional angle defined by N-Cα-Cβ-C of the i-th residue and $K_{chi}$ is set to, for example, 50.

$E_{hydr}$ relates to a hydrogen bond of the main-chain conserved in proteins with homologous sequences and is defined as the formula (17) below.

$$E_{hydr} = K_{hydr} \sum_{i,j} \left( D_{i,j}^{N-O} - 2.9 \right)^2$$

(17)

A hydrogen bond is set when the distance of an N atom and an O atom is 2.9±0.5 Å. When it is judged whether a hydrogen bond exists in the plural reference proteins or not, it is judged to exist a hydrogen bond if at least 75% (at least 3 out of 4) of the reference proteins are confirmed to have a hydrogen bond. $K_{hydr}$ is a constant and, for example, 0.6.

Next, the optimization of main-chain atoms including Cβ is performed by the simulated annealing method. In this procedure, the perturbation of the main-chain and Cβ atoms is set within 1.0 Å for the initial position. This procedure is generally repeated 200 times for the main-chain and Cβ atoms. The parameter corresponding to the temperature is generally started at 50 or 25, and decreased by a factor of 0.5 at every step until 0.01 is reached and then using a constant value for the parameter.

In order to take samples of a configuration of a main-chain widely, preferably, the above-mentioned method is performed six times and the main-chain atomic coordinates having the least value of the objective function ($E_{main}$) is regarded as an optimal solution. Then the above parameter corresponding to the temperature generally is started at 50 for the first and the second of six optimizations and is started at 25 for the third and the rest.

Step 70: Construction and optimization of side-chain atomic coordinates

The construction of a side-chain is roughly divided into two steps. It can be divided into "Construction of side-chain in structural conserved region" (Step 70(1)) and "Construction of all side-chains" (Step 70 (2)) (refer to K. Ogata and H. Umeyama, Journal of Molecular Graphics and Modeling 18, 258-272, 2000).

Step 70(1): Construction of side-chain in structural conserved region

For the calculated main-chain atom, in the case of structural conserved region (SCR), a torsional angle of a side chain is obtained from a protein with a homologous sequence using the method in the previous studies. The details of this method is described in "The role of played by environmental residues in side-chain torsional angles within homologous families of proteins: A new method of side-chain modeling." Ogata K and Umeyama H, Prot. Struct. Funct. Genet. 1998, 31, 255-369. The whole content is included for reference in this description as a part.
In this method, the ratio of side-chains conserved in the proteins with homologous sequences is calculated, and the modeling of side-chains is performed based on this information. The side-chain atomic coordinates in a conserved region of a side-chain is located to the fixed main-chain atom. For example, if the $\chi 1$ angle of an arginine residue in a protein with a homologous sequence is conserved, the $C\gamma$ atomic coordinates can be located, and if $\chi^1$ and $\chi^2$ angles of a Phe residue are conserved, all the side-chain atoms can be located. The optimization step of the simulated annealing using the formula (8) is performed only for the main-chain and the $C\beta$ atoms, and the perturbation of the atoms was set within 1.0 Å. This annealing step of the main-chain and the $C\beta$ atoms is repeated 200 times. The parameter corresponding to the temperature is started at 25 and decreased by a factor of 0.5 at every step until 0.01 is reached. $E_{non-bond}$ in the above-mentioned formula (8) is performed for the main-chain atom and the partially constructed side-chain atoms. At this time, the side-chain atomic coordinates are made to be conserved throughout the optimization step.
Mi in the above-mentioned formula (14), which is the information regarding structure, and a pair of N-O forming a hydrogen bond in the above-mentioned formula (17) are re-calculated for the distance in the optimization step. In particularly an N-O pair is used depending on the judgment of existence of a hydrogen bond. In order to obtain the configuration of the main-chain atom, the above-mentioned procedure is repeated 3 times and the main-chain atomic coordinates having the least value of the objective function is determined as the calculated structure.

Step: 70 (2): Construction of all side-chains

The construction of all the side-chains is performed in the group of the fixed main-chain and $C\beta$ atoms. This is performed according to the method described in the above-mentioned literature of Ogata K and Umeyama H, Prot. Struct. Funct. Genet. 1998, 31, 255-369. By using the method, an accurate model can be given in a short time. First, a main-chain structure (including $C\beta$) is optimized at low temperature by the Monte Carlo method using the objective function $E_{main}$ of the above-mentioned formula (8). At this time, the temperature is set at 0.001 and regarding $E_{non-bond}$ in the above-mentioned formula (8), the calculation between the main-chain atoms and all the side-chain atoms is performed. Then, the side-chain coordinates are re-located so as to maintain the torsional angle of the side-chains at the optimized state in the optimization step of N, $C\alpha$, C and $C\beta$ atoms. The perturbation of atoms is set within 0.5 Å. Next, the side-chains are deleted and the above-mentioned construction of the side-chains is repeated. This procedure is repeated until the constructed structure does not have a collision of atoms of 2.4 Å and the torsional angle of N-$C\alpha$-$C\beta$-C is fallen within the range of $-120\pm15°$.

Step 80: Evaluation of appropriateness of model structure

The evaluation of appropriateness of the tertiary structure of a completed model is performed by superposing the main-chain atomic coordinates of the model and the reference protein for regions excluding a loop insertion or a deletion region, and the model was judged appropriate if rmsd is 1 Å or less.

Step 90: Construction of final structure: Tertiary structure prediction

As mentioned above, based on the alignment obtained in Step 40, a tertiary structure is constructed using a modeling software such as newFAMS that was newly developed by the present inventors this time in Steps 50-80, and then a model is completed. Together, the method shown in the above-mentioned Steps 40-80 is referred to as "newFAMS". On the other hand, the conventional modeling software (refer to K. Ogata and H. Umeyama, Journal of Molecular Graphics and Modeling 18, 258-272, 2000), which is the basis for the present invention, is simply referred to as "FAMS".

The single chain modeling formulae has been corrected as described in this description of the above-mentioned formulae (2), (3), (4), (9), (10) and (12) to realize the temporary single chain modeling of the plural chains modeling. As another method, the temporary single chain can be realized by handling the corresponding coefficients of these formulae, KI of the above-mentioned formula (2), Ka of the formula (3), Kvdw of the formula (4), Kb of the formula (9), Ka of the formula (10) and Kt of the formula (11) as values dependent on the residue number, and adjusting them respectively. At the step in which the present invention is specifically implemented, this temporary single chain modeling can be derived easily from the constitutions of the right side of the above-mentioned formulae (2), (3), (4), (9), (10) and (12). In addition, this temporary single chain modeling gives exactly the same results as the below-mentioned Examples 1 to 4.

According to the present invention, a tertiary structure in which domains (plural chains) separated in the sequence or separated widely are contacted in the space each other can be selected as a reference protein (refer to Fig. 44). A tertiary structure of a complex in the state where domains (plural chains) separated in the sequence or separated widely, are contacted in the space each other is determined experimentally (refer to Fig. 44a). By performing the modeling by the present invention using the determined tertiary structure as a reference protein, the tertiary structure of the target protein can be constructed accurately (the method of the present invention; refer to Fig. 44c). On the other hand, if the modeling is similarly performed by the conventional method, a tertiary structure, in which the contact surface of both domains is inaccurate, is constructed as shown in Fig. 44b. In such construction of tertiary structure, it is understood that more accurate tertiary structure can be constructed according to the present invention compared to the conventional method.

As mentioned above, the whole content of the literature about the conventional method cited in this description is included for reference in this description as a part. Similarly, the whole content regarding the invention of application documents such as the specification included in the Japanese application applied on January 9th, 2002: JP-A 2002-2859, which is the basis for the present application, is included for reference in this description as a part.

Preferred Embodiments

Hereinafter, the present invention will be described in detail with reference to the following examples. These are only for the purpose of illustrating the present invention, therefore the present invention is not limited these examples.

(Example 1) Example of modeling in metabotropic glutamate receptor family

The primary amino acid sequence of metabotropic glutamate receptor 1 protein derived from rat (reference protein) was obtained from Swiss-Prot (entry name MGR1_RAT, accession number P23385). As shown in Fig. 13 (refer to SEQ IDNO: 1 in the sequence listing), it has been found by an X-ray structure analysis by Morikawa *et al.* that the whole is 1199 residues, among the former part of 478 residues indicated by underlining, the 9 residues located from the 448th to 456th become the contact region each other, a dimmer (homodimer) is formed by assembling two monomers, and the glutamate receptor domain is formed (refer to Kunishima, N., Shimada, Y., Tsuji, Y., Sato, T., Yamamoto, M., Kumasaka, T., Nakanishi, S., Jingami, H., Morikawa, K.: Structural Basis of Glutamate Recognition by a Dimeric Metabotropic Glutamate Receptor, Nature 407, 971, 2000). Three types of protein tertiary structures (1EWK, 1EWT, 1EWV) are registered in PDB. 1EWK is a structure including glutamate as a ligand and 1EWT is a structure not including glutamate as a ligand. From the result of an X-ray structural analysis, it has been known that 1EWK is a closed form in which monomers are relatively close to each other, on the other hand, 1EWT is an open form in which monomers are relatively separated each other. Both are different in the relative configuration of the monomers in this way. Therefore the states of the bound surfaces between domains are significantly different.

That is, the glutamate receptor 1 protein has the open form in the state where the ligand is not bound (1EWT), but has the closed form in the state where the ligand is bound (1EWK), which is presumed to be more stable. Therefore, in this protein, it is very important to accurately perform modeling of the state of the bound surface including relative configuration of monomers for the elucidation of the function. Consequently, as an example, using 1EWT as a reference protein for the search of a homologous sequence, the tertiary structure model of a protein dimer, which does not include a ligand (reference protein), was constructed. In addition, it was examined if relative merits and demerits of the tertiary structure (difference in an energetic stability) occurred in the state of the bound surface between dimers by comparing the case where the modeling of each monomer, which constitutes a dimer, was performed by the conventional method and both were combined to make a dimer model with the case where the modeling of a dimer itself was performed by the present invention.

First, using the 1199 residues of MGR1_RAT amino acid sequence as a query, using PIR as of November 2001 as a motif profile, a PSI-BLAST search was performed on 774804 sequences registered in protein amino acid sequence databases such as PIR, Swiss Prot, TREMBL, TREMBL_NEW, GenPept (all as of November 2001). As a result of the search performed under the condition where E-value is 0.001 or less, 14509 homologous sequences and alignments were obtained. Among them, there are 70 whose E-value is 0 (the homology is between 23 and 100%, and because E-value is very small it is described as 0 as the computer output) . These can be considered almost identical regarding function. Among them, a modeling of the protein dimer of a receptor, derived also from rat, whose Swiss Prot entry name is "MGR5_RAT" and accession number is "P31424" (target protein), was performed (refer to Abe T., Sugihara H., Nawa H., Shigemoto R., Mizuno N., Nakanishi S., Molecular characterization of a novel metabotropic glutamate receptor mGluR5 coupled to inositol phosphate/Ca2+ signal transduction, J. Biol. Chem. 267, 13361-13368, 1992). MGR5_RAT is a signal transduction protein related to inositol phosphate and calcium ion, and is a metabotropic glutamate receptor protein subtype 5 derived from rat, and its amino acid residue number is 1203. The homology between the reference sequence MGR1_RAT and the target sequence MGR5_RAT is 62.2% and the result of their alignment is shown in Fig. 14 (refer to SEQ ID Nos:1 and 2 in the sequence listing) .

(Result of alignment between MGR1_RAT and MGR5_RAT)

| >MGR5_RAT | 1203 | homology | match | mismatch | insertion | deletion |
|-----------|------|----------|-------|----------|-----------|----------|
| >MGR1_RAT | 1189 | 62.2% | 740 | 396 | 53 | 67 |

Fig. 15 only shows regions in which coordinates of 1EWT in PDB exist in the alignment of Fig. 14 (refer to SEQ ID Nos:3 and 4 in the sequence listing). A modeling was performed using this alignment.

(Result of alignment between MGR1_RAT and MGR5_RAT; only regions in which coordinates exist in 1EWT)

| >MGR5_RAT | 474 | homology | match | mismatch | insertion | deletion |
|-----------|-----|----------|-------|----------|-----------|----------|
| >1EWT_A | 456 | 73.9% | 337 | 118 | 1 | 19 |

The alignment in Fig. 15 is for a monomer and the input file format in the conventional FAMS (refer to K. Ogata and H. Umeyama, Journal of Molecular Graphics and Modeling 18, 258-272, 2000) is as shown in Fig. 16 (refer to SEQ ID Nos:3 and 4 in the sequence listing). This modeling was performed by using the conventional FAMS. The modeling result is shown in Fig. 17.

Further, in the case of handling the alignment as a homodimer, the alignment in Fig. 16 is connected with the character "U", which is as shown in Fig. 18. This input file format, in which "U" is used, is the one which has been developed by the present inventors. The modeling result is shown in Fig. 19.

Further, the enlarged drawing of the bound surface of a homodimer model by newFAMS of Fig. 19 is shown in Fig. 20. In this drawing, the modeling of the recognition region of protein-protein interaction is precisely performed without a collision within 2.4 Å in a main-chain or a side-chain. On the other hand, when a tertiary structure of each of MGR5_RAT was constructed independently with the conventional FAMS using the three-dimensional coordinates of each monomer of 1EWT homodimer, interatomic contact within 2.4 Å occurred at 8 positions on the bound surface. This structure is quite energetically unstable because lots of collisions occur at the bound surface. Meanwhile, the model structure by newFAMS is energetically stable because no collision occurs at the bound surface. This indicates the superiority (novelty) of newFAMS for performing a modeling of plural chain, which the present inventors have developed.

(Example 2) Modeling example of transporter

The tertiary structure of a homologue of ABC transporter, which is believed to be one of the causes of multidrug resistance, has been analyzed by an X-ray crystallography at the resolution of 4.5 Å and registered, although only for Cα coordinates, in PDB as 1JSQ (reference protein). This tertiary structure suggests that ABC transporter form a homodimer on plasma membrane and have a function to release a phospholipid from a cell by the flip-flop movement of relative position between monomers (refer to Geoffrey Vhang and Chistopher B. Roth, SCIENCE, Vol 293, pp. 1793).

1JSQ is composed of 8 chains (A chain, B chain, C chain, D chain, E chain, F chain, G chain and H chain) and is registered as 4 combinations of homodimers of A-B chains, C-D chains, E-F chains and G-H chains. In this example, first, a main-chain and a side-chain were constructed for the respective 8 chains only from Cα coordinates by the automatic modeling of the conventional FAMS. From the observation of the eight coordinates constructed up to side-chains, the chemical bond torsional angles ϕ and φ for the main-chain of B chain was the structure that least went into the energetically unstable region on a Ramachandran plot. Next, by the chimera modeling method (refer to T. Yoneda, H. Komooka, H. Umeyama, J. Prot. Chem., 16, 597-605, 1997), using B chain as a basic structure, a monomer structure modeling was performed by partially supplementing with the other chains. Then, the coordinates of B chain monomer were rotated, transferred and superposed on those of A chain and the coordinates after the transfer were defined as a new A chain. The A-B chain pair thus obtained was used in the below-mentioned modeling as a template structure MSBA (modeled reference protein). The amino acid sequence of 1JSQ monomer contains 555 residues (two underlined regions are coordinate deletion and it contains 450 residues if these are excluded), as shown in Fig. 21 (refer to SEQ ID NO:5 in the sequence listing) .

Using this sequence of 555 residues as a target sequence (query) to be referred to, the motif profile of the query was created using PIR database as of November 2001, and IMPALA search (although it is similar to PSI-BLAST, it is a method using the alignment by the Smith and Waterman method: refer to A. A. Schaffe et al., BIOINFORMATICS, 15(12), 1000-1011, 1999) was performed for 774 804 sequences in the protein amino acid sequence databases such as PIR, Swiss Prot, TREMBL, TREMBL_NEW and GenPept (all as of November 2001). As a result of the search under the condition where the E-value is 0.001 or less, alignments with 13705 homologous sequences were obtained.

As an example, a modeling was performed on ID "O93437" in Swiss Prot database. 093437 (target protein) is described as ABC transporter protein in chicken in Swiss-Prot homepage (refer to Edelmann H.M.L., Duchek P., Rosenthal F.E., Foeger N., Glackin C., Kane S.E., Kuchler K., "Cmdrl, a chicken P-glycoprotein, confers multidrug resistance and interacts with Estradiol", Biol. Chem. 380, 231-241, 1999). The number of amino acid residues is 1288 and it is a protein showing multidrug resistance and interaction with Estradiol, which is an estrogenic hormone. In order to perform a modeling of O93437, the result of the alignment with the above-mentioned MSBA is shown in Fig. 22 (refer to SEQ ID Nos: 6 and 7 in the sequence listing).

(Alignment result of "O93437" and MSBA)

| >093437 | 573 | homology | match | mismatch | insertion | deletion |
|---------|-----|----------|-------|----------|-----------|----------|
| >MSBA | 549 | 32.4% | 178 | 370 | 1 | 25 |

However, because MSBA, which is the reference protein, has two large coordinate deletions within it (the regions indicated by underlining in 1JSQ amino acid sequence in Fig. 21), a modeling cannot be performed with the alignment as it is in Fig. 22. Therefore, the character "U" was inserted in the deleted coordinate regions and the alignment was corrected as shown in Fig. 23. This is for describing a protein as if it were composed of three proteins and performing a modeling for plural proteins.

(New alignment result of "O93437" and MSBA)

| >O93437 | 467 | homology | match | mismatch | insertion | deletion |
|---------|-----|----------|-------|----------|-----------|----------|
| >msbA | 444 | 28.2% | 125 | 318 | 1 | 24 |

This becomes as shown in Fig. 24 when it is changed to the input file format for newFAMS.

Although in the alignment in Fig. 24, the protein is handled as a temporary single chain and a protein model is constructed, this input file is insistently for a monomer. The modeling result using this alignment is shown in Fig. 25.

Next, in the case where a model is constructed as a homodimer, the above alignment is further connected with the character "U" and becomes as shown in Fig. 26 below.

The modeling result by newFAMS using the input file format of Fig. 26 is shown in Fig. 27.

The graphic display of the contact region of the above-mentioned homodimer is as shown in Fig. 28.

In this way, even in the case where a coordinate deletion exists, by handling it as temporary plural chains and replacing with the character "U", the calculation of structure, in which a collision of a van der Waals atom is considered, becomes possible and a homodimer model that represents accurately the recognition region of interaction can be constructed. In the observation of the constructed homodimer model "O93437", the region, which is considered as the transmembrane region of the homodimer from an X-ray analysis and where a substance to be transported is mentioned to possibly bind, is presumed to be the region enclosed with a circle in Fig. 29. In this region, a considerable number of hydrophobic amino acid residues exist and an estrogenic hormone estradiol, which is believed to be the function of "O93437", is presumed to be able to bind easily. By the way, the estradiol, which interacts, is composed of a hydrophobic group throughout the molecule as shown in Fig. 30. On the other hand, when each model construction of "O93437" ABC transporter was independently performed using the three-dimensional coordinates of each monomer of the homodimer in which a model construction of a main-chain and a side-chain was performed based only on the Cα atomic coordinates described in the above-mentioned 1JSQ as a reference protein, and the recognition region of interaction was observed, because the interaction between the homodimers was not taken into consideration, interatomic contact within 2.4 Å occurred at 54 locations on the bound surface. As mentioned above, because the dimer contact region is located close to the region where estradiol of a ligand possibly binds, the model construction of the bound region of this homodimer is extremely important for describing the function accurately. This is also considered to indicate the superiority (novelty) or usefulness of the plural chain modeling method in the present invention.

(Example 3) Example of modification of peptide binding to lethal factor protein of anthrax

The tertiary structure of lethal factor (LF, molecular weight of 90, 000) that is the toxin of anthrax, which has made a sensation in these few years, was reported in the November 2001 issue of the British journal Nature (Pannifer et al., NATURE, vol 414, pp. 229-233) at the resolution of 3.90 Å by an X-ray crystallographic analysis. This protein is composed of four domains and is an essential enzyme for the pathogenicity of anthrax, and inhibits one or more signal transduction systems of human cells. Specifically, it has been reported to contact with the N terminal region of a protein family called mitogen-activated protein kinase kinase (MAPKK) and cleave the terminal region. LF is a protease with significantly high specificity. In PDB, LF itself is registered as 1J7N, and the complex of LF and the 16 residues in the N terminal region of a MAPKK family called MAPKK-2 is registered as 1JKY (reference protein). The 16 residues in the N terminal region of MAPKK-2 are cleaved by being caught in a long and deep groove formed with the three domains II, III and IV of LF. Because a drug targeted to this toxin is considered promising, it is possible to design a drug, which is not a peptide, by referring to the tertiary structure of the 16 residues in the N terminal region of MAPKK-2.

In this example, a model of a sequence having a hypothetical mutation in the 16 amino acid residues of MAPKK-2 (target protein) was constructed, and changes of the interaction mode between these models and LF was shown. Generally, drug design focuses on a hydrophobic binding region because drug absorption from the intestinal tract and the like need to be considered. PDB: 1JKY contains A chain (LF) and B chain (16 residues of MAPKK-2) and the amino acid sequences are shown in Fig. 31 (refer to SEQ ID Nos: 8 and 9 in the sequence listing).

In the 16 residues of MAPKK-2 of B chain, there is a spatial gap between the region of the second and the third residues "LA" and LF of A chain (refer to Fig. 32). Therefore, by replacing an amino acid residue in this region with an amino acid residue having a more bulky side-chain and designing such an inhibitor so as to further strengthen the contact by a hydrophobic interaction, the inhibitor binds to the LF in a competitive manner with the 16 residues in the N terminal of MAPKK-2, thereby it may be possible to inhibit the activity of LF as a protease.

A model in which, for example, "FF" is selected as an amino acid residue having the more bulky side-chain instead of "LA" was constructed.

Using the complex (1JKY) of this A chain (LF) and B chain (MAPKK-2) as a template, with the use of the input file format of Fig. 33 (refer to SEQ ID NO:10 in the sequence listing), a model construction was performed by newFAMS. The modeling result is shown in Fig. 34.

As above, an example is shown for the case where a model construction was performed by replacing "LA" with "FF". However, it is possible to analyze other mutants comprehensively using newFAMS and it becomes possible to assume a lead compound of a more effective inhibitor among them.

(Example 4) Verification example of the present invention by comparison of tertiary structure of protein composed of plural chains actually registered in PDB and constructed model (Blind test)

The tertiary structure of the complex protein 2PTC of trypsin, which is a kind of proteinase, and its protein inhibitor, pancreatic trypsin inhibitor (PTI), has been analyzed at the resolution of 1.90 Å by an X-ray crystallographic analysis and registered (refer to Marquart, M., Walter, J., Deisenhofer, J. , Bode, W. , Huber, R. : The Geometry of the Reactive Site and of the Peptide Groups in Trypsin, Trypsinogen and its Complexes with Inhibitors, Acta Crystallogr., Sect. B 39, 480, 1983). 2PTC has been registered as a complex composed of E chain of trypsin and I chain of PTI. Trypsin, which is a serine protease, is secreted from pancreas as an inactive trypsinogen and becomes an active form trypsin by an enzyme, Enterokinase, contained in duodenal juice. Trypsin is a kind of endpeptidase and a protein digesting enzyme which cleaves a peptide bond at the carboxyl group side of a basic amino acid such as Arg or Lys.

On the other hand, the tertiary structure of the complex protein 1BTH of thrombin E192Q enzyme, which is a kind of serine protease (glutamic acid 192 is replaced with glutamine) and PTI has been analyzed at the resolution of 2.3 Å by an X-ray crystallographic analysis and is registered (refer to van de Locht, A., Bode, W., Huber, R., Le Bonniec, B. F., Stone, S. R. , Esmon, C. T. , Stubbs, M. T., "The thrombin E192Q-BPTI complex reveals gross structural rearrangements: implications for the interaction with antithrombin and thrombomodulin" EMBO J. 16, 2977, 1997). This protein has been registered as the form of two sets of homodimers, thrombin E192Q composed of L chain and H chain and PTI composed of only P chain, and similarly, thrombin E192Q composed of J chain and K chain and PTI composed of only Qchain. Here, the former set of the protein complex is focused. Thrombin is an active form protein of prothrombin, which is one of the blood coagulation factors, and by forming this thrombin, fibrinogen becomes fibrin and blood coagulation is caused.

In this example, a modeling of H chain and P chain of 1BTH was performed from E chain and I chain of 2PTC using newFAMS, which is the software of the present invention, and a comparison was made with the X-ray structure of 1BTH actually registered in PDB (Protein Data Bank). However, 1BTH itself is not included in the database of Cα atoms and main-chain atoms used for the construction of the inserted loop of 1BTH model, and it was confirmed that a database including the structure of the right answer was not used in the verification of the model accuracy. Further, a comparison with the modeling of each 1BTH_H and 1BTH_E using the conventional FAMS is shown to verify the accuracy of newFAMS. What should be noted here is that, in H chain of 1BTH, the 192nd amino acid residue of thrombin is replaced from a glutamic acid to a glutamine. The 192nd amino acid residue of thrombin is glutamic acid, and generally the loop region of the enzyme sterically hinders to bind to PTI. However, if the 192nd amino acid residue is replaced with a glutamine (thrombin E192Q), when binding to PTI, the above-mentioned hindered loop region moves by induced-fit adaptation being affected by PTI, the both proteins bind to each other. This is the complex protein registered as the name of 1BTH.

The amino acid sequences of H chain and P chain of 1BTH, which is the target protein, are as shown in Fig. 35 (refer to SEQ ID Nos:11 and 12 of the sequence listing). The numbers of amino acid residue are 254 for H chain and 58 for P chain in 1BTH.

Oh the other hand, the amino acid sequences of E chain and I chain of 2PTC, which is the reference protein, are as shown in Fig. 36 (refer to SEQ ID NOs:12 and 13 of the sequence listing). The numbers of amino acid residues are 223 for E chain and 58 for I chain in 2PTC.

The alignment of H chain of 1BTH and E chain of 2PTC is as shown in Fig. 37. The homology was 43.5%.

(Alignment result of H chain of 1BTH and E chain of 2PTC)

| | | homology | match | mismatch | insertion | deletion |
|---|---|---|---|---|---|---|
| >1BTH_H | 254 | | | | | |
| >2PTC_E | 223 | 43.5% | 97 | 123 | 3 | 34 |

The alignment of P chain of 1BTH and I chain of 2PTC is as shown in Fig. 38. The homology was 100%.

(Alignment result of P chain of 1BTH and I chain of 2PTC)

| | | homology | match | mismatch | insertion | deletion |
|---|---|---|---|---|---|---|
| >1BTH_P | 58 | | | | | |
| >2PTC_1 | 58 | 100.0% | 58 | 0 | 0 | 0 |

The input file format of each alignment result for the conventional FAMS is as shown in Fig. 39.

The input file format for newFAMS this time is as shown in Fig. 40 by connecting the above-mentioned alignments with the character "U".

As a result of the model construction only for H chain and P chain by newFAMS this time, the r.m.s.d. value of 1BTH actually registered in PDB and the 1BTH model obtained this time using newFAMS became 2.11 Å on the whole, including both H chain and P chain. Further, ther.m.s.d. value of 1BTH actually registered in PDB and the 1BTH model using the conventional FAMS was 2.18 Å on the whole. It is recognized that the r.m.s.d. value is slightly improved.

Next, it was verified whether the atoms of amino acids in a model by the conventional FAMS collide each other and the contact of amino acid residues less than 2.4 Å exists on the contact surface of H chain and P chain. As a result, the contact between atoms occurred at 7 locations between H chain and P chain molecules.

On the other hand, when it was observed whether a collision between atoms of amino acids of H chain and P chain in the model occurred by newFAMS, which is the software usable in the present invention this time, there was no collision for 2.4 Å or less.

As shown above, atoms collide each other in the conventional FAMS, however no collision between atoms could not be found in newFAMS this time. H chain of 1BTH, which is a serine protease, has a catalytic site, which is an

## EP 1 471 443 B1

active site, and a substance binding site in the contact region with P chain, and for the accurate description of function, the model construction of the contact region of these H chain and P chain needs to be performed precisely. In this example, an X-ray analysis was available, and being compared with it, the highly accurate construction of a temporary single chain model of plural chains was demonstrated. This indicates, again, the superiority (novelty) of newFAMS.

(Example 5)

An interface could be developed, which was designed to enable to simply and easily access the objective protein without a preliminary knowledge by conjunction search such as partial agreement of an arbitrary symbol specific to living species, a protein code name, a reference protein name, a character string of an about one-line function description for a protein, which is desired to be browsed. In Fig. 43, as an example, an interface screen for a tertiary structure model database constructed based on an alignment that can be browsed in GTOP (a home page for alignment between amino acid sequences of genome sequences publicized by National Institute of Genetics and amino acid sequences in tertiary structure database PDB) is shown. This is an interface designed to enable to simply and easily access the objective protein without a preliminary knowledge by conjunction search such as partial agreement of an arbitrary symbol specific to living species, a protein code name, a reference protein name, a character string of an about one-line function description, in the case where the tertiary structure of a protein, which is desired to be browsed, based on three-dimensional coordinates is in the above mentioned tertiary structure database of a protein of a single chain or plural chains.

In the case of this example, symbols for 41 living species, such as aero and aful, are based on the names in GTOP (as of September 2001). When there is a protein, which is desired to be browsed, according to a tertiary structure, namely a three-dimensional coordinate, a checkbox next to the symbol of the living species is checked. Further, it has a search refinement function by conjunction, such as a protein code name, a reference protein name, and an about one-line function description.

Advantages of the Invention

According to the present invention, with respect to the prediction of a tertiary structure of a protein (including a gene encoding the protein) composed of plural chains with an unknown tertiary structure or the prediction of a tertiary structure of a conj ugated protein, in which an amino acid residue of each component of the plural chains is mutated, highly reliable information of the tertiary structure of a protein including a peptide, can be obtained more efficiently and simply than by a conventional method. As a result, in the case where an interesting gene or protein is found by a genome sequence analysis, an expression profiling analysis using a DNA chip, a proteome analysis or the like, it is possible to predict a function based on the tertiary structure of the protein. Accompanied by this, it is possible to modify the peptide or the protein efficiently. In addition, by predicting the functional region of the protein, information for designing a new drug of a protein or a low molecular compound can be obtained more efficiently or effectively than by the conventional method.

Industrial Applicability

As mentioned above, the method of the present invention is considered extremely useful in a field in which an analysis of biological information is performed (bioinformatics) for mainly molecular design or the like of medical and agricultural chemicals. The method can extend an applicable range of a protein automatic modeling system applicable to an amino acid sequence composed of a single chain to a protein composed of plural chains, and also can diversely modify a ligand molecule, a receptor molecule, an enzyme or the like. Therefore, the usefulness of the invention is expected to further increase.

The present invention can be widely implemented in many industrial fields, in particular, fields such as drugs, foods, cosmetics, medical services, and structure analyses, therefore, it is extremely useful.

## Claims

1. A method of constructing a tertiary structure of a protein composed of plural chains having given arbitrary amino acid sequences by extending an comparative modeling method of constructing a tertiary structure of a protein composed of a single chain having a given arbitrary amino acid sequence (extended modeling method), said method comprising the steps of:

   correcting an input file format of the plural chains in a computer software program so as to present a form of a temporary single chain (correction of sequence alignment), and
   constructing the tertiary structure based on the modeling method while assuming that the structure has plural chains in calculation of a potential formula by the computer software program;

   wherein said correction comprises selecting amino acid sequences of a reference protein composed of the same number of plural polypeptide chains as a target protein and adding a delimiter to the C terminal end of the amino acid sequences of the polypeptide chains, whereby enabling to handle the respective proteins as proteins composed of a single chain (correction of sequence alignment);
   and wherein the method additionally comprises the steps of:

   performing construction of $C\alpha$ atomic coordinates and main-chain atomic coordinates based on a tertiary structure database and/or a database obtained by modifying the tertiary structure database so as to avoid duplication of similar structures, by determining the terminal residue number of each protein chain from the temporary single chain after the correction, disconnecting a chemical bond potential and a chemical bond angle potential at a border of the terminal residue, and adding an interatomic interaction potential at the border, and
   performing minimization (optimization) of an objective function representing a temporary energy value by at least one of a simulated annealing method, a molecular dynamics calculation and a Monte Carlo method.

2. The method according to claim 1, wherein said comparative modeling method is a homology modeling method or a threading method.

3. The method according to claim 1, which is a fully automated construction method or a manual construction method.

4. The method according to claim 1, comprising a method of searching the reference protein from a tertiary structure database and performing sequence alignment between a target sequence and the amino acid sequence of the reference protein.

5. The method according to claim 4, wherein a software for searching the reference protein and outputting an alignment is at least one of FAMS, FASTA, PSI-BLAST, LIBRA, RPS-BLAST, IMPALA, ClustalW, HMMER and BIOCES.

6. The method according to claim 1 or 4, wherein the corrected sequence alignment is treatable to a multiple alignment, in which the amino acid sequences of the same type or a different type of reference protein is written, by using a file having a format in which an amino acid sequence of each polypeptide chain has the delimiter at the C terminal end of the amino acid sequence, and specifying a reference protein ID for each alignment section delimited by the delimiter, whereby enabling to obtain an average structure by superposing the sequences.

7. The method according to any one of claims 1 to 6, wherein at least two chains among the plural chains constituting the target protein are polypeptide chains, and a data set of a modification whose similarity is superior or inferior is created with the use of a potential energy value as an index and based on a possible combination of 20 amino acids for each amino acid residue located at a mutual protein-protein recognition region, whereby enabling to construct the tertiary structure of the at least two polypeptide chains whose functions as respective proteins are increased or decreased.

8. The method according to any one of claims 1 to 7, wherein, in a case where at least one chain among the plural chains constituting the target protein is an amino acid derivative or a peptide derivative (peptide ligand), and has a similar chemical structure to a corresponding ligand molecule in the reference protein, the alignment, in which the derivative of the target protein is defined as a new residue name and a one-character code and the ligand of the reference protein is defined as another new residue name and a one-character code, is created manually or automatically, and based on the possible combination of 20 amino acids and their derivatives for each residue constituting the ligand sequences, a rank is placed in the ascending order of the potential energy value, whereby enabling to construct a ligand model data set of the amino acid derivatives or the peptide derivatives, in which the plural higher-ranked sequences are stored as modifications with superior similarity to a binding region of a receptor protein.

9. The method according to any one of claims 1 to 8, wherein at least one component of the plural chains constituting the target protein is a peptide ligand, the amino acid sequence of the ligand is fixed, and based on the possible combination of 20 amino acids for each amino acid residue located at the region recognizing the ligand, a data set of a modification with superior similarity to a binding region of the plural higher-ranked receptor proteins is created with the use of the potential energy value as an index, whereby enabling to construct a tertiary structure of various ligand receptor proteins that can bind to the ligand.

10. The method according to any one of claims 1 to 9, wherein said plural chains are domains or modules into which a single chain peptide is divided, and are capable of being restored to a single temporary chain.

11. The method according to any one of claims 1 to 10, wherein said target protein contains a substance, which is not included in any of a common amino acid or a peptide in which the plural common amino acids are bound, and the substance is registered in PDB or a database obtained by modifying the PDB.

12. The method according to any one of claims 1 to 6 comprising the steps of:

   searching the reference protein which is appropriate for the target sequence from the tertiary structure database and performing the sequence alignment with the amino acid sequences of the searched plural reference proteins; selecting the amino acid sequence of the reference protein whose E-value is small for the target sequence; and adding the delimiter to the terminal end of each amino acid sequence of the chain included in the reference protein and also adding the delimiter to the corresponding position to the target sequence (correction of sequence alignment).

13. The method according to claim 12, further comprising the steps of:

   obtaining coordinates from a reference structure determined in the step of selecting the amino acid sequence of the reference protein for a $C\alpha$ atom which is one of the constitutive atoms in an amino acid of the target sequence based on the alignment information and optimizing the $C\alpha$ atomic coordinates;

adding a main-chain atomic coordinates from the tertiary structure database to the obtained $C\alpha$ atomic coordinate and optimizing the main-chain atomic coordinates; and

adding a side-chain atomic coordinates from the tertiary structure database to the obtained main-chain atomic coordinates and optimizing the side-chain atomic coordinates.

**14.** The method according to claim 1, wherein said potential formula includes the following content:

in the potential formula when the total chain number = M, wherein N represents the protein chain number, kN represents the serial number of the C terminal residue in the N-th protein chain, and i=1, ..., M-1 is simplistically described as i=1, M-1,

(A) in the calculation for construction and optimization steps of the $C\alpha$ atomic coordinates, a case where i=kN(N=1,M-1) of a temporary chemical bond potential is not included and cases where i=kN(N=1,M-1), i=kN(N=1,M-1)+1 of a temporary chemical bond angle potential are not included, and, in the case of an interatomic interaction potential, j>i+1 is added if i=kN-1, and j>i is added if i=kN respectively,

(B) in the calculation for construction and optimization steps of the main-chain atomic coordinates, a bond between Ci and Ni+1 is not included in a chemical bond potential when i=kN(N=1,M-1), angles $C\alpha$i-Ci-Ni+1, Oi-Ci-Ni+1 and Ci-Ni+1-$C\alpha$i+1 when i=kN(N=1,M-1) wherein C and O represent a carbon atom and an oxygen atom in a carbonyl respectively, $C\alpha$ represents an $\alpha$ carbon atom and N represents a nitrogen atom are not included in a chemical bond angle potential, further, angles Ni-$C\alpha$i-Ci-Ni+1, $C\alpha$i-Ci-Ni+1-$C\alpha$i+1, and Ci-Ni+1-$C\alpha$i+1-Ci+1 when i=kN(N=1,M-1) are not included in a chemical bond torsional angle potential, in addition, for an interatomic interaction potential, wherein the length between atoms is represented by r, a case of rip $\leq$ a specified value for rij $\in$ {rNi, Ni+1; r$C\alpha$i, Ni+1; r$C\alpha$i, $C\alpha$i+1; rCi, Ni+1; rCi, $C\alpha$i+1; rCi, C$\beta$i+1; rCi, Ci+1; rOi, Ni+1; rOi, $C\alpha$i+1} when i=kN(N=1,M-1) is added.

**15.** The method according to claim 8, wherein said amino acid derivative is a non-natural amino acid such as $\beta$Asp or $\gamma$Glu or a derivative thereof.

**16.** A computer program which when run on a computer executes the steps of the method according to any one of claims 1 to 14.

**17.** The method according to claim 1, wherein said target protein composed of plural chains includes one or more polypeptide chains.

**18.** The method according to claim 17, wherein at least one chain among the plural chains is selected from the group consisting of natural or non-natural amino acids and amino acid derivatives such as their derivatives, peptide derivatives, pharmaceutical substances, nucleic acids, saccharides, organic metal compounds, metal oxides and their ions, metals and their ions.

**19.** The method according to claim 1, comprising the steps of, for the target protein and the selected reference protein, assuming each amino acid sequence of the plural chains included in the target protein and the selected reference protein respectively as a single chain in a state where the N terminal ends and the C terminal ends are bound sequentially, performing sequence alignment between the reference sequence of the thus obtained temporary single chain and the target sequence of the thus obtained temporary single chain to confirm their correspondence relationship, locating a $C\alpha$ atom, which is one of the constitutive atoms in the amino acid residue in the target sequence, binding the $C\alpha$ atoms by an amide bond, further adding a side-chain to construct coordinates for other constitutive atoms, performing optimization, and constructing the tertiary structure of the target protein by the modeling method.

**20.** The method according to claim 1 or 19, wherein the construction of the tertiary structure of the target protein is performed by obtaining the coordinates from the tertiary structure of the reference protein selected for the $C\alpha$ atom in a main-chain amino acid in the target protein based on the obtained alignment information, optimizing the $C\alpha$ atomic coordinates so as to minimize an objective function, adding coordinates of other atoms (including a $C\beta$ atomic coordinates) of the main-chain to the optimized $C\alpha$ atomic coordinates, optimizing the main-chain atomic coordinates so as to minimize the objective function, adding coordinates of other atoms of the side-chain to the optimized main-chain atomic coordinates, and optimizing the side-chain atomic coordinates so as to minimize the objective function.

**Patentansprüche**

1. Verfahren zur Erstellung einer Tertiärstruktur eines Proteins, das aus mehreren Ketten mit gegebenen willkürlichen Aminosäuresequenzen besteht, durch Erweiterung eines komparativen Modellierungsverfahrens zur Erstellung einer Tertiärstruktur eines Proteins, das aus einer einzelnen Kette mit einer gegebenen Aminosäuresequenz besteht (erweitertes Modellierungsverfahren),
wobei das Verfahren folgende Schritte umfasst:

Korrigieren eines Eingabedateiformats der mehreren Ketten in einem Computersoftwareprogramm, um eine Form einer temporären einzelnen Kette bereitzustellen (Korrektur der Sequenzanordnung), und
Erstellen der Tertiärstruktur basierend auf dem Modellierungsverfahren unter der Annahme, dass die Struktur mehrere Ketten aufweist, indem eine potenzielle Formel durch das Computersoftwareprogramm berechnet wird;

wobei das Korrigieren das Auswählen von Aminosäuresequenzen eines Bezugsproteins, das aus der gleichen Anzahl an mehreren Polypeptidketten besteht wie das Zielprotein, und das Hinzufügen eines Begrenzers zum C-terminalen Ende der Aminosäuresequenzen der Polypeptidketten umfasst, wodurch die jeweiligen Proteine als Proteine aus einer einzigen Ketten gehandhabt werden können (Korrektur der Sequenzanordnung);
und wobei das Verfahren weiters folgende Schritte umfasst:

Durchführen der Erstellung von Cα-Atomkoordinaten und Hauptketten-Atomkoordinaten basierend auf einer Tertiärstruktur-Datenbank und/oder einer Datenbank, die durch Modifizieren der Tertiärstruktur-Datenbank erhalten wurde, um eine Verdoppelung von ähnlichen Strukturen zu vermeiden, indem die Anzahl an terminalen Resten jeder Proteinkette aus der temporären einzelnen Kette nach der Korrektur bestimmt wird,
Abtrennen eines chemischen Bindungspotenzials und eines chemischen Bindungswinkelpotenzials am Rand des terminalen Restes und Hinzufügen eines interatomaren Wechselwirkungspotenzials am Rand und
Durchführen der Minimierung (Optimierung) einer objektiven Funktion, die einen temporären Energiewert darstellt, durch zumindest eines aus einem simulierten Annealing-Verfahren, einer Berechnung der molekularen Dynamik und einem Monte-Carlo-Verfahren.

2. Verfahren nach Anspruch 1, wobei das komparative Modellierungsverfahren ein Homologiemodellierungsverfahren oder ein Threading-Verfahren ist.

3. Verfahren nach Anspruch 1, das ein vollständig automatisiertes Erstellungsverfahren oder ein manuelles Erstellungsverfahren ist.

4. Verfahren nach Anspruch 1, das ein Verfahren zur Suche nach dem Bezugsprotein in einer Tertiärstruktur-Datenbank und das Durchführen einer Sequenzanordnung zwischen einer Zielsequenz und der Aminosäuresequenz des Bezugsproteins umfasst.

5. Verfahren nach Anspruch 4, wobei die Software zur Suche nach dem Bezugsprotein und Ausgabe einer Anordnung zumindest eines aus FAMS, FASTA, PSI-BLAST, LIBRA, RPS-BLAST, IMPALA, ClustalW, HMMER und BIOCES ist.

6. Verfahren nach einem der Ansprüche 1 bis 4, wobei die korrigierte Sequenzanordnung einer mehrfachen Anordnung unterzogen werden kann, in der Aminosäuresequenzen des Bezugsproteins derselben Art oder unterschiedlicher Arten enthalten sind, unter Verwendung einer Datei mit einem Format, in dem eine Aminosäuresequenz jeder Polypeptidkette einen Begrenzer am C-terminalen Ende der Aminosäuresequenz aufweist, und Spezifizieren einer Bezugsprotein-ID für jeden Anordnungsabschnitt, der durch einen Begrenzer begrenzt ist, wodurch durch Übereinanderlegen der Sequenzen eine mittlere Struktur erhalten werden kann.

7. Verfahren nach einem der Ansprüche 1 bis 6, worin zumindest zwei Ketten von den mehreren Ketten, aus denen das Zielprotein besteht, Polypeptidketten sind und ein Datensatz einer Modifikation, deren Ähnlichkeit größer oder kleiner ist, erzeugt wird, und zwar unter Verwendung eines potenziellen Energiewerts als Index und basierend auf einer möglichen Kombination aus 20 Aminosäuren für jeden Aminosäurerest an einer gegenseitigen Protein-Protein-Erkennungsstelle, wodurch die Erstellung der Tertiärstruktur der zumindest zwei Polypeptidketten ermöglicht wird, deren Funktionen als jeweilige Proteine gesteigert oder verringert wird.

8. Verfahren nach einem der Ansprüche 1 bis 7, worin, wenn zumindest eine Kette von den mehreren Ketten, aus denen das Zielprotein besteht, ein Aminosäurederivat oder ein Peptidderivat (Peptidligand) ist und eine ähnliche chemische Struktur aufweist wie ein entsprechendes Ligandenmolekül im Bezugsprotein, die Anordnung, in der das Derivat des Zielproteins als neuer Restname und als Einbuchstabencode definiert ist und der Ligand des Bezugsproteins als ein weiterer neuer Restname und Einbuchstabencode definiert ist, manuell oder automatisch durchgeführt wird und basierend auf der möglichen Kombination aus 20 Aminosäuren und ihren Derivaten jedem Rest der Ligandensequenzen eine Position in der aufsteigenden Reihenfolge des potenziellen Energiewerts zugeordnet wird, wodurch die Erstellung eines Ligandenmodell-Datensatzes der Aminosäurederivate oder Peptidderivate ermöglicht wird, in dem die mehreren höher eingeordneten Sequenzen als Modifikationen mit größerer Ähnlichkeit mit einer Bindungsregion eines Rezeptorproteins gespeichert sind.

9. Verfahren nach einem der Ansprüche 1 bis 8, wobei zumindest ein Bestandteil der mehreren Ketten, aus denen das Zielprotein besteht, ein Peptidligand ist, die Aminosäuresequenz des Liganden fixiert ist und, basierend auf der möglichen Kombination aus 20 Aminosäuren für jeden Aminosäurerest an der den Liganden erkennenden Region, ein Datensatz einer Modifikation mit größerer Ähnlichkeit zu einer Bindungsregion der mehreren höher eingeordneten Rezeptorproteine geschaffen wird, unter Verwendung des potenziellen Energiewerts als Index, wodurch die Erstellung einer Tertiärstruktur verschiedener Ligandenrezeptorproteine ermöglicht wird, die an den Liganden binden können.

10. Verfahren nach einem der Ansprüche 1 bis 9, wobei die mehreren Ketten Domänen oder Module sind, in die ein Einzelkettenpeptid unterteilt wird und die zu einer einzelnen temporären Kette wiederhergestellt werden können.

11. Verfahren nach einem der Ansprüche 1 bis 10, wobei das Zielprotein eine Substanz enthält, die in keiner herkömmlichen Aminosäure oder keinem herkömmlichen Peptid, in dem die mehreren herkömmlichen Aminosäuren gebunden sind, enthalten ist, und die Substanz im PDB oder einer Datenbank, die durch Modifikation der PDB erhalten ist, registriert ist.

12. Verfahren nach einem der Ansprüche 1 bis 6, das die folgenden Schritte umfasst:

Suchen des Bezugsproteins, das für die Zielsequenz geeignet ist, in der Tertiärstruktur-Datenbank und Durchführen der Sequenzanordnung mit den Aminosäuresequenzen der gesuchten mehreren Bezugsproteine;
Auswählen der Aminosäuresequenz des Bezugsproteins, deren E-Wert bezüglich der Zielsequenz gering ist; und
Hinzufügen des Begrenzers zum terminalen Ende jeder Aminosäuresequenz der Kette im Bezugsprotein und außerdem Hinzufügen des Begrenzers zur entsprechenden Position der Zielsequenz (Korrektur der Sequenzanordnung).

13. Verfahren nach Anspruch 12, das weiters folgende Schritte umfasst:

Erhalten von Koordinaten aus einer Bezugsstruktur, die im Schritt des Auswählens der Aminosäuresequenz des Bezugsproteins bestimmt wurde, für das C$\alpha$-Atom, das eines der konstitutiven Atome in einer Aminosäure der Zielsequenz ist, basierend auf den Anordnungsinformationen, und Optimieren der C$\alpha$-Atomkoordinaten;
Hinzufügen von Hauptketten-Atomkoordinaten aus der Tertiärstruktur-Datenbank zur erhaltenen C$\alpha$-Atomko-ordinate und Optimieren der Hauptketten-Atomkoordinaten; und
Hinzufügen von Seitenketten-Atomkoordinaten aus der Tertiärstruktur-Datenbank zu den erhaltenen Hauptketten-Atomkoordinaten und Optimieren der Seitenketten-Atomkoordinaten.

14. Verfahren nach Anspruch 1, wobei die potenzielle Formel folgenden Inhalt umfasst:

in der potenziellen Formel, wenn die Gesamtkettenzahl = M ist, worin N für die Proteinkettenzahl steht, kN für die Seriennummer des C-terminalen Rests in der N-ten Proteinkette steht und i=1, ..., M-1 vereinfachend als i=1, M-1 beschrieben wird,

(A) bei der Berechnung der Erstellungs- und Optimierungsschritte der C$\alpha$-Atomkoordinaten, wenn i=kN (N=1, M-1) eines temporären chemischen Bindungspotenzials nicht eingeschlossen ist und wenn i=kN (N=1, M-1), i=kN (N=1, M-1) + 1 eines temporären chemischen Bindungswinkels nicht eingeschlossen sind, und im Falle eines interatomaren Wechselwirkungspotenzials, j>i+1 hinzugefügt wird, wenn i=kN-1 ist, bzw. j>i hinzugefügt wird, wenn i=kN ist,
(B) bei der Berechnung der Erstellungs- und Optimierungsschritte der Hauptketten-Atomkoordinaten eine Bindung zwischen Ci und Ni+1 nicht in einem chemischen Bindungspotenzial enthalten ist, wenn i=kN (N=1, M-1) ist, Winkel Cαi-Ci-Ni+1, Oi-Ci-Ni+1 und Ci-Ni+1-Cαi+1, wenn i=kN (N=1, M-1) ist, worin C und O für ein Kohlenstoffatom bzw. ein Sauerstoffatom in einem Carbonyl stehen, Cα für ein α-Kohlenstoffatom steht und N für ein Stickstoffatom steht, nicht in einem chemischen Bindungswinkelpotenzial eingeschlossen sind, weiters Winkel Ni-Cαi-Ci-Ni+1, Cai-Ci-Ni+1-Cai+1 und Ci-Ni+1-Cαi+1-Ci+1, wenn i=kN (N=1, M-1) ist, nicht in einem chemischen Bindungstorsionswinkelpotenzial enthalten sind, und außerdem, für ein interatomares Wechselwirkungspotenzial, worin die Länge zwischen Atomen durch r dargestellt ist, im Fall von als rij ≤ a ein spezifizierter Wert für rij ∈ {rNi, Ni+1; rCαi,Ni+1; rCαi,Cαi+1; rCi,Ni+1; rCi,Cαi+1; rCi, Cβi+1; rCi,Ci+1; rOi,Ni+1; rOi,Cαi+1} hinzugefügt wird, wenn i=kN (N=1,M-1) ist.

15. Verfahren nach Anspruch 8, wobei das Aminosäurederivat eine nichtnatürliche Aminosäure ist, wie z.B. βAsp oder γGlu oder ein Derivat davon.

16. Computerprogramm, das, wenn es auf einem Computer laufen gelassen wird, die Schritte eines Verfahrens nach einem der Ansprüche 1 bis 14 ausführt.

17. Verfahren nach Anspruch 1, wobei das Zielprotein, das aus mehreren Ketten besteht, eine oder mehrere Polypeptidketten umfasst.

18. Verfahren nach Anspruch 17, wobei zumindest eine Kette von den mehreren Ketten aus der Gruppe ausgewählt ist, die aus natürlichen oder nichtnatürlichen Aminosäuren und Aminosäurederivaten, z.B. ihren Derivaten, Peptidderivaten, pharmazeutischen Substanzen Nucleinsäuren, Sacchariden, organischen Metallverbindungen, Metalloxiden und ihren Ionen, Metallen und ihren Ionen, besteht.

19. Verfahren nach Anspruch 1, das die Schritte des Annehmens, für das Zielprotein und das ausgewählte Bezugsprotein, jeder Aminosäuresequenz der mehreren Ketten im Zielprotein bzw. im ausgewählten Bezugsprotein als einzelne Kette in einem Zustand, in dem die N-terminalen Enden und die C-terminalen Enden sequenziell gebunden sind, Durchführens einer Sequenzanordnung zwischen der Bezugssequenz der so erhaltenen temporären einzelnen Kette und der Zielsequenz der so erhaltenen temporären einzelnen Kette, um ihre Übereinstimmungsbeziehung zu bestätigen, Lokalisierens eines Cα-Atoms, das eines der konstitutiven Atome im Aminosäurerest in der Zielsequenz ist, Bindens der Cα-Atome durch eine Amidbindung, weiters des Hinzufügens einer Seitenkette, um Koordinaten für weitere konstitutive Atome zu erstellen, des Durchführens einer Optimierung und des Erstellens der Tertiärstruktur des Zielproteins durch das Modellierungsverfahren umfasst.

20. Verfahren nach einem der Ansprüche 1 bis 19, wobei die Erstellung der Tertiärstruktur des Zielproteins durch Erhalten der Koordinaten aus der Tertiärstruktur des ausgewählten Bezugsproteins für das Cα-Atom in einer Hauptketten-Aminosäure im Zielprotein basierend auf den erhaltenen Anordnungsinformationen, Optimieren der Cα-Atomkoordination, um eine objektive Funktion zu minimieren, Hinzufügen der Koordinaten von anderen Atomen (einschließlich Cβ-Atomkoordinaten) der Hauptkette zu den optimierten Cα-Atomkoordinaten, Optimieren der Hauptketten-Atomkoordinaten, um die objektive Funktion zu minimieren, Hinzufügen der Koordinaten anderer Atome der Seitenkette zu den optimierten Hauptketten-Atomkoordinaten und Optimieren der Seitenketten-Atomkoordinaten, um die objektive Funktion zu minimieren, erfolgt.

## Revendications

1. Procédé pour construire une structure tertiaire d'une protéine composée de plusieurs chaînes ayant des séquences d'acides aminés arbitraires données par extension d'un procédé de modélisation comparative pour construire une structure tertiaire d'une protéine composée d'une seule chaîne ayant une séquence d'acides aminés arbitraire donnée (procédé de modélisation étendu),

ledit procédé comprenant les étapes consistant à:

corriger un format de fichier d'entrée des plusieurs chaînes dans un programme de logiciel informatique de sorte à présenter une forme de chaîne unique temporaire (correction d'un alignement de séquences), et construire la structure tertiaire en se basant sur le procédé de modélisation tout en supposant que la structure possède plusieurs chaînes lors du calcul d'une formule potentielle par le programme du logiciel informatique; où ladite correction consiste à sélectionner les séquences d'acides aminés d'une protéine de référence composée du même nombre des plusieurs chaînes polypeptidiques en tant que protéine cible, et à ajouter un délimiteur à l'extrémité C-terminale des séquences d'acides aminés des chaînes polypeptidiques, ce qui permet ainsi de manipuler les protéines respectives en tant que protéines composées d'une seule chaîne (correction d'un alignement de séquences);

et où le procédé comprend en outre les étapes consistant à:

réaliser la construction des coordonnées atomiques de C$\alpha$ et des coordonnées atomiques de la chaîne principale en se basant sur une base de données de structures tertiaires et/ou une base de données obtenue en modifiant la base de données de structures tertiaires de sorte à éviter la duplication de structures similaires, en déterminant le numéro du résidu terminal de chaque chaîne protéique à partir de la chaîne unique temporaire après la correction,

déconnecter un potentiel de liaison chimique et un potentiel d'angle de liaison chimique à la frontière du résidu terminal, et ajouter un potentiel d'interaction interatomique au niveau de la frontière, et

réaliser une minimisation (optimisation) d'une fonction objective représentant une valeur d'énergie temporaire par au moins un procédé parmi un procédé d'annelage simulé, un calcul de dynamique moléculaire et une méthode de Monte-Carlo.

2. Procédé selon la revendication 1, dans lequel ledit procédé de modélisation comparative est un procédé de modélisation par homologie ou un procédé de reconnaissance de repliements.

3. Procédé selon la revendication 1, qui est un procédé de construction entièrement automatisé ou un procédé de construction manuel.

4. Procédé selon la revendication 1, qui comprend un procédé pour rechercher la protéine de référence à partir d'une base de données de structures tertiaires et à réaliser un alignement de séquences entre une séquence cible et la séquence d'acides aminés de la protéine de référence.

5. Procédé selon la revendication 4, dans lequel un logiciel pour rechercher la protéine de référence et générer un alignement est au moins un logiciel parmi FAMS, FASTA, PSI-BLAST, LIBRA, RPS-BLAST, IMPALA, ClustalW, HMMER et BIOCES.

6. Procédé selon la revendication 1 ou 4, dans lequel l'alignement de séquences corrigé peut être traité comme un alignement multiple, dans lequel les séquences d'acides aminés d'un même type ou d'un type différent de protéine de référence sont écrites, en utilisant un fichier ayant un format dans lequel une séquence d'acides aminés de chaque chaîne polypeptidique comporte le délimiteur au niveau de l'extrémité C-terminale de la séquence d'acides aminés, et en spécifiant une ID de protéine de référence pour chaque section de l'alignement délimitée par le délimiteur, ce qui permet ainsi d'obtenir une structure moyenne en superposant les séquences.

7. Procédé selon l'une quelconque des revendications 1 à 6, dans lequel au moins deux chaînes parmi les plusieurs chaînes constituant la protéine cible sont des chaînes polypeptidiques, et un ensemble de données d'une modification dont la similarité est supérieure ou inférieure est créé en utilisant une valeur d'énergie potentielle en tant qu'index et en se basant sur une combinaison possible de 20 acides aminés pour chaque résidu d'acide aminé situé au niveau de la région de reconnaissance mutuelle protéine-protéine, ce qui permet ainsi de construire la structure tertiaire des au moins deux chaînes polypeptidiques dont les fonctions, en tant que protéines respectives, sont augmentées ou réduites.

8. Procédé selon l'une quelconque des revendications 1 à 7, dans lequel, dans un cas où au moins une chaîne parmi les plusieurs chaînes constituant la protéine cible est un dérivé d'acide aminé ou un dérivé peptidique (ligand peptidique) et possède une structure chimique similaire à une molécule de ligand correspondante dans la protéine de référence, l'alignement, dans lequel le dérivé de la protéine cible est défini par un nouveau nom de résidu et un code à un seul caractère et le ligand de la protéine de référence est défini par un autre nouveau nom de résidu et un code à un seul caractère, est créé manuellement ou automatiquement et, en se basant sur la possible combinaison de 20 acides aminés et de leurs dérivés pour chaque résidu constituant les séquences de ligand, un classement est établi dans l'ordre ascendant de la valeur d'énergie potentielle, ce qui permet de construire un ensemble de données de modèle de ligand des dérivés d'acides aminés ou des dérivés peptidiques, dans lequel les plusieurs séquences les mieux classées sont stockées en tant que modifications présentant une similarité supérieure avec une région de liaison d'une protéine réceptrice.

9. Procédé selon l'une quelconque des revendications 1 à 8, dans lequel au moins un composant des plusieurs chaînes constituant la protéine cible est un ligand peptidique, la séquence d'acides aminés du ligand est fixe et, en se basant sur la possible combinaison de 20 acides aminés pour chaque résidu d'acide aminé situé au niveau de la région reconnaissant le ligand, un ensemble de données d'une modification présentant une similarité supérieure avec une région de liaison des plusieurs protéines réceptrices les mieux classées est créé en utilisant la valeur d'énergie potentielle en tant qu'index, ce qui permet ainsi de construire une structure tertiaire de diverses protéines réceptrices de ligand qui peuvent se lier au ligand.

10. Procédé selon l'une quelconque des revendications 1 à 9, dans lequel lesdites plusieurs chaînes sont des domaines ou des modules dans lesquels un peptide simple chaîne est divisé, et sont capables d'être restaurés en une seule chaîne temporaire.

11. Procédé selon l'une quelconque des revendications 1 à 10, dans lequel ladite protéine cible contient une substance qui n'est pas incluse dans l'un quelconque parmi un acide aminé courant ou un peptide dans lequel sont liés les plusieurs acides aminés courants, et la substance est enregistrée dans la PDB ou dans une base de données en modifiant la PDB.

12. Procédé selon l'une quelconque des revendications 1 à 6, comprenant les étapes consistant à:

rechercher la protéine de référence qui est appropriée pour la séquence cible à partir de la base de données de structures tertiaires et réaliser l'alignement de séquences avec les séquences d'acides aminés des plusieurs protéines de référence recherchées;
sélectionner la séquence d'acides aminés de la protéine de référence présentant une faible valeur E pour la séquence cible; et
ajouter le délimiteur à l'extrémité terminale de chaque séquence d'acides aminés de la chaîne incluse dans la protéine de référence et également ajouter le délimiteur à la position correspondante par rapport à la séquence cible (correction d'un alignement de séquences).

13. Procédé selon la revendication 12, comprenant en outre les étapes consistant à:

obtenir les coordonnées, à partir d'une structure de référence déterminée au cours de l'étape de sélection de la séquence d'acides aminés de la protéine de référence, d'un atome C$\alpha$ qui est l'un des atomes constitutifs dans un acide aminé de la séquence cible en se basant sur les informations de l'alignement, et optimiser les coordonnées atomiques de C$\alpha$;
ajouter les coordonnées atomiques d'une chaîne principale à partir de la base de données de structures tertiaires aux coordonnées atomiques de C$\alpha$ obtenues et optimiser les coordonnées atomiques de la chaîne principale; et
ajouter les coordonnées atomiques d'une chaîne latérale à partir de la base de données de structures tertiaires aux coordonnées atomiques de la chaîne principale obtenues et optimiser les coordonnées atomiques de la chaîne latérale.

14. Procédé selon la revendication 1, dans lequel ladite formule potentielle comprend les éléments suivants:

dans la formule potentielle, lorsque le nombre de chaînes total = M, où N représente le nombre de chaînes protéiques, kN représente le numéro de série du résidu C-terminal dans la chaîne protéique N-th, et i=1, ..., M-1 est décrit de manière simpliste comme i=1, M-1,

(A) dans le calcul pour les étapes de construction et d'optimisation des coordonnées atomiques de C$\alpha$, un cas où i=kN(N=1, M-1) d'un potentiel de liaison chimique temporaire n'est pas inclus et les cas où i=kN (N=1, M-1), i=kN(N=1 M-1)+1 d'un potentiel d'angle de liaison chimique temporaire ne sont pas inclus, et, dans le cas d'un potentiel d'interaction interatomique, j>i+1 est ajouté si i=kN-1, et j>i est ajouté si i=kN respectivement,
(B) dans le calcul pour les étapes de construction et d'optimisation des coordonnées atomiques de la chaîne principale, une liaison entre Ci et Ni+1 n'est pas incluse dans un potentiel de liaison chimique lorsque i=kN (N=1, M-1), les angles C$\alpha$i-Ci-Ni+1, Oi-Ci-Ni+1 et Ci-Ni+1-C$\alpha$i+1 lorsque i=kN(N=1,M-1), où C et O représentent un atome de carbone et un atome d'oxygène dans un carbonyle respectivement, C$\alpha$ représente un atome de carbone $\alpha$ et N représente un atome d'azote, ne sont pas inclus dans un potentiel d'angle de liaison chimique et, en outre, les angles Ni-C$\alpha$i-Ci-Ni+1, C$\alpha$i-Ci-Ni+1-C$\alpha$i+1, et Ci-Ni+1-C$\alpha$i+1-C$\alpha$+1 lorsque i=kN(N=1, M-1) ne sont pas inclus dans un potentiel d'angle de torsion, et de plus, pour un potentiel d'interaction interatomique, où la longueur entre les atomes est représentée par r, un cas de rij $\leq$ à une

valeur spécifiée pour rij ε {rNi, NI+1; rCαi, Ni+1; rCαi, Cαi+1; rCi, Ni+1; rCi, Cαi+1; rCi, Cβi+1; rCi, Ci+1; rOi, Ni+1; rOi, Cαi+1} lorsque i=kN(N=1, M-1) est ajouté.

15. Procédé selon la revendication 8, dans lequel ledit dérivé d'acide aminé est un acide aminé non naturel comme βAsp ou γGlu ou un dérivé de ceux-ci.

16. Programme informatique qui, lorsqu'il est lancé sur un ordinateur, exécute les étapes du procédé selon l'une quelconque des revendications 1 à 14.

17. Procédé selon la revendication 1, dans lequel ladite protéine cible composée de plusieurs chaînes comprend une ou plusieurs chaînes polypeptidiques.

18. Procédé selon la revendication 17, dans lequel au moins une chaîne parmi les plusieurs chaînes est sélectionnée dans le groupe consistant en des acides aminés et des dérivés d'acides aminés naturels ou non naturels comme leurs dérivés, des dérivés peptidiques, des substances pharmaceutiques, des acides nucléiques, des saccharides, des composés à base de métaux organiques, des oxydes métalliques et leurs ions, des métaux et leurs ions.

19. Procédé selon la revendication 1, comprenant les étapes consistant à, pour la protéine cible et la protéine de référence sélectionnée, supposer que chaque séquence d'acides aminés des plusieurs chaînes incluses dans la protéine cible et la protéine de référence sélectionnée, respectivement, sont une seule chaîne dans un état où les extrémités N-terminales et les extrémités C-terminales sont liées de manière séquentielle, réaliser un alignement de séquences entre la séquence de référence de la chaîne unique temporaire ainsi obtenue et la séquence cible de la chaîne unique temporaire ainsi obtenue afin de confirmer leur relation de correspondance, localiser un atome Cα, qui est l'un des atomes constitutifs dans le résidu d'acide aminé dans la séquence cible, lier les atomes Cα par une liaison amide, en outre ajouter une chaîne latérale afin de construire des coordonnées pour d'autres atomes constitutifs, réaliser une optimisation, et construire la structure tertiaire de la protéine cible par le procédé de modélisation.

20. Procédé selon la revendication 1 ou 19, dans lequel la construction de la structure tertiaire de la protéine cible est réalisée en obtenant les coordonnées à partir de la structure tertiaire de la protéine de référence sélectionnée pour l'atome Cα dans un acide aminé de chaîne principale dans la protéine cible en se basant sur les informations de l'alignement obtenues, en optimisant les coordonnées atomiques de Cα de sorte à minimiser une fonction objective, en ajoutant les coordonnées d'autres atomes (comprenant des coordonnées atomiques de Cβ) de la chaîne principale aux coordonnées atomiques de Cα optimisées, en optimisant les coordonnées atomiques de la chaîne principale de sorte à minimiser la fonction objective, en ajoutant les coordonnées des autres atomes de la chaîne latérale aux coordonnées atomiques de la chaîne principale optimisées, et en optimisant les coordonnées atomiques de la chaîne latérale de sorte à minimiser la fonction objective.

## FIG. 1

| First Chain | Second Chain | N-th Chain | (M-1)th Chain | M-th Chain |
|---|---|---|---|---|
| N----C | N----C | N----C | N----C | N----C |
| $1$----$k_1$ | $k_1+1$---$k_2$ | $k_{N-1}+1$---$k_N$ | $k_{M-2}+1$---$k_{M-1}$ | $k_{M-1}+1$---$k_M$ |

Relationship between Whole Amino Acid Sequences and
Serial Numbers $k_N$ of C terminal residue of Each Chain

## FIG. 2

In the Case of $E_{len}$

Occupied Region of
Protein N+1 Chain

N terminal residue

$Ca$  i+1

3.8Å

Occupied Region
of Protein N Chain

$Di, i+1$

$Ca$

$Ca$

3.8Å

$Ca$  i= $k_N$

C terminal residue

Deleted from Consideration
of Potential

## FIG. 3

In the Case of $E_{ang}$

Occupied Region of Protein N+1 Chain

Occupied Region of Protein N Chain

N terminal residue

$\theta_{k_N}+2$

$\theta_{k_N}+1$

$C\alpha$ i+1

$C\alpha$ i+2

$C\alpha$ i-1

$\theta_{k_N}$

$\theta_{k_N}-1$

$C\alpha$ i= $k_N$

C terminal residue

Deleted from Consideration of Potential

## FIG. 4

In the Case of $E_{vdw}$

Occupied Region of Protein N+1 Chain

N terminal residue

Occupied Region of Protein N Chain

$C\alpha$ j= $k_N$+1

$C\alpha$

$C\alpha$

$C\alpha$ i= $k_N$-1

$C\alpha$

$C\alpha$ i= $k_N$

C terminal residue

Added to Consideration of Potential

## FIG. 5

In the Case of E_bond

Occupied Region of Protein N+1 Chain

Amide Nitrogen Atom of N terminal residue : Ni+1

Occupied Region of Protein N Chain

1.45 Å

Dci, Ni+1

1.54 Å

j= kN

Carbonyl Carbon Atom of C terminal residue : Ci

Deleted from Consideration of Potential

## FIG. 6

In the Case of E_anp

Amide Nitrogen Atom of N terminal residue : Ni+1

Occupied Region of Protein N+1 Chain

θ Ni+1,Cai+1,Ci+1

θ Ci,Ni+1,Cai+1

Occupied Region of Protein N Chain

θ Cai,Ci,Ni+1

θ Ni,Cai,Ci       θ Oi,Ci,Ni+1

θ Cai,Ci,Oi

Carbonyl Carbon group of C terminal residue : Ci

Deleted from Consideration of Potential

73

## FIG. 7

In the Case of $E_{tor}$

Occupied Region of Protein N+1 Chain

Amide Nitrogen atom of N terminal residue : Ni+1

Occupied Region of Protein N Chain

Deleted from Consideration of Potential

Newman Projected Drawing

Carbonyl Carbon atom of C terminal residue : Ci

## FIG. 8

In the Case of $E_{non\text{-}bond}$

Occupied Region of Protein N+1 Chain

Amide Nitrogen atom of N terminal residue : Ni+1

Occupied Region of Protein N Chain

Carbonyl group of C terminal residue : Ci

Added to Consideration of Potential

# FIG. 9

TARGET AMINO ACID SEQUENCE OF PROTEIN WITH UNKNOWN
STRUCTURE : ARBITRARY NUMBER OF PLURAL CHAINS

↓ STEP 10

HOMOLOGY SEARCH AND SEQUENCE ALIGNMENT BY ALIGNMENT SOFTWARE

↓ STEP 20

SELECTION OF AMINO ACID SEQUENCES OF ONE OR MORE
REFERENCE PROTEINS FROM SEARCH RESULTS

↓ STEP 30

CORRECTION OF SEQUENCE ALIGNMENT : ADDITION OF STOP SIGNAL
U TO END OF EACH AMINO ACID SEQUENCE OF PLURAL CHAINS

↓ STEP 40

CONSTRUCTION AND OPTIMIZATION OF Cα ATOMIC COORDINATES

↓ STEP 50

CONSTRUCTION AND OPTIMIZATION OF MAIN-CHAIN ATOMIC COORDINATES

↓ STEP 60

CONSTRUCTION AND OPTIMIZATION OF SIDE-CHAIN ATOMIC COORDINATES

↓ STEP 70

VALIDATION OF MODEL STRUCTURE

↓ STEP 80

CONSTRUCTION OF FINAL STRUCTURE : PREDICTION OF 3-D STRUCTURE

STEP 90

FLOW CHART SHOWING PREDICTION OF 3-D STRUCTURE OF
PROTEIN ACCORDING TO THE INVENTION (EXAMPLE)

## FIG. 10

Construction Method of C$\alpha$ Atomic Cordinate of Target Protein

# FIG. 11

AKG |ERQ - S| PV |D I DTHTAKYD| P S L |K| P L
AKG |NRE  AS| SP |NVDTHSARYD| - - L |K| P L
*    *     *       *         * * *   *   * *       *   *  * *

Conceptional Drawing of Local Space Homology (LSH)

# FIG. 12

Relationship between Local Space Homology (LSH) and
Ratio Contained in Structurally Conserved Regions(SCRS)

## FIG. 13

```
MVRLLLIFFP MIFLEMSILP RMPDRKVLLA GASSQRSVAR MDGDVIIGAL FSVHHQPPAE KVPERKCGEI REQYGIQRVE
AMFHTLDKIN ADPVLLPNIT LGSEIRDSCW HSSVALEQSI EFIRDSLISI RDEKDGLNRC LPDGQTLPPG RTKKPIAGVI
GPGSSSVAIQ VQNLLQLFDI PQIAYSATSI DLSDKTLYKY FLRVVPSDTL QARAMLDIVK RYNWTYVSAV HTEGNYGESG
MDAFKELAAQ EGLCIAHSDK IYSNAGEKSF DRLLRKLRER LPKARVVVCF CEGMTVRGLL SAMRRLGVVG EFSLIGSDGW
ADRDEVIEGY EVEANGGITI KLQSPEVRSF DDYFLKLRLD TNTRNPWFPE FWQHRFQCRL PGHLLENPNF KKVCTGNESL
EENYVQDSKM GFVINAIYAM AHGLQNMHHA LCPGHVGLCD AMKPIDGRKL LDFLIKSSFV GVSGEEVWFD EKGDAPGRYD
IMNLQYTEAN RYDYVHVGTW HEGVLNIDDY KIQMNKSGMV RSVCSEPCLK GQIKVIRKGE VSCCWICTAC KENEFVQDEF
TCRACDLGWW PNAELTGCEP IPVRYLEWSD IESIIAIAFS CLGILVTLFV TLIFVLYRDT PVVKSSSREL CYIILAGIFL
GYVCPFTLIA KPTTTSCYLQ RLLVGLSSAM CYSALVTKTN RIARILAGSK KKICTRKPRF MSAWAQVIIA SILISVQLTL
VVTLIIMEPP MPILSYPSIK EVYLICNTSN LGVVAPVGYN GLLIMSCTYY AFKTRNVPAN FNEAKYIAFT MYTTCIIWLA
FVPIYFGSNY KIITTCFAVS LSVTVALGCM FTPKMYIIIA KPERNVRSAF TTSDVVRMHV GDGKLPCRSN TFLNIFRRKK
PGAGNANSNG KSVSWSEPGG RQAPKGQHVW QRLSVHVKTN ETACNQTAVI KPLTKSYQGS GKSLTFSDAS TKTLYNVEEE
DNTPSAHFSP PSSPSMVVHR RGPPVATTPP LPPHLTAEET PLFLADSVIP KGLPPPLPQQ QPQQPPPQQP PQQPKSLMDQ
LQGVVTNFGS GIPDFHAVLA GPGTPGNSLR SLYPPPPPPQ HLQMLPLHLS TFQEESISPP GEDIDDDSER FKLLQEFVYE
REGNTEEDEL EEEEDLPTAS KLTPEDSPAL TPPSPFRDSV ASGSSVPSSP VSESVLCTPP NVTYASVILR DYKQSSSTL
```

## FIG. 14-1

```
>MGR5_RAT        1203     homology     match     mismatch     insertion     deletion
>MGR1_RAT        1189     62.2%        740       396           53            67


MVLLLILSVLLLKEDVRGSAQSSERRVVAHMPGDIIIGALFSVHHQPTVDKVHERKCGAVREQYGIQRVEAMLHTLERIN
MIFLEMSILPRMPDRKVLLAGASSQRSVARMDGDVIIGALFSVHHQPPAEKVPERKCGEIREQYGIQRVEAMFHTLDKIN
++++++++++++++++++++++++++++++++++++++++++++++++++++++++++++++++++++++++++++++++++++


SDPTLLPNITLGCEIRDSCWHSAVALEQSIEFIRDSLISS-EEEEGLVRCVDGSSSF---RSKKPIVGVIGPGSSSVAIQ
ADPVLLPNITLGSEIRDSCWHSSVALEQSIEFIRDSLISIRDEKDGLNRCLPDGQTLPPGRTKKPIAGVIGPGSSSVAIQ
+++++++++++++++++++++++++++++++++++++++++++++++++++++++++++++++++++++++++++++++++++


VQNLLQLFNIPQIAYSATSMDLSDKTLFKYFMRVVPSDAQQARAMVDIVKRYNWTYVSAVHTEGNYGESGMEAFKDMSAK
VQNLLQLFDIPQIAYSATSIDLSDKTLYKYFLRVVPSDTLQARAMLDIVKRYNWTYVSAVHTEGNYGESGMDAFKELAAQ
++++++++++++++++++++++++++++++++++++++++++++++++++++++++++++++++++++++++++++++++++++


EGICIAHSYKIYSNAGEQSFDKLLKKLRSHLPKARVVACFCEGMTVRGLLMAMRRLGLAGEFLLLGSDGWADRYDVTDGY
EGLCIAHSDKIYSNAGEKSFDRLLRKLRERLPKARVVVCFCEGMTVRGLLSAMRRLGVVGEFSLIGSDGWADRDEVIEGY
++++++++++++++++++++++++++++++++++++++++++++++++++++++++++++++++++++++++++++++++++++


QREAVGGITIKLQSPDVKWFDDYYLKLRPETNLRNPWFQEFWQHRFQCRLEGFAQENSKYNKTCNSSLTLRTHHVQDSKM
EVEANGGITIKLQSPEVRSFDDYFLKLRLDTNTRNPWFPEFWQHRFQCRLPGHLLENPNFKKVCTGNESLEENYVQDSKM
++++++++++++++++++++++++++++++++++++++++++++++++++++++++++++++++++++++++++++++++++++


GFVINAIYSMAYGLHNMQMSLCPGYAGLCDAMKPIDGRKLLDSLMKTNFTGVSGDMILFDENGDSPGRYEIMNFKEMGKD
GFVINAIYAMAHGLQNMHHALCPGHVGLCDAMKPIDGRKLLDFLIKSSFVGVSGEEVWFDEKGDAPGRYDIMNLQYTEAN
+++++++++++++++++++++++++++++++++++++++++++++++++++++++++++++++++++++++++++++++++++++


YFDYINVGSWDNGELKMDDDEVWSKKNNIIRSVCSEPCEKGQIKVIRKGEVSCCWTCTPCKENEYVFDEYTCKACQLGSW
RYDYVHVGTWHEGVLNIDDYKIQMNKSGMVRSVCSEPCLKGQIKVIRKGEVSCCWICTACKENEFVQDEFTCRACDLGWW
++++++++++++++++++++++++++++++++++++++++++++++++++++++++++++++++++++++++++++++++++++
```

# FIG. 14-2

```
PTDDLTGCDLIPVQYLRWGDPEPIAAVVFACLGLLATLFVTVIFIIYRDTPVVKSSSRELCYIILAGICLGYLCTFCLIA
PNAELTGCEPIPVRYLEWSDIESIIAIAFSCLGILVTLFVTLIFVLYRDTPVVKSSSRELCYIILAGIFLGYVCPFTLIA
+++++++++++++++++++++++++++++++++++++++++++++++++++++++++++++++++++++++++++++++++


KPKQIYCYLQRIGIGLSPAMSYSALVTKTNRIARILAGSKKKICTKKPRFMSACAQLVIAFILICIQLGIIVALFIMEPP
KPTTTSCYLQRLLVGLSSAMCYSALVTKTNRIARILAGSKKKICTRKPRFMSAWAQVIIASILISVQLTLVVTLIIMEPP
+++++++++++++++++++++++++++++++++++++++++++++++++++++++++++++++++++++++++++++++++


DIMHDYPSIREVYLICNTTNLGVVTPLGYNGLLILSCTFYAFKTRNVPANFNEAKYIAFTMYTTCIIWLAFVPIYFGSNY
MPILSYPSIKEVYLICNTSNLGVVAPVGYNGLLIMSCTYYAFKTRNVPANFNEAKYIAFTMYTTCIIWLAFVPIYFGSNY
+++++++++++++++++++++++++++++++++++++++++++++++++++++++++++++++++++++++++++++++++


KIITMCFSVSLSATVALGCMFVPKVYIILAKPERNVRSAFTTSTVVRMHVGDGKSSSAASRSSSLVNLWKRRGSSGETLR
KIITTCFAVSLSVTVALGCMFTPKMYIIIAKPERNVRSAFTTSDVVRMHVGDGK---LPCRSNTFLNIFRRKKPCAGN--
+++++++++++++++++++++++++++++++++++++++++++++++++++++++++++++++++++++++++++++++++


YKDRRLAQHKSEIECFTPKGSMGNGGRATMSSSNGKSVTWAQ--NEKSTRGQHLWQRLSVHINKKEN-PNQTAVIKPFPK
---------------------------------ANSNGKSVSWSEPGGRQAPKGQHVWQRLSVHVKTNETACNQTAVIKPLTK
+++++++++++++++++++++++++++++++++++++++++++++++++++++++++++++++++++++++++++++++++


STENRGPGAAAGGGSGPGVAGAGNAGCTATGGPEPPDAGPKALYDVAEAEESFPAA--ARPRSPS--------PISTLSH
SYQ---------GSGKSLTF-------------SDASTKTLYNVEE-EDNTPSAHFSPPSSPSMVVHRRGPPVATTPP
+++++++++++++++++++++++++++++++++++++++++++++++++++++++++++++++++++++++++++++++++


LAGSAGRTDDDAPSLHSETAA------------------RSSSSQGSLMEQISSVVTRFTANISELNSMMLSTAATPGP
LPPH--LTAEETPLFLADSVIPKGLPPPLPQQQPQQPPPQQPPQQPKSLMDQLQGVVTNFGSGIPDFHAVL----AGPGT
+++++++++++++++++++++++++++++++++++++++++++++++++++++++++++++++++++++++++++++++++


PGTPICSSYLIPKEIQ----LPTTMTTFAE--IQPLPAIEVTGGAQGATGVSPAQETPTGAESAP-----------GKPD
PGNSLRSLYPPPPPPQHLQMLPLHLSTFQEESISP-PGEDIDDDSERFKLLQEFVYEREGNTEEDELEEEDLPTASKLT
+++++++++++++++++++++++++++++++++++++++++++++++++++++++++++++++++++++++++++++++++


LEELVALTPPSPFRDSVDSGSTTPNSPVSESALCIPSSPKYDTLIIRDYTQSSSSL
PEDSPALTPPSPFRDSVASGSSVPSSPVSESVLCTPPNVTYASVILRDYKQSSSTL
++++++++++++++++++++++++++++++++++++++++++++++++++++++++
```

## FIG. 15

| >MGR5_RAT | 474 | homology | match | mismatch | insertion | deletion |
|---|---|---|---|---|---|---|
| >1EWT_A | 456 | 73.9% | 337 | 118 | 1 | 19 |

```
RRVVAHMPGDI I IGALFSVHHQPTVDKVHERKCGAVREQYGIQRVEAMLHTLERINSDPTLLPNITLGCEIRDSCWHSAV
QRSVARMDGDVI IGALFSVHHQPPAEKVPERKCGEIREQYGIQRVEAMFHTLDKINADPVLLPNITLGSEIRDSCWHSSV
+#+##+#+##+###############+++##+######++######################+###+++##+##+#######+####+######++#+######+####+


ALEQSIEFIRDSLISS-EEEEGLVRCVDGSSSFRSKKPIVGVIGPGSSSVAIQVQNLLQLFNIPQIAYSATSMDLSDKTL
ALEQSIEFIRDSLISIR------------------KPIAGVIGPGSSSVAIQVQNLLQLFDIPQIAYSATSIDLSDKTL
#############+++++++++++++++++#+#######################+#############+#######


FKYFMRVVPSDAQQARAMVDIVKRYNWTYVSAVHTEGNYGESGMEAFKDMSAKEGICIAHSYKIYSNAGEQSFDKLLKKL
YKYFLRVVPSDTLQARAMLDIVKRYNWTYVSAVHTEGNYGESGMDAFKELAAQEGLCIAHSDKIYSNAGEKSFDRLLRKL
+####+######++######+###################################+###+++#+#+######+########+###+##+##


RSHLPKARVVACFCEGMTVRGLLMAMRRLGLAGEFLLLGSDGWADRYDVTDGYQREAVGGITIKLQSPDVKWFDDYYLKL
RERLPKARVVVCFCEGMTVRGLLSAMRRLGVVGEFSLIGSDGWADRDEVIEGYEVEANGGITIKLQSPEVRSFDDYFLKL
#++########+################+######++###+#+#########+#+##+###+#+######################+#++#####+###


RPETNLRNPWFQEFWQHRFQCRLEGFAQENSKYNKTCNSSLTLRTHHVQDSKMGFVINAIYSMAYGLHNMQMSLCPGYAG
RLDTNTRNPWFPEFWQHRFQCRLPGHLLENPNFKKVCTGNESLEENYVQDSKMGFVINAIYAMAHGLQNMHHALCPGHVG
#++##+#####+#################+#+++##+#++###+##+##+++#+############################+##+#++#+###+#++###+#+


LCDAMKPIDGRKLLDSLMKTNFTGVSGDMILFDENGDSPGRYEIMNFKEMGKDYFDYINVGSWDNGELKMDDDEV
LCDAMKPIDGRKLLDFLIKSSFVGVSGEEVWFDEKGDAPGRYDIMNLQYTEANRYDYVHVGTWHEGVLNIDDYKI
#################+#+#+#+#+#####+++#####+##+#+#+####+######+++++++++##+##+##+#+#+##+##+++
```

## FIG. 16

```
>MGR5_RAT
RRVVAHMPGDI I IGALFSVHHQPTVDKVHERKCGAVREQYGIQRVEAMLHTLERINSDPTLLPNITLGCEIRDSCWHSAVALEQS
IEFIRDSLISS-EEEEGLVRCVDGSSSFRSKKPIVGVIGPGSSSVAIQVQNLLQLFNIPQIAYSATSMDLSDKTLFKYFMRVVPS
DAQQARAMVDIVKRYNWTYVSAVHTEGNYGESGMEAFKDMSAKEGICIAHSYKIYSNAGEQSFDKLLKKLRSHLPKARVVACFCE
GMTVRGLLMAMRRLGLAGEFLLLGSDGWADRYDVTDGYQREAVGGITIKLQSPDVKWFDDYYLKLRPETNLRNPWFQEFWQHRFQ
CRLEGFAQENSKYNKTCNSSLTLRTHHVQDSKMGFVINAIYSMAYGLHNMQMSLCPGYAGLCDAMKPIDGRKLLDSLMKTNFTGV
SGDMILFDENGDSPGRYEIMNFKEMGKDYFDYINVGSWDNGELKMDDDEV
>1EWT_A
QRSVARMDGDVI IGALFSVHHQPPAEKVPERKCGEIREQYGIQRVEAMFHTLDKINADPVLLPNITLGSEIRDSCWHSSVALEQS
IEFIRDSLISIR------------------KPIAGVIGPGSSSVAIQVQNLLQLFDIPQIAYSATSIDLSDKTLYKYFLRVVPS
DTLQARAMLDIVKRYNWTYVSAVHTEGNYGESGMDAFKELAAQEGLCIAHSDKIYSNAGEKSFDRLLRKLRERLPKARVVVCFCE
GMTVRGLLSAMRRLGVVGEFSLIGSDGWADRDEVIEGYEVEANGGITIKLQSPEVRSFDDYFLKLRLDTNTRNPWFPEFWQHRFQ
CRLPGHLLENPNFKKVCTGNESLEENYVQDSKMGFVINAIYAMAHGLQNMHHALCPGHVGLCDAMKPIDGRKLLDFLIKSSFVGV
SGEEVWFDEKGDAPGRYDIMNLQYTEANRYDYVHVGTWHEGVLNIDDYKI
```

## FIG. 17

## FIG. 18

```
>MGR5_RAT
RRVVAHMPGDI I IGALFSVHHQPTVDKVHERKCGAVREQYGIQRVEAMLHTLERINSDPTLLPNITLGCEIRDSCWHSAVALEQS
IEFIRDSLISS-EEEEGLVRCVDGSSSFRSKKPIVGVIGPGSSSVAIQVQNLLQLFNIPQIAYSATSMDLSDKTLFKYFMRVVPS
DAQQARAMVDIVKRYNWTYVSAVHTEGNYGESGMEAFKDMSAKEGICIAHSYKIYSNAGEQSFDKLLKKLRSHLPKARVVACFCE
GMTVRGLLMAMRRLGLAGEFLLLGSDGWADRYDVTDGYQREAVGGITIKLQSPDVKWFDDYYLKLRPETNLRNPWFQEFWQHRFQ
CRLEGFAQENSKYNKTCNSSLTLRTHHVQDSKMGFVINAIYSMAYGLHNMQMSLCPGYAGLCDAMKPIDGRKLLDSLMKTNFTGV
SGDMILFDENGDSPGRYEIMNFKEMGKDYFDYINVGSWDNGELKMDDDEVURRVVAHMPGDI I IGALFSVHHQPTVDKVHERKCG
AVREQYGIQRVEAMLHTLERINSDPTLLPNITLGCEIRDSCWHSAVALEQSIEFIRDSLISS-EEEEGLVRCVDGSSSFRSKKPI
VGVIGPGSSSVAIQVQNLLQLFNIPQIAYSATSMDLSDKTLFKYFMRVVPSDAQQARAMVDIVKRYNWTYVSAVHTEGNYGESGM
EAFKDMSAKEGICIAHSYKIYSNAGEQSFDKLLKKLRSHLPKARVVACFCEGMTVRGLLMAMRRLGLAGEFLLLGSDGWADRYDV
TDGYQREAVGGITIKLQSPDVKWFDDYYLKLRPETNLRNPWFQEFWQHRFQCRLEGFAQENSKYNKTCNSSLTLRTHHVQDSKMG
FVINAIYSMAYGLHNMQMSLCPGYAGLCDAMKPIDGRKLLDSLMKTNFTGVSGDMILFDENGDSPGRYEIMNFKEMGKDYFDYIN
VGSWDNGELKMDDDEV
>1EWT_A
QRSVARMDGDVI IGALFSVHHQPPAEKVPERKCGEIREQYGIQRVEAMFHTLDKINADPVLLPNITLGSEIRDSCWHSSVALEQS
IEFIRDSLISIR------------------KPIACVIGPGSSSVAIQVQNLLQLFDIPQIAYSATSIDLSDKTLYKYFLRVVPS
DTLQARAMLDIVKRYNWTYVSAVHTEGNYGESGMDAFKELAAQEGLCIAHSDKIYSNAGEKSFDRLLRKLRERLPKARVVVCFCE
GMTVRGLLSAMRRLGVVGEFSLIGSDGWADRDEVIEGYEVEANGGITIKLQSPEVRSFDDYFLKLRLDTNTRNPWFPEFWQHRFQ
CRLPGHLLENPNFKKVCTGNESLEENYVQDSKMGFVINAIYAMAHGLQNMHHALCPGHVGLCDAMKPIDGRKLLDFLIKSSFVGV
SGEEVWFDEKGDAPGRYDIMNLQYTEANRYDYVHVGTWHEGVLNIDDYKIUQRSVARMDGDVI IGALFSVHHQPPAEKVPERKCG
EIREQYGIQRVEAMFHTLDKINADPVLLPNITLGSEIRDSCWHSSVALEQSIEFIRDSLISIR------------------KPI
ACVIGPGSSSVAIQVQNLLQLFDIPQIAYSATSIDLSDKTLYKYFLRVVPSDTLQARAMLDIVKRYNWTYVSAVHTEGNYGESGM
DAFKELAAQEGLCIAHSDKIYSNAGEKSFDRLLRKLRERLPKARVVVCFCEGMTVRGLLSAMRRLGVVGEFSLIGSDGWADRDEV
IEGYEVEANGGITIKLQSPEVRSFDDYFLKLRLDTNTRNPWFPEFWQHRFQCRLPGHLLENPNFKKVCTGNESLEENYVQDSKMG
FVINAIYAMAHGLQNMHHALCPGHVGLCDAMKPIDGRKLLDFLIKSSFVGVSGEEVWFDEKGDAPGRYDIMNLQYTEANRYDYVH
VGTWHEGVLNIDDYKI
```

FIG. 19

FIG. 20

FIG. 21

WQTFRRLWPTIAPFKAGLIVAGVALILNAASDTFMLSLLKPLLDDGFGKTDRSVLVWMPLVVIGLMILRGITSYVSSYCI
SWVSGKVVMTMRRRLFGHMMGMPVSFFDKQSTGTLLSRITYDSEQVASSSSGALITVVREGASIIGLFIMMFYYSWQLSI
ILIVLAPIVSIAIRVVSKRFRNISKNMQNTMGQVTTSAEQMLKGHKEVLIFGGQEVETKRFDKVSNRMRLQGMKMVSASS
ISDPIIQLIASLALAFVLYAASFPSVMDSLTAGTITVVFSSMIALMRPLKSLTNVNAQFQRGMAACQTLFTILDSEQEKD
EGKRVIERATGDVEFRNVTFTYPGRDVPALRNINLKIPAGKTVALVGRSGSGKSTIASLITRFYDIDEGEILMDGHDLRE
YTLASLRNQVALVSQNVHLFNDTVANNIAYARTEQYSREQIEEAARMAYAMDFINKMDNGLDTVIGENGVLLSGGQRQRI
AIARALLRDSPILILDEATSALDTESERAIQAALDELQKNRTSLVIAHRLSTIEKADEIVVVEDGVIVERGTHND

# FIG. 22

```
>093437        573    homology      match   mismatch  insertion  deletion
>MSBA          549    32.4%         178     370       1          25


LFRYSSCTDKLLMIFGSLLAIAHGTSLPIAMIIFGDMTDSFVTSGMTNITGNSSGLNSSADVFNKLEEEMTRYAYYYSAI
LWPTIAP-FKAGLIVAGVALILNAASDTFMLSLLKPLLDDGFGKTDR------------------SVLVWMPLVVIGL
+++++++++++*+++*+++++++*+++*+*+++++++++++++++*+++++++++++++++++++++++++++++++++++++


AAAVLVAAYIQTSFWTLAAGRQVKKIREKFFHAIMRQEIGWFDVNDAGELNTRLIDDVSKINEGIGDKIGFLIQSETTFL
MILRGITSYVSSYCISWVSGKVVMTMRRRLFGHMMGMPVSFFDKQSTGTLLSRITYDSEQVASSSSGALITVVREGASII
++++++++++*+++++++++++*+++*+++*+++*+++*+++++++*+++*+*+++*++*+*+++++++++++++++++++++


TGFIVGFIRGWKLTLVILAVSPVLGLSAALWAKILTAFTDKEQAAYAKAGAVAEEVLSAVRTVIAFGGQEKEIKRYHKNL
GLFIMMFYYSWQLSIILIVLAPIVSIAIRVVSKRFRNISKNMQNTMGQVTTSAEQMLKGHKEVLIFGGQEVETKRFDKVS
++*++++*++++*+*+++++++++*+++++++++++*+++++++++++*+++*+*+++++*++*+*+++++*+*++++*++*+


EDAKRIGIRKAITSNISMGAAFLLIYASYALAFWYGTTLILANEYSIGNVLTVFFSVLIGAFSIGQTAPSIEAFANARGA
NRMRLQGMKMVSASSISDPIIQLIASLALAFVLYAASFPSVMDSLTAGTITVVFSSMIALMRPLKSLTNVNAQFQRGMAA
++++++*++++++*++*+++++++++++*+++++++++++++++++*+++*+++++*+*+++++*++++++++++++*++++*


AYAIFNIIDNEPEIDSYSDAGHKPDHIKGNLEFQNVFFNYPSRPDVEILKGLNLKVNCGQTVALVGGSGCGKSTTVQLIQ
CQTLFTILDSEQEKD---EGKRVIERATGDVEFRNVTFTYPGR-DVPALRNINLKIPAGKTVALVGRSGSGKSTIASLIT
++++++*+*+*+*+++++++++++++f+++*++*++*+*+*++*++*++*+++*+*++++++*++*+*++++*++*+++*++*+


RFYDPKEGTITIDGQDLKSLNVRYLREIIGVVNQEPVLFATTIAENIRYG-REDVTMEEIERATKEANAYDFIMKLPKKF
RFYDIDEGEILMDGHDLREYTLASLRNQVALVSQNVHLFNDTVANNIAYARTEQYSREQIEEAARMAYAMDFINKMDNGL
+*++++++*+*+++*++*+++++++*++*+++*+*++*+++*+++*+*+++++*+++++*+*++*+++++++*+*+++++++


ETVVGERGAQMSGGQKQRIAIARALVHNPKILLLDEATSALDTESESVVQAALDKAREGRTTVVVAHRLSTVRNADLIAV
DTVIGENGVLLSGGQRQRIAIARALLRDSPILILDEATSALDTESERAIQAALDELQKNRTSLVIAHRLSTIEKADEIVV
+*++*++*++++++*+*+++++++++++*+++++*+++++++++++++++++++++++++++++++*++++*+*+++++*+*+*


FESGVITEQGNHSQ
VEDGVIVERGTHND
+*+**+*+*+*+++
```

## FIG. 23

```
>093437            467   homology   match   mismatch  insertion  deletion
>msbA              444   28.2%      125     318       1          24


LFRYSSCTDKLLMIFGSLLAIAHGTSLPIAMIIFGDMTDSFVTSGMTNITGNSSGLNSSADVFNKLEEEMTRYAYYYSAI
LWPTIAP-FKAGLIVAGVALILNAASDTFMLSLLKPLLDDGFGKTDR------------------SVLVWMPLVVIGL
*+++++++++*+++*++++++++*++++*++++++++++++++*+++++++++++++++++++++++++++++++++++++


AAAVLVAAYIQTSFWTLAAGRQVKKIREKFFHAIMRQEIGWFDVNDAGELNTRLIDDVSKINEGIGDKIGFLIQSETTFL
MILRGITSYVSSYCISWVSGKVVMTMRRRLFGHMMGMPVSFFDKQSTGTLLSRITYDSEQVASSSSGALITVVREGASII
++++++++*+++++++++++*++*+++*+++*++++++*++++++**++++++*+*++*+++*++++++++++++++++++++


TGFIVGFIRGWKLTLVILAVSPVLGLSAALWAKILTAFTDKEQAAYAKAGAVUKRIGIRKAITSNISMGAAFLLIYASYA
GLFIMMFYYSWQLSIILIVLAPIVSIAIRVVSKRFRNISKNMQNTMGQVTTSURLQGMKMVSASSISDPIIQLIASLALA
++*++*++*+*++++*+++++++*+++++++++++++*+++++++++*++++++++++*+++++*+++++*+*+*+++++++*


LAFWYGTTLILANEYSIGNVLTVFFSVLIGAFSIGQTAPSIEAFANARGAAYAIFNIIDNEPEIDSYSDAGHKPDHIKGN
FVLYAASFPSVMDSLTAGTITVVFSSMIALMRPLKSLTNVNAQFQRGMAACQTLFTILDSEQEKD---EGKRVIERATGD
+++++++++++++*+++++*+*++++++++++++++++++*+++++*++++++*+*+*+*+*+++++++++++++++++++


LUIGVVNQEPVLFATTIAENIRYG-REDVTMEEIERATKEANAYDFIMKLPKKFETVVGERGAQMSGGQKQRIAIARALV
VUVALVSQNVHLFNDTVANNIAYARTEQYSREQIEEAARMAYAMDFINKMDNGLDTVIGENGVLLSGGQRQRIAIARALL
+*++++*+++++*+*++*++++++++++++++*++*+*++++*++++++++++++*+++*++*+*++++++++**+*******+


HNPKILLLDEATSALDTESESVVQAALDKAREGRTTVVVAHRLSTVRNADLIAVFESGVITEQGNHSQ
RDSPILILDEATSALDTESERAIQAALDELQKNRTSLVIAHRLSTIEKADEIVVVEDGVIVERGTHND
++++++*+++++++++++++*++++++++++*+++*++*+*++++++*+++*+*+*+*+++*+*+*+++
```

## FIG. 24

```
>093437
LFRYSSCTDKLLMIFGSLLAIAHGTSLPIAMIIFGDMTDSFVTSGMTNITGNSSGLNSSADVFNKLEEEMTRYAYYYSAI
AAAVLVAAYIQTSFWTLAAGRQVKKIREKFFHAIMRQEIGWFDVNDAGELNTRLIDDVSKINEGIGDKIGFLIQSETTFL
TGFIVGFIRGWKLTLVILAVSPVLGLSAALWAKILTAFTDKEQAAYAKAGAVUKRIGIRKAITSNISMGAAFLLIYASYA
LAFWYGTTLILANEYSIGNVLTVFFSVLIGAFSIGQTAPSIEAFANARGAAYAIFNIIDNEPEIDSYSDAGHKPDHIKGN
LUIGVVNQEPVLFATTIAENIRYG-REDVTMEEIERATKEANAYDFIMKLPKKFETVVGERGAQMSGGQKQRIAIARALV
HNPKILLLDEATSALDTESESVVQAALDKAREGRTTVVVAHRLSTVRNADLIAVFESGVITEQGNHSQ
>msbA
LWPTIAP-FKAGLIVAGVALILNAASDTFMLSLLKPLLDDGFGKTDR------------------SVLVWMPLVVIGL
MILRGITSYVSSYCISWVSGKVVMTMRRRLFGHMMGMPVSFFDKQSTGTLLSRITYDSEQVASSSSGALITVVREGASII
GLFIMMFYYSWQLSIILIVLAPIVSIAIRVVSKRFRNISKNMQNTMGQVTTSURLQGMKMVSASSISDPIIQLIASLALA
FVLYAASFPSVMDSLTAGTITVVFSSMIALMRPLKSLTNVNAQFQRGMAACQTLFTILDSEQEKD---EGKRVIERATGD
VUVALVSQNVHLFNDTVANNIAYARTEQYSREQIEEAARMAYAMDFINKMDNGLDTVIGENGVLLSGGQRQRIAIARALL
RDSPILILDEATSALDTESERAIQAALDELQKNRTSLVIAHRLSTIEKADEIVVVEDGVIVERGTHND
```

## FIG. 25

## FIG. 26

```
>093437
LFRYSSCTDKLLMIFGSLLAIAHGTSLPIAMIIFGDMTDSFVTSGMTNITGNSSGLNSSADVFNKLEEEMTRYAYYYSAI
AAAVLVAAYIQTSFWTLAAGRQVKKIREKFFHAIMRQEIGWFDVNDAGELNTRLIDDVSKINEGIGDKIGFLIQSETTFL
TGFIVGFIRGWKLTLVILAVSPVLGLSAALWAKILTAFTDKEQAAYAKAGAVUKRIGIRKAITSNISMGAAFLLIYASYA
LAFWYGTTLILANEYSIGNVLTVFFSVLIGAFSIGQTAPSIEAFANARGAAYAIFNIIDNEPEIDSYSDAGHKPDHIKGN
LUIGVVNQEPVLFATTIAENIRYG-REDVTMEEIERATKEANAYDFIMKLPKKFETVVGERGAQMSGGQKQRIAIARALV
HNPKILLLDEATSALDTESESVVQAALDKAREGRTTVVVAHRLSTVRNADLIAVFESGVITEQGNHSQULFRYSSCTDKL
LMIFGSLLAIAHGTSLPIAMIIFGDMTDSFVTSGMTNITGNSSGLNSSADVFNKLEEEMTRYAYYYSAIAAAVLVAAYIQ
TSFWTLAAGRQVKKIREKFFHAIMRQEIGWFDVNDAGELNTRLIDDVSKINEGIGDKIGFLIQSETTFLTGFIVGFIRGW
KLTLVILAVSPVLGLSAALWAKILTAFTDKEQAAYAKAGAVUKRIGIRKAITSNISMGAAFLLIYASYALAFWYGTTLIL
ANEYSIGNVLTVFFSVLIGAFSIGQTAPSIEAFANARGAAYAIFNIIDNEPEIDSYSDAGHKPDHIKGNLUIGVVNQEPV
LFATTIAENIRYG-REDVTMEEIERATKEANAYDFIMKLPKKFETVVGERGAQMSGGQKQRIAIARALVHNPKILLLDEA
TSALDTESESVVQAALDKAREGRTTVVVAHRLSTVRNADLIAVFESGVITEQGNHSQ
>msbA
LWPTIAP-FKAGLIVAGVALILNAASDTFMLSLLKPLLDDGFGKTDR-------------------SVLVWMPLVVIGL
MILRGITSYVSSYCISWVSGKVVMTMRRRLFGHMMGMPVSFFDKQSTGTLLSRITYDSEQVASSSSGALITVVREGASII
GLFIMMFYYSWQLSIILIVLAPIVSIAIRVVSKRFRNISKNMQNTMGQVTTSURLQGMKMVSASSISDPIIQLIASLALA
FVLYAASFPSVMDSLTAGTITVVFSSMIALMRPLKSLTNVNAQFQRGMAACQTLFTILDSEQEKD---EGKRVIERATGD
VUVALVSQNVHLFNDTVANNIAYARTEQYSREQIEEAARMAYAMDFINKMDNGLDTVIGENGVLLSGGQRQRIAIARALL
RDSPILILDEATSALDTESERAIQAALDELQKNRTSLVIAHRLSTIEKADEIVVVEDGVIVERGTHNDULWPTIAP-FKA
GLIVAGVALILNAASDTFMLSLLKPLLDDGFGKTDR-------------------SVLVWMPLVVIGLMILRGITSYVS
SYCISWVSGKVVMTMRRRLFGHMMGMPVSFFDKQSTGTLLSRITYDSEQVASSSSGALITVVREGASIIGLFIMMFYYSW
QLSIILIVLAPIVSIAIRVVSKRFRNISKNMQNTMGQVTTSURLQGMKMVSASSISDPIIQLIASLALAFVLYAASFPSV
MDSLTAGTITVVFSSMIALMRPLKSLTNVNAQFQRGMAACQTLFTILDSEQEKD---EGKRVIERATGDVUVALVSQNVH
LFNDTVANNIAYARTEQYSREQIEEAARMAYAMDFINKMDNGLDTVIGENGVLLSGGQRQRIAIARALLRDSPILILDEA
TSALDTESERAIQAALDELQKNRTSLVIAHRLSTIEKADEIVVVEDGVIVERGTHND
```

FIG. 27

FIG. 28

FIG. 29

FIG. 30

## FIG. 31

```
>1JKY_A          748 AMINO ACIDS
NKTQEEHLKEIMKHIVKIEVKGEEAVKKEAAEKLLEKVPSDVLEMYKAIGGKIYIVDGDITKHISLEALSEDKKKIKDIY
GKDALLHEHYVYAKEGYEPVLVIQSSEDYVENTEKALNVYYEIGKILSRDILSKINQPYQKFLDVLNTIKNASDSDGQDL
LFTNQLKEHPTDFSVEFLEQNSNEVQEVFAKAFAYYIEPQHRDVLQLYAPEAFNYMDKFNEQEINLSLEELKDQRMLSRY
EKWEKIKQHYQHWSDSLSEEGRGLLKKLQIPIEPKKDDIIHSLSQEEKELLKRIQIDSSDFLSTEEKEFLKKLQIDIRDS
LSEEEKELLNRIQVDSSNPLSEKEKEFLKKLKLDIQPYDINQRLQDTGGLIDSPSINLDVRKQYKRDIQNIDALLHQSIG
STLYNKIYLYENMNINNLTATLGADLVDSTDNTKINRGIFNEFKKNFKYSISSNYMIVDINERPALDNERLKWRIQLSPD
TRAGYLENGKLILQRNIGLEIKDVQIIKQSEKEYIRIDAKVVPKSKIDTKIQEAQLNINQEWNKALGLPKYTKLITFNVH
NRYASNIVESAYLILNEWKNNIQSDLIKKVTNYLVDGNGRFVFTDITLPNIAEQYTHQDEIYEQVHSKGLYVPESRSILL
HGPSKGVELRNDSEGFIHEFGIIAVDDYAGYLLDKNQSDLVTNSKKFIDIFKEEGSNLTSYGRTNEAEFFAEAFRLMHSTD
HAERLKVQKNAPKTFQFINDQIKFIINS
>1JKY_B         16 AMINO ACIDS

MLARRKPVLPALTINP
```

## FIG. 32

## FIG. 33

```
>UMFFRR
NKTQEEHLKEIMKHIVKIEVKGEEAVKKEAAEKLLEKVPSDVLEMYKAIGGKIYIVDGDITKHISLEALSEDKKKIKDIY
GKDALLHEHYVYAKEGYEPVLVIQSSEDYVENTEKALNVYYEIGKILSRDILSKINQPYQKFLDVLNTIKNASDSDGQDL
LFTNQLKEHPTDFSVEFLEQNSNEVQEVFAKAFAYYIEPQHRDVLQLYAPEAFNYMDKFNEQEINLSLEELKDQRMLSRY
EKWEKIKQHYQHWSDSLSEEGRGLLKKLQIPIEPKKDDIIHSLSQEEKELLKRIQIDSSDFLSTEEKEFLKKLQIDIRDS
LSEEEKELLNRIQVDSSNPLSEKEKEFLKKLKLDIQPYDINQRLQDTGGLIDSPSINLDVRKQYKRDIQNIDALLHQSIG
STLYNKIYLYENMNINNLTATLGADLVDSTDNTKINRGIFNEFKKNFKYSISSNYMIVDINERPALDNERLKWRIQLSPD
TRAGYLENGKLILQRNIGLEIKDVQIIKQSEKEYIRIDAKVVPKSKIDTKIQEAQLNINQEWNKALGLPKYTKLITFNVH
NRYASNIVESAYLILNEWKNNIQSDLIKKVTNYLVDGNGRFVFTDITLPNIAEQYTHQDEIYEQVHSKGLYVPESRSILL
HGPSKGVELRNDSEGFIHEFGHAVDDYAGYLLDKNQSDLVTNSKKFIDIFKEEGSNLTSYGRTNEAEFFAEAFRLMHSTD
HAERLKVQKNAPKTFQFINDQIKFIINSUMFFRRKPVLPALTINP
>1JKY
NKTQEEHLKEIMKHIVKIEVKGEEAVKKEAAEKLLEKVPSDVLEMYKAIGGKIYIVDGDITKHISLEALSEDKKKIKDIY
GKDALLHEHYVYAKEGYEPVLVIQSSEDYVENTEKALNVYYEIGKILSRDILSKINQPYQKFLDVLNTIKNASDSDGQDL
LFTNQLKEHPTDFSVEFLEQNSNEVQEVFAKAFAYYIEPQHRDVLQLYAPEAFNYMDKFNEQEINLSLEELKDQRMLSRY
EKWEKIKQHYQHWSDSLSEEGRGLLKKLQIPIEPKKDDIIHSLSQEEKELLKRIQIDSSDFLSTEEKEFLKKLQIDIRDS
LSEEEKELLNRIQVDSSNPLSEKEKEFLKKLKLDIQPYDINQRLQDTGGLIDSPSINLDVRKQYKRDIQNIDALLHQSIG
STLYNKIYLYENMNINNLTATLGADLVDSTDNTKINRGIFNEFKKNFKYSISSNYMIVDINERPALDNERLKWRIQLSPD
TRAGYLENGKLILQRNIGLEIKDVQIIKQSEKEYIRIDAKVVPKSKIDTKIQEAQLNINQEWNKALGLPKYTKLITFNVH
NRYASNIVESAYLILNEWKNNIQSDLIKKVTNYLVDGNGRFVFTDITLPNIAEQYTHQDEIYEQVHSKGLYVPESRSILL
HGPSKGVELRNDSEGFIHEFGHAVDDYAGYLLDKNQSDLVTNSKKFIDIFKEEGSNLTSYGRTNEAEFFAEAFRLMHSTD

HAERLKVQKNAPKTFQFINDQIKFIINSUMLARRKPVLPALTINP
```

## FIG. 34

## FIG. 35

```
>1BTH_H        254 AMINO ACIDS
I V E G S D A E I G M S P W Q V M L F R K S P Q E L L C G A S L I S D R
W V L T A A H C L L Y P P W D K N F T E N D L L V R I G K H S R T R Y E
R N I E K I S M L E K I Y I H P R Y N W R E N L D R D I A L M K L K K P
V A F S D Y I H P V C L P D R E T A A S L L Q A G Y K G R V T G W G N L
K E T W T T N V G K G Q P S V L Q V V N L P I V E R P V C K D S T R I R
I T D N M F C A G Y K P D E G K R G D A C Q G D S G G P F V M K S P F N
N R W Y Q M G I V S W G E G C D R D G K Y G F Y T H V F R L K K W I Q K
V I
>1BTH_P        58 AMINO ACIDS
R P D F C L E P P Y T G P C K A R I I R Y F Y N A K A G L C Q T F V Y G
G C R A K R N N F K S A E D C M R T C G G A
```

## FIG. 36

```
>2PTC_E        223 AMINO ACIDS
 I V G G Y T C G A N T V P Y Q V S L N S G Y H F C G G S L I N S Q W V V
S A A H C Y K S G I Q V R L G E D N I N V V E G N E Q F I S A S K S I V
H P S Y N S N T L N N D I M L I K L K S A A S L N S R V A S I S L P T S
C A S A G T Q C L I S G W G N T K S S G T S Y P D V L K C L K A P I L S
D S S C K S A Y P G Q I T S N M F C A G Y L E G G K D S C Q G D S G G P
V V C S G K L Q G I V S W G S G C A Q K N K P G V Y T K V C N Y V S W I
K Q T I A S N
>2PTC_I        58 AMINO ACIDS
R P D F C L E P P Y T G P C K A R I I R Y F Y N A K A G L C Q T F V Y G
G C R A K R N N F K S A E D C M R T C G G A
```

## FIG. 37

```
>1BTH_H      254   homology    match   mismatch   insertion   deletion
>2PTC_E      223   43.5%        97       123          3          34


IVEGSDAEIGMSPWQVMLFRKSPQELLCGASLISDRWVLTAAHCLLYPPWDKNFTENDLLVRIGKHSRTRYERNIEKISM
IVGGYTCGANTVPYQVSLN--S-GYHFCGGSLINSQWVVSAAHC--YKSG-----IQ---VRLGEDNINVVEGNEQFISA
**+*++++++++++#+*+*+*+++++++++*+++++++++*+++++++*++*++++++++++++++++*+*+++++++*+++++*++


LEKIYIHPRYNWRENLDRDIALMKLKKPVAFSDYIHPVCLPDRETAASLLQAGYKGRVTGWGNLKETWTTNVGKGQPSVL
SKSI-VHPSYNSNT-LNNDIMLIKLKSAASLNSRVASISLPT--SCAS---AGTQCLISGWGNTKSS-----GTSYPDVL
+++*+++*+*++++++*+*++*+++++*+*++++++++++++*+*++++*++++*++*+++++++++++*++++++++*+++*++


QVVNLPIVERPVCKDSTRIRITDNMFCAGYKPDEGKRGDACQGDSGGPFVMKSPFNNRWYQMGIVSWGEGCDRDGKYGFY
KCLKAPILSDSSCKSAYPGQITSNMFCAGYL-EGGK--DSCQGDSGGPVVC-S----GKLQ-GIVSWGSGCAQKNKPGVY
+++++*++++++++*++++++++++++++*+++++++++*+++++*++++*+*+++++++++++*+++++++++*++++*+*+*+


THVFRLKKWIQKVI---
TKVCNYVSWIKQTIASN
++++++++++*++++*++++
```

## FIG. 38

| >1BTH_P | 58 | HOMOLOGY | MATCH | MISMATCH | INSERTION | DELETION |
|---|---|---|---|---|---|---|
| >2PTC_I | 58 | 100.0% | 58 | 0 | 0 | 0 |

RPDFCLEPPYTGPCKARIIRYFYNAKAGLCQTFVYGGCRAKRNNFKSAEDCMRTCGGA
RPDFCLEPPYTGPCKARIIRYFYNAKAGLCQTFVYGGCRAKRNNFKSAEDCMRTCGGA
**********************************************************

## FIG. 39

For Enzyme Region.
>1BTH_H
IVEGSDAEIGMSPWQVMLFRKSPQELLCGASLISDRWVLTAAHCLLYPPWDKNFTENDLLVRIGKHSRTRYERNIEKISM
LEKIYIHPRYNWRENLDRDIALMKLKKPVAFSDYIHPVCLPDRETAASLLQAGYKGRVTGWGNLKETWTTNVGKGQPSVL
QVVNLPIVERPVCKDSTRIRITDNMFCAGYKPDEGKRGDACQGDSGGPFVMKSPFNNRWYQMGIVSWGEGCDRDGKYGFY
THVFRLKKWIQKVI
>2PTC_E
IVGGYTCGANTVPYQVSLN--S-GYHFCGGSLINSQWVVSAAHC--YKSG-----IQ---VRLGEDNINVVEGNEQFISA
SKSI-VHPSYNSNT-LNNDIMLIKLKSAASLNSRVASISLPT--SCAS---AGTQCLISGWGNTKSS-----GTSYPDVL
KCLKAPILSDSSCKSAYPGQITSNMFCAGYL-EGGK--DSCQGDSGGPVVC-S----GKLQ-GIVSWGSGCAQKNKPGVY
TKVCNYVSWIKQTI


For Inhibitor Protein,
>1BTH_P
RPDFCLEPPYTGPCKARIIRYFYNAKAGLCQTFVYGGCRAKRNNFKSAEDCMRTCGGA
>2PTC_I
RPDFCLEPPYTGPCKARIIRYFYNAKAGLCQTFVYGGCRAKRNNFKSAEDCMRTCGGA

## FIG. 40

>1BTH
IVEGSDAEIGMSPWQVMLFRKSPQELLCGASLISDRWVLTAAHCLLYPPWDKNFTENDLLVRIGKHSRTRYERNIEKISM
LEKIYIHPRYNWRENLDRDIALMKLKKPVAFSDYIHPVCLPDRETAASLLQAGYKGRVTGWGNLKETWTTNVGKGQPSVL
QVVNLPIVERPVCKDSTRIRITDNMFCAGYKPDEGKRGDACQGDSGGPFVMKSPFNNRWYQMGIVSWGEGCDRDGKYGFY
THVFRLKKWIQKVIURPDFCLEPPYTGPCKARIIRYFYNAKAGLCQTFVYGGCRAKRNNFKSAEDCMRTCGGA
>2PTC
IVGGYTCGANTVPYQVSLN--S-GYHFCGGSLINSQWVVSAAHC--YKSG-----IQ---VRLGEDNINVVEGNEQFISA
SKSI-VHPSYNSNT-LNNDIMLIKLKSAASLNSRVASISLPT--SCAS---AGTQCLISGWGNTKSS-----GTSYPDVL
KCLKAPILSDSSCKSAYPGQITSNMFCAGYL-EGGK--DSCQGDSGGPVVC-S----GKLQ-GIVSWGSGCAQKNKPGVY
TKVCNYVSWIKQTIURPDFCLEPPYTGPCKARIIRYFYNAKAGLCQTFVYGGCRAKRNNFKSAEDCMRTCGGA

FIG. 41

FIG. 42

FIG. 43

## Modling 41 Species

Clicking 'Search' button invokes 'AND' search for ORFs satisfying six conditions below

Order by [Gene Name ▼]    ⊙ asc    ○ desc

### 1. Search for ORFs by Species Code

Code is based on GTOP/IMG => <u>Old GTOP's Page</u>

Species :
☐ aero ☐ aful ☐ aqua ☐ bbur ☐ bhal ☐ bsub ☐ buch ☐ cele
☐ cjej ☐ cpneu ☐ ctra ☐ ctraM ☐ dmel ☐ drad ☑ ecoli
☑ ecoli_O157 ☐ hbsp ☐ hinf ☐ hpyl ☐ huge ☐ llact ☐ mgen
☐ mjan ☐ mpneu ☐ mthe ☐ mtub ☐ nmen ☐ pabys ☐ paer
☐ pmul ☐ pyro ☐ rpxx ☐ syne ☐ t4 ☐ tacid ☐ tmar ☐ tpal
☐ uure ☐ vcho ☐ xfas ☐ yst

### 2. Search for ORFs by Gene(ORF) Name

Gene Name: [                    ] (ex.yitB)

### 3. Search for ORFs by PDB ID of Reference Protein

PDB ID: [                    ] (ex.1RKD 1POD)

### 4. Search for ORFs by Motif Name

Keywords: [                    ] (Unavailable Now)

### 5. Search for ORFs by Keywords in ORF Product

Keywords: [toxin              ] (ex.RNA factor)

### 6. Search for ORFs by FAMS Results

○ Search for ORFs with 3D structures produced by FAMS.
○ Search for ORFs without 3D structures produced by FAMS.
⊙ both

FIG. 44

a

b

c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0135316 A
- US 5884230 A
- JP 2002112782 A
- JP 2002002859 A

### Non-patent literature cited in the description

- **YONEDA T ; KOMOOKA H ; UMEYAMA H.** *JOURNAL OF PROTEIN CHEMISTRY,* 1997, vol. 16, #6, 597-605
- **KAWABATA T et al.** NUCLEIC ACIDS RESEARCH. OXFORD UNIVERSITY PRESS, 01 January 2002, vol. 30, #1, 294-298
- **KIHARA D et al.** *PROCEEDINGS OF THE NATIONAL ACADEMY OF SCIENCES OF USA, NATIONAL ACADEMY OF SCIENCE,* vol. 98, #18, 10125-10130
- **KOLINSKI A et al.** PROTEINS: STRUCTURE, FUNCTION AND GENETICS. John Wiley & Sons, Inc, vol. 44, #2, 133-149
- **Gerardo Jimenez-Sanchez.** *Nature,* 2001, vol. 409, 853-855
- **T. Yoneda ; H. Komooka ; H. Umeyama.** *J. Protein Chem.,* 1997, vol. 16, 597-605
- **Pearson WR.** *Methods Enzymol,* 1996, vol. 266, 227-258
- **Schaffer AA ; Wolf YI ; Ponting CP ; Koonin EV ; Aravind L ; Altschul SF.** *Bioinformatics,* 1999, vol. 12, 1000-1011
- **Ota, M. ; Nishikawa, K.** *Protein Engineering,* 1997, vol. 10, 339-351
- **K. Ogata ; H. Umeyama.** *Journal of Molecular Graphics and Modeling,* 2000, vol. 18, 258-272
- **M. Takeda-Shitaka ; H. Umeyama.** *FEBS Letters,* 1998, vol. 425, 448-452
- *Protein Engineering,* 1989, vol. 2 (5), 347-351
- **Pearson W R.** Effective protein sequence comparison. *Methods Enzymol,* 1996, vol. 266, 227-58
- **Schaffer AA ; Wolf YI ; Ponting CP ; Koonin EV ; Aravind L ; Altschul SF.** Matching a protein sequence against a collection of PSI-BLAST-constructed position-specific score matrices. *Bioinformatics,* 1999, vol. 12, 1000-11
- **A.A. Schaffe et al.** *Bioinformatics,* 1999, vol. 15 (12), 1000-1011
- **A.A. Schaffe et al.** *Bioinformatics,* 1999, vol. 15 (12), 1000-1011
- **Koji Ogata.** *doctoral dissertation,* 1999
- **Ogata K ; Umeyama H.** The role of played by environmental residues in side-chain torsional angles within homologous families of proteins: A new method of side-chain modeling. *Prot. Struct. Funct. Genet.,* 1998, vol. 31, 255-369
- **Ogata K ; Umeyama H.** *Prot. Struct. Funct. Genet.,* 1998, vol. 31, 255-369
- **Kunishima, N. ; Shimada, Y. ; Tsuji, Y. ; Sato, T. ; Yamamoto, M. ; Kumasaka, T. ; Nakanishi, S. ; Jingami, H. ; Morikawa, K.** Structural Basis of Glutamate Recognition by a Dimeric Metabotropic Glutamate Receptor. *Nature,* 2000, vol. 407, 971
- **Abe T. ; Sugihara H. ; Nawa H. ; Shigemoto R. ; Mizuno N. ; Nakanishi S.** Molecular characterization of a novel metabotropic glutamate receptor mGluR5 coupled to inositol phosphate/Ca2+ signal transduction. *J. Biol. Chem.,* 1992, vol. 267, 13361-13368
- **Geoffrey Vhang ; Chistopher B. Roth.** *SCIENCE,* vol. 293, 1793
- **T. Yoneda ; H. Komooka ; H. Umeyama.** *J. Prot. Chem.,* 1997, vol. 16, 597-605
- **A. A. Schaffe et al.** *BIOINFORMATICS,* 1999, vol. 15 (12), 1000-1011
- **Edelmann H.M.L. ; Duchek P. ; Rosenthal F.E. ; Foeger N. ; Glackin C. ; Kane S.E. ; Kuchler K.** Cmdrl, a chicken P-glycoprotein, confers multidrug resistance and interacts with Estradiol. *Biol. Chem.,* 1999, vol. 380, 231-241
- **Pannifer et al.** *NATURE,* November 2001, vol. 414, 229-233
- **Marquart, M. ; Walter, J. ; Deisenhofer, J. ; Bode, W. ; Huber, R.** The Geometry of the Reactive Site and of the Peptide Groups in Trypsin, Trypsinogen and its Complexes with Inhibitors. *Acta Crystallogr., Sect. B,* 1983, vol. 39, 480
- **van de Locht, A. ; Bode, W. ; Huber, R. ; Le Bonniec, B. F. ; Stone, S. R. ; Esmon, C. T. ; Stubbs, M. T.** The thrombin E192Q-BPTI complex reveals gross structural rearrangements: implications for the interaction with antithrombin and thrombomodulin. *EMBO J.,* 1997, vol. 16, 2977

SEQUENCE LISTING

<110> UMEYAMA. Hideaki
Ajinomoto Co.. Inc.

<120> Method for Constructing Three-dimensional Structure of Protein Having Plural Chains

32

<130> P2722PCT-AJ

<140>
<141>

<150> JP 2002-002859
<151> 2002-01-09

<160> 13

<170> Patent In Ver. 2.1

<210> 1
<211> 1199
<212> PRT
<213> Rattus norvegicus

<4100> 1

```
Met Val Arg Leu Leu Leu Ile Phe Phe Pro Met Ile Phe Leu Glu Met
 1               5                  10                  15

Ser Ile Leu Pro Arg Met Pro Asp Arg Lys Val Leu Leu Ala Gly Ala
            20                  25                  30

Ser Ser Gln Arg Ser Val Ala Arg Met Asp Gly Asp Val Ile Ile Gly
            35                  40                  45
```

Ala Leu Phe Ser Val His His Gln Pro Pro Ala Glu Lys Val Pro Glu
50                    55                    60

Arg Lys Cys Gly Glu Ile Arg Glu Gln Tyr Gly Ile Gln Arg Val Glu
65                    70                    75                    80

Ala Met Phe His Thr Leu Asp Lys Ile Asn Ala Asp Pro Val Leu Leu
                 85                    90                    95

Pro Asn Ile Thr Leu Gly Ser Glu Ile Arg Asp Ser Cys Trp His Ser
                 100                   105                   110

Ser Val Ala Leu Glu Gln Ser Ile Glu Phe Ile Arg Asp Ser Leu Ile
              115                   120                   125

Ser Ile Arg Asp Glu Lys Asp Gly Leu Asn Arg Cys Leu Pro Asp Gly
              130                   135                   140

Gln Thr Leu Pro Pro Gly Arg Thr Lys Lys Pro Ile Ala Gly Val Ile
145                   150                   155                   160

Gly Pro Gly Ser Ser Ser Val Ala Ile Gln Val Gln Asn Leu Leu Gln
                 165                   170                   175

Leu Phe Asp Ile Pro Gln Ile Ala Tyr Ser Ala Thr Ser Ile Asp Leu
                 180                   185                   190

Ser Asp Lys Thr Leu Tyr Lys Tyr Phe Leu Arg Val Val Pro Ser Asp
              195                   200                   205

Thr Leu Gln Ala Arg Ala Met Leu Asp Ile Val Lys Arg Tyr Asn Trp
210                   215                   220

Thr Tyr Val Ser Ala Val His Thr Glu Gly Asn Tyr Gly Glu Ser Gly
225                   230                   235                   240

Met Asp Ala Phe Lys Glu Leu Ala Ala Gln Glu Gly Leu Cys Ile Ala
                 245                   250                   255

His Ser Asp Lys Ile Tyr Ser Asn Ala Gly Glu Lys Ser Phe Asp Arg
              260                   265                   270

Leu Leu Arg Lys Leu Arg Glu Arg Leu Pro Lys Ala Arg Val Val Val
              275                   280                   285

Cys Phe Cys Glu Gly Met Thr Val Arg Gly Leu Leu Ser Ala Met Arg
              290                   295                   300

Arg Leu Gly Val Val Gly Glu Phe Ser Leu Ile Gly Ser Asp Gly Trp
305                   310                   315                   320

Ala Asp Arg Asp Glu Val Ile Glu Gly Tyr Glu Val Glu Ala Asn Gly
325 330 335

Gly Ile Thr Ile Lys Leu Gln Ser Pro Glu Val Arg Ser Phe Asp Asp
340 345 350

Tyr Phe Leu Lys Leu Arg Leu Asp Thr Asn Thr Arg Asn Pro Trp Phe
355 360 365

Pro Glu Phe Trp Gln His Arg Phe Gln Cys Arg Leu Pro Gly His Leu
370 375 380

Leu Glu Asn Pro Asn Phe Lys Lys Val Cys Thr Gly Asn Glu Ser Leu
385 390 395 400

Glu Glu Asn Tyr Val Gln Asp Ser Lys Met Gly Phe Val Ile Asn Ala
405 410 415

Ile Tyr Ala Met Ala His Gly Leu Gln Asn Met His His Ala Leu Cys
420 425 430

Pro Gly His Val Gly Leu Cys Asp Ala Met Lys Pro Ile Asp Gly Arg
435 440 445

Lys Leu Leu Asp Phe Leu Ile Lys Ser Ser Phe Val Gly Val Ser Gly
450 455 460

Glu Glu Val Trp Phe Asp Glu Lys Gly Asp Ala Pro Gly Arg Tyr Asp
465 470 475 480

Ile Met Asn Leu Gln Tyr Thr Glu Ala Asn Arg Tyr Asp Tyr Val His
485 490 495

Val Gly Thr Trp His Glu Gly Val Leu Asn Ile Asp Asp Tyr Lys Ile
500 505 510

Gln Met Asn Lys Ser Gly Met Val Arg Ser Val Cys Ser Glu Pro Cys
515 520 525

Leu Lys Gly Gln Ile Lys Val Ile Arg Lys Gly Glu Val Ser Cys Cys
530 535 540

Trp Ile Cys Thr Ala Cys Lys Glu Asn Glu Phe Val Gln Asp Glu Phe
545 550 555 560

Thr Cys Arg Ala Cys Asp Leu Gly Trp Trp Pro Asn Ala Glu Leu Thr
565 570 575

Gly Cys Glu Pro Ile Pro Val Arg Tyr Leu Glu Trp Ser Asp Ile Glu

580                          585                          590

Ser Ile Ile Ala Ile Ala Phe Ser Cys Leu Gly Ile Leu Val Thr Leu
595                          600                          605

Phe Val Thr Leu Ile Phe Val Leu Tyr Arg Asp Thr Pro Val Val Lys
610                          615                          620

Ser Ser Ser Arg Glu Leu Cys Tyr Ile Ile Leu Ala Gly Ile Phe Leu
625                          630                          635                          640

Gly Tyr Val Cys Pro Phe Thr Leu Ile Ala Lys Pro Thr Thr Thr Ser
645                          650                          655

Cys Tyr Leu Gln Arg Leu Leu Val Gly Leu Ser Ser Ala Met Cys Tyr
660                          665                          670

Ser Ala Leu Val Thr Lys Thr Asn Arg Ile Ala Arg Ile Leu Ala Gly
675                          680                          685

Ser Lys Lys Lys Ile Cys Thr Arg Lys Pro Arg Phe Met Ser Ala Trp
690                          695                          700

Ala Gln Val Ile Ile Ala Ser Ile Leu Ile Ser Val Gln Leu Thr Leu
705                          710                          715                          720

Val Val Thr Leu Ile Ile Met Glu Pro Pro Met Pro Ile Leu Ser Tyr
725                          730                          735

Pro Ser Ile Lys Glu Val Tyr Leu Ile Cys Asn Thr Ser Asn Leu Gly
740                          745                          750

Val Val Ala Pro Val Gly Tyr Asn Gly Leu Leu Ile Met Ser Cys Thr
755                          760                          765

Tyr Tyr Ala Phe Lys Thr Arg Asn Val Pro Ala Asn Phe Asn Glu Ala
770                          775                          780

Lys Tyr Ile Ala Phe Thr Met Tyr Thr Thr Cys Ile Ile Trp Leu Ala
785                          790                          795                          800

Phe Val Pro Ile Tyr Phe Gly Ser Asn Tyr Lys Ile Ile Thr Thr Cys
805                          810                          815

Phe Ala Val Ser Leu Ser Val Thr Val Ala Leu Gly Cys Met Phe Thr
820                          825                          830

Pro Lys Met Tyr Ile Ile Ile Ala Lys Pro Glu Arg Asn Val Arg Ser
835                          840                          845

Ala Phe Thr Thr Ser Asp Val Val Arg Met His Val Gly Asp Gly Lys
850                855                860

Leu Pro Cys Arg Ser Asn Thr Phe Leu Asn Ile Phe Arg Arg Lys Lys
865                870                875                880

Pro Gly Ala Gly Asn Ala Asn Ser Asn Gly Lys Ser Val Ser Trp Ser
885                890                895

Glu Pro Gly Gly Arg Gln Ala Pro Lys Gly Gln His Val Trp Gln Arg
900                905                910

Leu Ser Val His Val Lys Thr Asn Glu Thr Ala Cys Asn Gln Thr Ala
915                920                925

Val Ile Lys Pro Leu Thr Lys Ser Tyr Gln Gly Ser Gly Lys Ser Leu
930                935                940

Thr Phe Ser Asp Ala Ser Thr Lys Thr Leu Tyr Asn Val Glu Glu Glu
945                950                955                960

Asp Asn Thr Pro Ser Ala His Phe Ser Pro Pro Ser Ser Pro Ser Met
965                970                975

Val Val His Arg Arg Gly Pro Pro Val Ala Thr Thr Pro Pro Leu Pro
980                985                990

Pro His Leu Thr Ala Glu Glu Thr Pro Leu Phe Leu Ala Asp Ser Val
995                1000                1005

Ile Pro Lys Gly Leu Pro Pro Pro Leu Pro Gln Gln Gln Pro Gln Gln
1010                1015                1020

Pro Pro Pro Gln Gln Pro Pro Gln Gln Pro Lys Ser Leu Met Asp Gln
1025                1030                1035                1040

Leu Gln Gly Val Val Thr Asn Phe Gly Ser Gly Ile Pro Asp Phe His
1045                1050                1055

Ala Val Leu Ala Gly Pro Gly Thr Pro Gly Asn Ser Leu Arg Ser Leu
1060                1065                1070

Tyr Pro Pro Pro Pro Pro Pro Gln His Leu Gln Met Leu Pro Leu His
1075                1080                1085

Leu Ser Thr Phe Gln Glu Glu Ser Ile Ser Pro Pro Gly Glu Asp Ile
1090                1095                1100

Asp Asp Asp Ser Glu Arg Phe Lys Leu Leu Gln Glu Phe Val Tyr Glu
1105                1110                1115                1120

```
Arg Glu Gly Asn Thr Glu Glu Asp Glu Leu Glu Glu Glu Gln Asp Leu
                1125              1130              1135

Pro Thr Ala Ser Lys Leu Thr Pro Glu Asp Ser Pro Ala Leu Thr Pro
                1140              1145              1150

Pro Ser Pro Phe Arg Asp Ser Val Ala Ser Gly Ser Ser Val Pro Ser
                1155              1160              1165

Ser Pro Val Ser Glu Ser Val Leu Cys Thr Pro Pro Asn Val Thr Tyr
                1170              1175              1180

Ala Ser Val Ile Leu Arg Asp Tyr Lys Gln Ser Ser Ser Thr Leu
1185                1190              1195
```

<210> 2
<211> 1203
<212> PRT
<213> Rattus norvegicus

<400> 2

Met Val Leu Leu Leu Ile Leu Ser Val Leu Leu Leu Lys Glu Asp Val
1          5          10         15

Arg Gly Ser Ala Gln Ser Ser Glu Arg Arg Val Val Ala His Met Pro
20          25          30

Gly Asp Ile Ile Ile Gly Ala Leu Phe Ser Val His His Gln Pro Thr
35          40          45

Val Asp Lys Val His Glu Arg Lys Cys Gly Ala Val Arg Glu Gln Tyr
50          55          60

Gly Ile Gln Arg Val Glu Ala Met Leu His Thr Leu Glu Arg Ile Asn
65          70          75         80

Ser Asp Pro Thr Leu Leu Pro Asn Ile Thr Leu Gly Cys Glu Ile Arg
85          90          95

Asp Ser Cys Trp His Ser Ala Val Ala Leu Glu Gln Ser Ile Glu Phe
100          105          110

Ile Arg Asp Ser Leu Ile Ser Ser Glu Glu Glu Glu Gly Leu Val Arg
115          120          125

Cys Val Asp Gly Ser Ser Ser Phe Arg Ser Lys Lys Pro Ile Val Gly
130          135          140

Val Ile Gly Pro Gly Ser Ser Ser Val Ala Ile Gln Val Gln Asn Leu
145                 150                 155                 160

Leu Gln Leu Phe Asn Ile Pro Gln Ile Ala Tyr Ser Ala Thr Ser Met
                165                 170                 175

Asp Leu Ser Asp Lys Thr Leu Phe Lys Tyr Phe Met Arg Val Val Pro
                180                 185                 190

Ser Asp Ala Gln Gln Ala Arg Ala Met Val Asp Ile Val Lys Arg Tyr
        195                 200                 205

Asn Trp Thr Tyr Val Ser Ala Val His Thr Glu Gly Asn Tyr Gly Glu
    210                 215                 220

Ser Gly Met Glu Ala Phe Lys Asp Met Ser Ala Lys Glu Gly Ile Cys
225                 230                 235                 240

Ile Ala His Ser Tyr Lys Ile Tyr Ser Asn Ala Gly Glu Gln Ser Phe
                245                 250                 255

Asp Lys Leu Leu Lys Lys Leu Arg Ser His Leu Pro Lys Ala Arg Val
        260                 265                 270

Val Ala Cys Phe Cys Glu Gly Met Thr Val Arg Gly Leu Leu Met Ala
        275                 280                 285

Met Arg Arg Leu Gly Leu Ala Gly Glu Phe Leu Leu Leu Gly Ser Asp
290                 295                 300

Gly Trp Ala Asp Arg Tyr Asp Val Thr Asp Gly Tyr Gln Arg Glu Ala
305                 310                 315                 320

Val Gly Gly Ile Thr Ile Lys Leu Gln Ser Pro Asp Val Lys Trp Phe
                325                 330                 335

Asp Asp Tyr Tyr Leu Lys Leu Arg Pro Glu Thr Asn Leu Arg Asn Pro
                340                 345                 350

Trp Phe Gln Glu Phe Trp Gln His Arg Phe Gln Cys Arg Leu Glu Gly
        355                 360                 365

Phe Ala Gln Glu Asn Ser Lys Tyr Asn Lys Thr Cys Asn Ser Ser Leu
370                 375                 380

Thr Leu Arg Thr His His Val Gln Asp Ser Lys Met Gly Phe Val Ile
385                 390                 395                 400

Asn Ala Ile Tyr Ser Met Ala Tyr Gly Leu His Asn Met Gln Met Ser
                405                 410                 415

40

Leu Cys Pro Gly Tyr Ala Gly Leu Cys Asp Ala Met Lys Pro Ile Asp
420                     425             430

Gly Arg Lys Leu Leu Asp Ser Leu Met Lys Thr Asn Phe Thr Gly Val
435                     440             445

Ser Gly Asp Met Ile Leu Phe Asp Glu Asn Gly Asp Ser Pro Gly Arg
450                     455             460

Tyr Glu Ile Met Asn Phe Lys Glu Met Gly Lys Asp Tyr Phe Asp Tyr
465             470             475             480

Ile Asn Val Gly Ser Trp Asp Asn Gly Glu Leu Lys Met Asp Asp Asp
485             490                     495

Glu Val Trp Ser Lys Lys Asn Asn Ile Ile Arg Ser Val Cys Ser Glu
500             505             510

Pro Cys Glu Lys Gly Gln Ile Lys Val Ile Arg Lys Gly Glu Val Ser
515             520             525

Cys Cys Trp Thr Cys Thr Pro Cys Lys Glu Asn Glu Tyr Val Phe Asp
530             535             540

Glu Tyr Thr Cys Lys Ala Cys Gln Leu Gly Ser Trp Pro Thr Asp Asp
545             550             555             560

Leu Thr Gly Cys Asp Leu Ile Pro Val Gln Tyr Leu Arg Trp Gly Asp
565             570             575

Pro Glu Pro Ile Ala Ala Val Val Phe Ala Cys Leu Gly Leu Leu Ala
580             585             590

Thr Leu Phe Val Thr Val Ile Phe Ile Ile Tyr Arg Asp Thr Pro Val
595             600             605

Val Lys Ser Ser Ser Arg Glu Leu Cys Tyr Ile Ile Leu Ala Gly Ile
610             615             620

Cys Leu Gly Tyr Leu Cys Thr Phe Cys Leu Ile Ala Lys Pro Lys Gln
625             630             635             640

Ile Tyr Cys Tyr Leu Gln Arg Ile Gly Ile Gly Leu Ser Pro Ala Met
645             650             655

Ser Tyr Ser Ala Leu Val Thr Lys Thr Asn Arg Ile Ala Arg Ile Leu
660             665             670

Ala Gly Ser Lys Lys Lys Ile Cys Thr Lys Lys Pro Arg Phe Met Ser

675                          680                          685

Ala Cys Ala Gln Leu Val Ile Ala Phe Ile Leu Ile Cys Ile Gln Leu
690                     695                     700

Gly Ile Ile Val Ala Leu Phe Ile Met Glu Pro Pro Asp Ile Met His
705                     710                     715                     720

Asp Tyr Pro Ser Ile Arg Glu Val Tyr Leu Ile Cys Asn Thr Thr Asn
725                     730                     735

Leu Gly Val Val Thr Pro Leu Gly Tyr Asn Gly Leu Leu Ile Leu Ser
740                     745                     750

Cys Thr Phe Tyr Ala Phe Lys Thr Arg Asn Val Pro Ala Asn Phe Asn
755                     760                     765

Glu Ala Lys Tyr Ile Ala Phe Thr Met Tyr Thr Thr Cys Ile Ile Trp
770                     775                     780

Leu Ala Phe Val Pro Ile Tyr Phe Gly Ser Asn Tyr Lys Ile Ile Thr
785                     790                     795                     800

Met Cys Phe Ser Val Ser Leu Ser Ala Thr Val Ala Leu Gly Cys Met
805                     810                     815

Phe Val Pro Lys Val Tyr Ile Ile Leu Ala Lys Pro Glu Arg Asn Val
820                     825                     830

Arg Ser Ala Phe Thr Thr Ser Thr Val Val Arg Met His Val Gly Asp
835                     840                     845

Gly Lys Ser Ser Ser Ala Ala Ser Arg Ser Ser Leu Val Asn Leu
850                     855                     860

Trp Lys Arg Arg Gly Ser Ser Gly Glu Thr Leu Arg Tyr Lys Asp Arg
865                     870                     875                     880

Arg Leu Ala Gln His Lys Ser Glu Ile Glu Cys Phe Thr Pro Lys Gly
885                     890                     895

Ser Met Gly Asn Gly Gly Arg Ala Thr Met Ser Ser Ser Asn Gly Lys
900                     905                     910

Ser Val Thr Trp Ala Gln Asn Glu Lys Ser Thr Arg Gly Gln His Leu
915                     920                     925

Trp Gln Arg Leu Ser Val His Ile Asn Lys Lys Glu Asn Pro Asn Gln
930                     935                     940

Thr Ala Val Ile Lys Pro Phe Pro Lys Ser Thr Glu Asn Arg Gly Pro
945              950              955              960

Gly Ala Ala Ala Gly Gly Gly Ser Gly Pro Gly Val Ala Gly Ala Gly
          965              970              975

Asn Ala Gly Cys Thr Ala Thr Gly Gly Pro Glu Pro Pro Asp Ala Gly
          980              985              990

Pro Lys Ala Leu Tyr Asp Val Ala Glu Ala Glu Glu Ser Phe Pro Ala
          995              1000             1005

Ala Ala Arg Pro Arg Ser Pro Ser Pro Ile Ser Thr Leu Ser His Leu
     1010             1015             1020

Ala Gly Ser Ala Gly Arg Thr Asp Asp Asp Ala Pro Ser Leu His Ser
1025             1030             1035             1040

Glu Thr Ala Ala Arg Ser Ser Ser Ser Gln Gly Ser Leu Met Glu Gln
          1045             1050             1055

Ile Ser Ser Val Val Thr Arg Phe Thr Ala Asn Ile Ser Glu Leu Asn
          1060             1065             1070

Ser Met Met Leu Ser Thr Ala Ala Thr Pro Gly Pro Pro Gly Thr Pro
          1075             1080             1085

Ile Cys Ser Ser Tyr Leu Ile Pro Lys Glu Ile Gln Leu Pro Thr Thr
     1090             1095             1100

Met Thr Thr Phe Ala Glu Ile Gln Pro Leu Pro Ala Ile Glu Val Thr
1105             1110             1115             1120

Gly Gly Ala Gln Gly Ala Thr Gly Val Ser Pro Ala Gln Glu Thr Pro
          1125             1130             1135

Thr Gly Ala Glu Ser Ala Pro Gly Lys Pro Asp Leu Glu Glu Leu Val
          1140             1145             1150

Ala Leu Thr Pro Pro Ser Pro Phe Arg Asp Ser Val Asp Ser Gly Ser
     1155             1160             1165

Thr Thr Pro Asn Ser Pro Val Ser Glu Ser Ala Leu Cys Ile Pro Ser
     1170             1175             1180

Ser Pro Lys Tyr Asp Thr Leu Ile Ile Arg Asp Tyr Thr Gln Ser Ser
1185             1190             1195             1200

Ser Ser Leu

<210> 3
<211> 474
<212> PRT
<213> Rattus norvegicus

<400> 3

```
Arg Arg Val Val Ala His Met Pro Gly Asp Ile Ile Ile Gly Ala Leu
1               5               10              15

Phe Ser Val His His Gln Pro Thr Val Asp Lys Val His Glu Arg Lys
        20              25              30

Cys Gly Ala Val Arg Glu Gln Tyr Gly Ile Gln Arg Val Glu Ala Met
        35              40              45

Leu His Thr Leu Glu Arg Ile Asn Ser Asp Pro Thr Leu Leu Pro Asn
        50              55              60

Ile Thr Leu Gly Cys Glu Ile Arg Asp Ser Cys Trp His Ser Ala Val
65              70              75              80

Ala Leu Glu Gln Ser Ile Glu Phe Ile Arg Asp Ser Leu Ile Ser Ser
                85              90              95

Glu Glu Glu Glu Gly Leu Val Arg Cys Val Asp Gly Ser Ser Ser Phe
                100             105             110

Arg Ser Lys Lys Pro Ile Val Gly Val Ile Gly Pro Gly Ser Ser Ser
        115             120             125

Val Ala Ile Gln Val Gln Asn Leu Leu Gln Leu Phe Asn Ile Pro Gln
130             135             140

Ile Ala Tyr Ser Ala Thr Ser Met Asp Leu Ser Asp Lys Thr Leu Phe
145             150             155             160

Lys Tyr Phe Met Arg Val Val Pro Ser Asp Ala Gln Gln Ala Arg Ala
                165             170             175

Met Val Asp Ile Val Lys Arg Tyr Asn Trp Thr Tyr Val Ser Ala Val
                180             185             190

His Thr Glu Gly Asn Tyr Gly Glu Ser Gly Met Glu Ala Phe Lys Asp
                195             200             205

Met Ser Ala Lys Glu Gly Ile Cys Ile Ala His Ser Tyr Lys Ile Tyr
210             215             220
```

Ser Asn Ala Gly Glu Gln Ser Phe Asp Lys Leu Leu Lys Lys Leu Arg
225 230 235 240

Ser His Leu Pro Lys Ala Arg Val Val Ala Cys Phe Cys Glu Gly Met
245 250 255

Thr Val Arg Gly Leu Leu Met Ala Met Arg Arg Leu Gly Leu Ala Gly
260 265 270

Glu Phe Leu Leu Leu Gly Ser Asp Gly Trp Ala Asp Arg Tyr Asp Val
275 280 285

Thr Asp Gly Tyr Gln Arg Glu Ala Val Gly Gly Ile Thr Ile Lys Leu
290 295 300

Gln Ser Pro Asp Val Lys Trp Phe Asp Asp Tyr Tyr Leu Lys Leu Arg
305 310 315 320

Pro Glu Thr Asn Leu Arg Asn Pro Trp Phe Gln Glu Phe Trp Gln His
325 330 335

Arg Phe Gln Cys Arg Leu Glu Gly Phe Ala Gln Glu Asn Ser Lys Tyr
340 345 350

Asn Lys Thr Cys Asn Ser Ser Leu Thr Leu Arg Thr His His Val Gln
355 360 365

Asp Ser Lys Met Gly Phe Val Ile Asn Ala Ile Tyr Ser Met Ala Tyr
370 375 380

Gly Leu His Asn Met Gln Met Ser Leu Cys Pro Gly Tyr Ala Gly Leu
385 390 395 400

Cys Asp Ala Met Lys Pro Ile Asp Gly Arg Lys Leu Leu Asp Ser Leu
405 410 415

Met Lys Thr Asn Phe Thr Gly Val Ser Gly Asp Met Ile Leu Phe Asp
420 425 430

Glu Asn Gly Asp Ser Pro Gly Arg Tyr Glu Ile Met Asn Phe Lys Glu
435 440 445

Met Gly Lys Asp Tyr Phe Asp Tyr Ile Asn Val Gly Ser Trp Asp Asn
450 455 460

Gly Glu Leu Lys Met Asp Asp Asp Glu Val
465 470

<210> 4

<211> 456
<212> PRT
<213> Rattus norvegicus

<400> 4

Gln Arg Ser Val Ala Arg Met Asp Gly Asp Val Ile Ile Gly Ala Leu
1               5                   10                  15

Phe Ser Val His His Gln Pro Pro Ala Glu Lys Val Pro Glu Arg Lys
            20                  25                  30

Cys Gly Glu Ile Arg Glu Gln Tyr Gly Ile Gln Arg Val Gln Ala Met
            35                  40                  45

Phe His Thr Leu Asp Lys Ile Asn Ala Asp Pro Val Leu Leu Pro Asn
        50                  55                  60

Ile Thr Leu Gly Ser Glu Ile Arg Asp Ser Cys Trp His Ser Ser Val
65                  70                  75                  80

Ala Leu Glu Gln Ser Ile Glu Phe Ile Arg Asp Ser Leu Ile Ser Ile
                85                  90                  95

Arg Lys Pro Ile Ala Gly Val Ile Gly Pro Gly Ser Ser Ser Val Ala
            100                 105                 110

Ile Gln Val Gln Asn Leu Leu Gln Leu Phe Asp Ile Pro Gln Ile Ala
        115                 120                 125

Tyr Ser Ala Thr Ser Ile Asp Leu Ser Asp Lys Thr Leu Tyr Lys Tyr
    130                 135                 140

Phe Leu Arg Val Val Pro Ser Asp Thr Leu Gln Ala Arg Ala Met Leu
145                 150                 155                 160

Asp Ile Val Lys Arg Tyr Asn Trp Thr Tyr Val Ser Ala Val His Thr
                165                 170                 175

Glu Gly Asn Tyr Gly Glu Ser Gly Met Asp Ala Phe Lys Glu Leu Ala
                180                 185                 190

Ala Gln Glu Gly Leu Cys Ile Ala His Ser Asp Lys Ile Tyr Ser Asn
        195                 200                 205

Ala Gly Glu Lys Ser Phe Asp Arg Leu Leu Arg Lys Leu Arg Glu Arg
    210                 215                 220

Leu Pro Lys Ala Arg Val Val Val Cys Phe Cys Glu Gly Met Thr Val
225                 230                 235                 240

Arg Gly Leu Leu Ser Ala Met Arg Arg Leu Gly Val Val Gly Glu Phe
245                     250                     255

Ser Leu Ile Gly Ser Asp Gly Trp Ala Asp Arg Asp Glu Val Ile Glu
260                     265                     270

Gly Tyr Glu Val Glu Ala Asn Gly Gly Ile Thr Ile Lys Leu Gln Ser
275                     280                     285

Pro Glu Val Arg Ser Phe Asp Asp Tyr Phe Leu Lys Leu Arg Leu Asp
290                     295                     300

Thr Asn Thr Arg Asn Pro Trp Phe Pro Glu Phe Trp Gln His Arg Phe
305                     310                     315                     320

Gln Cys Arg Leu Pro Gly His Leu Leu Glu Asn Pro Asn Phe Lys Lys
325                     330                     335

Val Cys Thr Gly Asn Glu Ser Leu Glu Glu Asn Tyr Val Gln Asp Ser
340                     345                     350

Lys Met Gly Phe Val Ile Asn Ala Ile Tyr Ala Met Ala His Gly Leu
355                     360                     365

Gln Asn Met His His Ala Leu Cys Pro Gly His Val Gly Leu Cys Asp
370                     375                     380

Ala Met Lys Pro Ile Asp Gly Arg Lys Leu Leu Asp Phe Leu Ile Lys
385                     390                     395                     400

Ser Ser Phe Val Gly Val Ser Gly Glu Glu Val Trp Phe Asp Glu Lys
405                     410                     415

Gly Asp Ala Pro Gly Arg Tyr Asp Ile Met Asn Leu Gln Tyr Thr Glu
420                     425                     430

Ala Asn Arg Tyr Asp Tyr Val His Val Gly Thr Trp His Glu Gly Val
435                     440                     445

Leu Asn Ile Asp Asp Tyr Lys Ile
450                     455

<210> 5
<211> 555
<212> PRT
<213> Escherichia coli

<400> 5

Trp Gln Thr Phe Arg Arg Leu Trp Pro Thr Ile Ala Pro Phe Lys Ala

|   | 1 |   |   |   | 5 |   |   |   |   | 10 |   |   |   |   | 15 |   |
|---|---|---|---|---|---|---|---|---|---|---|---|---|---|---|---|---|
| Gly | Leu | Ile | Val | Ala | Gly | Val | Ala | Leu | Ile | Leu | Asn | Ala | Ala | Ser | Asp |
|   |   |   | 20 |   |   |   | 25 |   |   |   | 30 |   |   |   |   |   |
| Thr | Phe | Met | Leu | Ser | Leu | Leu | Lys | Pro | Leu | Leu | Asp | Asp | Gly | Phe | Gly |
|   |   | 35 |   |   |   | 40 |   |   |   | 45 |   |   |   |   |   |   |
| Lys | Thr | Asp | Arg | Ser | Val | Leu | Val | Trp | Met | Pro | Leu | Val | Val | Ile | Gly |
|   | 50 |   |   |   | 55 |   |   |   | 60 |   |   |   |   |   |   |   |
| Leu | Met | Ile | Leu | Arg | Gly | Ile | Thr | Ser | Tyr | Val | Ser | Ser | Tyr | Cys | Ile |
| 65 |   |   |   | 70 |   |   |   | 75 |   |   |   | 80 |   |   |   |   |
| Ser | Trp | Val | Ser | Gly | Lys | Val | Val | Met | Thr | Met | Arg | Arg | Arg | Leu | Phe |
|   |   |   | 85 |   |   |   | 90 |   |   |   | 95 |   |   |   |   |   |
| Gly | His | Met | Met | Gly | Met | Pro | Val | Ser | Phe | Phe | Asp | Lys | Gln | Ser | Thr |
|   |   | 100 |   |   |   | 105 |   |   |   | 110 |   |   |   |   |   |   |
| Gly | Thr | Leu | Leu | Ser | Arg | Ile | Thr | Tyr | Asp | Ser | Glu | Gln | Val | Ala | Ser |
|   |   | 115 |   |   |   | 120 |   |   |   | 125 |   |   |   |   |   |   |
| Ser | Ser | Ser | Gly | Ala | Leu | Ile | Thr | Val | Val | Arg | Glu | Gly | Ala | Ser | Ile |
|   | 130 |   |   |   | 135 |   |   |   | 140 |   |   |   |   |   |   |   |
| Ile | Gly | Leu | Phe | Ile | Met | Met | Phe | Tyr | Tyr | Ser | Trp | Gln | Leu | Ser | Ile |
| 145 |   |   |   | 150 |   |   |   | 155 |   |   |   | 160 |   |   |   |   |
| Ile | Leu | Ile | Val | Leu | Ala | Pro | Ile | Val | Ser | Ile | Ala | Ile | Arg | Val | Val |
|   |   |   | 165 |   |   |   | 170 |   |   |   | 175 |   |   |   |   |   |
| Ser | Lys | Arg | Phe | Arg | Asn | Ile | Ser | Lys | Asn | Met | Gln | Asn | Thr | Met | Gly |
|   |   |   | 180 |   |   |   | 185 |   |   |   | 190 |   |   |   |   |   |
| Gln | Val | Thr | Thr | Ser | Ala | Glu | Gln | Met | Leu | Lys | Gly | His | Lys | Glu | Val |
|   |   | 195 |   |   |   | 200 |   |   |   | 205 |   |   |   |   |   |   |
| Leu | Ile | Phe | Gly | Gly | Gln | Glu | Val | Glu | Thr | Lys | Arg | Phe | Asp | Lys | Val |
|   | 210 |   |   |   | 215 |   |   |   | 220 |   |   |   |   |   |   |   |
| Ser | Asn | Arg | Met | Arg | Leu | Gln | Gly | Met | Lys | Met | Val | Ser | Ala | Ser | Ser |
| 225 |   |   |   | 230 |   |   |   | 235 |   |   |   | 240 |   |   |   |   |
| Ile | Ser | Asp | Pro | Ile | Ile | Gln | Leu | Ile | Ala | Ser | Leu | Ala | Leu | Ala | Phe |
|   |   |   | 245 |   |   |   | 250 |   |   |   | 255 |   |   |   |   |   |
| Val | Leu | Tyr | Ala | Ala | Ser | Phe | Pro | Ser | Val | Met | Asp | Ser | Leu | Thr | Ala |
|   |   |   | 260 |   |   |   | 265 |   |   |   | 270 |   |   |   |   |   |

Gly Thr Ile Thr Val Val Phe Ser Ser Met Ile Ala Leu Met Arg Pro
275 280 285

Leu Lys Ser Leu Thr Asn Val Asn Ala Gln Phe Gln Arg Gly Met Ala
290 , 295 300

Ala Cys Gln Thr Leu Phe Thr Ile Leu Asp Ser Glu Gln Glu Lys Asp
305 310 315 320

Glu Gly Lys Arg Val Ile Glu Arg Ala Thr Gly Asp Val Glu Phe Arg
325 330 335

Asn Val Thr Phe Thr Tyr Pro Gly Arg Asp Val Pro Ala Leu Arg Asn
340 345 350

Ile Asn Leu Lys Ile Pro Ala Gly Lys Thr Val Ala Leu Val Gly Arg
355 360 365

Ser Gly Ser Gly Lys Ser Thr Ile Ala Ser Leu Ile Thr Arg Phe Tyr
370 375 380

Asp Ile Asp Glu Gly Glu Ile Leu Met Asp Gly His Asp Leu Arg Glu
385 390 395 400

Tyr Thr Leu Ala Ser Leu Arg Asn Gln Val Ala Leu Val Ser Gln Asn
405 410 415

Val His Leu Phe Asn Asp Thr Val Ala Asn Asn Ile Ala Tyr Ala Arg
420 425 430

Thr Glu Gln Tyr Ser Arg Glu Gln Ile Glu Glu Ala Ala Arg Met Ala
435 440 445

Tyr Ala Met Asp Phe Ile Asn Lys Met Asp Asn Gly Leu Asp Thr Val
450 455 460

Ile Gly Glu Asn Gly Val Leu Leu Ser Gly Gly Gln Arg Gln Arg Ile
465 470 475 480

Ala Ile Ala Arg Ala Leu Leu Arg Asp Ser Pro Ile Leu Ile Leu Asp
485 490 495

Glu Ala Thr Ser Ala Leu Asp Thr Glu Ser Glu Arg Ala Ile Gln Ala
500 505 510

Ala Leu Asp Glu Leu Gln Lys Asn Arg Thr Ser Leu Val Ile Ala His
515 520 525

Arg Leu Ser Thr Ile Glu Lys Ala Asp Glu Ile Val Val Val Glu Asp
530 535 540

Gly Val Ile Val Glu Arg Gly Thr His Asn Asp
545　　　　　　　 550　　　　　　　　 555

<210> 6
<211> 573
<212> PRT
<213> Gallus gallus

<400> 6

Leu Phe Arg Tyr Ser Ser Cys Thr Asp Lys Leu Leu Met Ile Phe Gly
1　　　　　　 5　　　　　　　 10　　　　　　　　 15

Ser Leu Leu Ala Ile Ala His Gly Thr Ser Leu Pro Ile Ala Met Ile
　　　　　 20　　　　　　 25　　　　　　　 30

Ile Phe Gly Asp Met Thr Asp Ser Phe Val Thr Ser Gly Met Thr Asn
　　　　 35　　　　　　 40　　　　　　　 45

Ile Thr Gly Asn Ser Ser Gly Leu Asn Ser Ser Ala Asp Val Phe Asn
　　　 50　　　　　　 55　　　　　　　 60

Lys Leu Glu Glu Glu Met Thr Arg Tyr Ala Tyr Tyr Tyr Ser Ala Ile
65　　　　　　 70　　　　　　　 75　　　　　　　　 80

Ala Ala Ala Val Leu Val Ala Ala Tyr Ile Gln Thr Ser Phe Trp Thr
　　　　　　 85　　　　　　 90　　　　　　　 95

Leu Ala Ala Gly Arg Gln Val Lys Lys Ile Arg Glu Lys Phe Phe His
　　　　　 100　　　　　　 105　　　　　　　 110

Ala Ile Met Arg Gln Glu Ile Gly Trp Phe Asp Val Asn Asp Ala Gly
　　　　 115　　　　　　 120　　　　　　　 125

Glu Leu Asn Thr Arg Leu Ile Asp Asp Val Ser Lys Ile Asn Glu Gly
　　 130　　　　　　 135　　　　　　　 140

Ile Gly Asp Lys Ile Gly Phe Leu Ile Gln Ser Glu Thr Thr Phe Leu
145　　　　　　 150　　　　　　　 155　　　　　　　　 160

Thr Gly Phe Ile Val Gly Phe Ile Arg Gly Trp Lys Leu Thr Leu Val
　　　　　　 165　　　　　　 170　　　　　　　 175

Ile Leu Ala Val Ser Pro Val Leu Gly Leu Ser Ala Ala Leu Trp Ala
　　　　　 180　　　　　　 185　　　　　　　 190

Lys Ile Leu Thr Ala Phe Thr Asp Lys Glu Gln Ala Ala Tyr Ala Lys
　　　　 195　　　　　　 200　　　　　　　 205

51

```
Ala Gly Ala Val Ala Glu Glu Val Leu Ser Ala Val Arg Thr Val Ile
    210             215             220

Ala Phe Gly Gly Gln Glu Lys Glu Ile Lys Arg Tyr His Lys Asn Leu
225             230             235             240

Glu Asp Ala Lys Arg Ile Gly Ile Arg Lys Ala Ile Thr Ser Asn Ile
            245             250             255

Ser Met Gly Ala Ala Phe Leu Leu Ile Tyr Ala Ser Tyr Ala Leu Ala
            260             265             270

Phe Trp Tyr Gly Thr Thr Leu Ile Leu Ala Asn Glu Tyr Ser Ile Gly
            275             280             285

Asn Val Leu Thr Val Phe Phe Ser Val Leu Ile Gly Ala Phe Ser Ile
    290             295             300

Gly Gln Thr Ala Pro Ser Ile Glu Ala Phe Ala Asn Ala Arg Gly Ala
305             310             315             320

Ala Tyr Ala Ile Phe Asn Ile Ile Asp Asn Glu Pro Glu Ile Asp Ser
                325             330             335

Tyr Ser Asp Ala Gly His Lys Pro Asp His Ile Lys Gly Asn Leu Glu
            340             345             350

Phe Gln Asn Val Phe Phe Asn Tyr Pro Ser Arg Pro Asp Val Glu Ile
    355             360             365

Leu Lys Gly Leu Asn Leu Lys Val Asn Cys Gly Gln Thr Val Ala Leu
370             375             380

Val Gly Gly Ser Gly Cys Gly Lys Ser Thr Thr Val Gln Leu Ile Gln
385             390             395             400

Arg Phe Tyr Asp Pro Lys Glu Gly Thr Ile Thr Ile Asp Gly Gln Asp
            405             410             415

Leu Lys Ser Leu Asn Val Arg Tyr Leu Arg Glu Ile Ile Gly Val Val
            420             425             430

Asn Gln Glu Pro Val Leu Phe Ala Thr Thr Ile Ala Glu Asn Ile Arg
            435             440             445

Tyr Gly Arg Glu Asp Val Thr Met Glu Glu Ile Glu Arg Ala Thr Lys
    450             455             460

Glu Ala Asn Ala Tyr Asp Phe Ile Met Lys Leu Pro Lys Lys Phe Glu
465             470             475             480
```

Thr Val Val Gly Glu Arg Gly Ala Gln Met Ser Gly Gly Gln Lys Gln
485 490 495

Arg Ile Ala Ile Ala Arg Ala Leu Val His Asn Pro Lys Ile Leu Leu
500 505 510

Leu Asp Glu Ala Thr Ser Ala Leu Asp Thr Glu Ser Glu Ser Val Val
515 520 525

Gln Ala Ala Leu Asp Lys Ala Arg Glu Gly Arg Thr Thr Val Val Val
530 535 540

Ala His Arg Leu Ser Thr Val Arg Asn Ala Asp Leu Ile Ala Val Phe
545 550 555 560

Glu Ser Gly Val Ile Thr Glu Gln Gly Asn His Ser Gln
565 570

<210> 7
<211> 549
<212> PRT
<213> Escherichia coli

<400> 7

Leu Trp Pro Thr Ile Ala Pro Phe Lys Ala Gly Leu Ile Val Ala Gly
1                5                10               15

Val Ala Leu Ile Leu Asn Ala Ala Ser Asp Thr Phe Met Leu Ser Leu
              20            25            30

Leu Lys Pro Leu Leu Asp Asp Gly Phe Gly Lys Thr Asp Arg Ser Val
         35            40            45

Leu Val Trp Met Pro Leu Val Val Ile Gly Leu Met Ile Leu Arg Gly
      50            55            60

Ile Thr Ser Tyr Val Ser Ser Tyr Cys Ile Ser Trp Val Ser Gly Lys
65             70            75            80

Val Val Met Thr Met Arg Arg Arg Leu Phe Gly His Met Met Gly Met
         85            90            95

Pro Val Ser Phe Phe Asp Lys Gln Ser Thr Gly Thr Leu Leu Ser Arg
         100           105           110

Ile Thr Tyr Asp Ser Gln Gln Val Ala Ser Ser Ser Ser Gly Ala Leu
         115           120           125

```
Ile Thr Val Val Arg Glu Gly Ala Ser Ile Ile Gly Leu Phe Ile Met
    130              135               140

Met Phe Tyr Tyr Ser Trp Gln Leu Ser Ile Ile Leu Ile Val Leu Ala
145              150               155               160

Pro Ile Val Ser Ile Ala Ile Arg Val Val Ser Lys Arg Phe Arg Asn
                165               170               175

Ile Ser Lys Asn Met Gln Asn Thr Met Gly Gln Val Thr Thr Ser Ala
                180               185               190

Glu Gln Met Leu Lys Gly His Lys Glu Val Leu Ile Phe Gly Gly Gln
        195               200               205

Glu Val Glu Thr Lys Arg Phe Asp Lys Val Ser Asn Arg Met Arg Leu
    210               215               220

Gln Gly Met Lys Met Val Ser Ala Ser Ser Ile Ser Asp Pro Ile Ile
225               230               235               240

Gln Leu Ile Ala Ser Leu Ala Leu Ala Phe Val Leu Tyr Ala Ala Ser
                245               250               -   255

Phe Pro Ser Val Met Asp Ser Leu Thr Ala Gly Thr Ile Thr Val Val
                260               265               270

Phe Ser Ser Met Ile Ala Leu Met Arg Pro Leu Lys Ser Leu Thr Asn
                275               280               285

Val Asn Ala Gln Phe Gln Arg Gly Met Ala Ala Cys Gln Thr Leu Phe
    290               295               300

Thr Ile Leu Asp Ser Glu Gln Glu Lys Asp Glu Gly Lys Arg Val Ile
305               310               315               320

Glu Arg Ala Thr Gly Asp Val Glu Phe Arg Asn Val Thr Phe Thr Tyr
                325               330               335

Pro Gly Arg Asp Val Pro Ala Leu Arg Asn Ile Asn Leu Lys Ile Pro
                340               345               350

Ala Gly Lys Thr Val Ala Leu Val Gly Arg Ser Gly Ser Gly Lys Ser
                355               360               365

Thr Ile Ala Ser Leu Ile Thr Arg Phe Tyr Asp Ile Asp Glu Gly Glu
370               375               380

Ile Leu Met Asp Gly His Asp Leu Arg Glu Tyr Thr Leu Ala Ser Leu
385               390               395               400
```

```
Arg Asn Gln Val Ala Leu Val Ser Gln Asn Val His Leu Phe Asn Asp
                    405                 410                 415

Thr Val Ala Asn Asn Ile Ala Tyr Ala Arg Thr Glu Gln Tyr Ser Arg
                    420                 425                 430

Glu Gln Ile Glu Glu Ala Ala Arg Met Ala Tyr Ala Met Asp Phe Ile
            435                 440                 445

Asn Lys Met Asp Asn Gly Leu Asp Thr Val Ile Gly Gln Asn Gly Val
            450                 455                 460

Leu Leu Ser Gly Gly Gln Arg Gln Arg Ile Ala Ile Ala Arg Ala Leu
465                 470                 475                 480

Leu Arg Asp Ser Pro Ile Leu Ile Leu Asp Glu Ala Thr Ser Ala Leu
                    485                 490                 495

Asp Thr Glu Ser Glu Arg Ala Ile Gln Ala Ala Leu Asp Glu Leu Gln
            500                 505                 510

Lys Asn Arg Thr Ser Leu Val Ile Ala His Arg Leu Ser Thr Ile Glu
            515                 520                 525

Lys Ala Asp Glu Ile Val Val Val Glu Asp Gly Val Ile Val Glu Arg
            530                 535                 540

Gly Thr His Asn Asp
545
```

<210> 8
<211> 748
<212> PRT
<213> Bacillus anthracis

<400> 8

```
Asn Lys Thr Gln Glu Glu His Leu Lys Gln Ile Met Lys His Ile Val
 1                  5                   10                  15

Lys Ile Glu Val Lys Gly Glu Glu Ala Val Lys Lys Glu Ala Ala Glu
            20                  25                  30

Lys Leu Leu Glu Lys Val Pro Ser Asp Val Leu Glu Met Tyr Lys Ala
            35                  40                  45

Ile Gly Gly Lys Ile Tyr Ile Val Asp Gly Asp Ile Thr Lys His Ile
            50                  55                  60
```

Ser Leu Glu Ala Leu Ser Glu Asp Lys Lys Lys Ile Lys Asp Ile Tyr
65          70          75          80

Gly Lys Asp Ala Leu Leu His Glu His Tyr Val Tyr Ala Lys Glu Gly
              85          90          95

Tyr Glu Pro Val Leu Val Ile Gln Ser Ser Glu Asp Tyr Val Glu Asn
          100          105          110

Thr Glu Lys Ala Leu Asn Val Tyr Tyr Glu Ile Gly Lys Ile Leu Ser
          115          120          125

Arg Asp Ile Leu Ser Lys Ile Asn Gln Pro Tyr Gln Lys Phe Leu Asp
    130          135          140

Val Leu Asn Thr Ile Lys Asn Ala Ser Asp Ser Asp Gly Gln Asp Leu
145          150          155          160

Leu Phe Thr Asn Gln Leu Lys Glu His Pro Thr Asp Phe Ser Val Glu
          165          170          175

Phe Leu Glu Gln Asn Ser Asn Glu Val Gln Glu Val Phe Ala Lys Ala
          180          185          190

Phe Ala Tyr Tyr Ile Glu Pro Gln His Arg Asp Val Leu Gln Leu Tyr
          195          200          205

Ala Pro Glu Ala Phe Asn Tyr Met Asp Lys Phe Asn Glu Gln Glu Ile
    210          215          220

Asn Leu Ser Leu Glu Glu Leu Lys Asp Gln Arg Met Leu Ser Arg Tyr
225          230          235          240

Glu Lys Trp Glu Lys Ile Lys Gln His Tyr Gln His Trp Ser Asp Ser
              245          250          255

Leu Ser Glu Glu Gly Arg Gly Leu Leu Lys Lys Leu Gln Ile Pro Ile
          260          265          270

Glu Pro Lys Lys Asp Asp Ile Ile His Ser Leu Ser Gln Glu Glu Lys
    275          280          285

Glu Leu Leu Lys Arg Ile Gln Ile Asp Ser Ser Asp Phe Leu Ser Thr
    290          295          300

Glu Glu Lys Glu Phe Leu Lys Lys Leu Gln Ile Asp Ile Arg Asp Ser
305          310          315          320

Leu Ser Glu Glu Glu Lys Glu Leu Leu Asn Arg Ile Gln Val Asp Ser
              325          330          335

Ser Asn Pro Leu Ser Glu Lys Glu Lys Glu Phe Leu Lys Lys Leu Lys
340 345 350

Leu Asp Ile Gln Pro Tyr Asp Ile Asn Gln Arg Leu Gln Asp Thr Gly
355 360 365

Gly Leu Ile Asp Ser Pro Ser Ile Asn Leu Asp Val Arg Lys Gln Tyr
370 375 380

Lys Arg Asp Ile Gln Asn Ile Asp Ala Leu Leu His Gln Ser Ile Gly
385 390 395 400

Ser Thr Leu Tyr Asn Lys Ile Tyr Leu Tyr Glu Asn Met Asn Ile Asn
405 410 415

Asn Leu Thr Ala Thr Leu Gly Ala Asp Leu Val Asp Ser Thr Asp Asn
420 425 430

Thr Lys Ile Asn Arg Gly Ile Phe Asn Glu Phe Lys Lys Asn Phe Lys
435 440 445

Tyr Ser Ile Ser Ser Asn Tyr Met Ile Val Asp Ile Asn Glu Arg Pro
450 455 460

Ala Leu Asp Asn Glu Arg Leu Lys Trp Arg Ile Gln Leu Ser Pro Asp
465 470 475 480

Thr Arg Ala Gly Tyr Leu Glu Asn Gly Lys Leu Ile Leu Gln Arg Asn
485 490 495

Ile Gly Leu Glu Ile Lys Asp Val Gln Ile Ile Lys Gln Ser Glu Lys
500 505 510

Glu Tyr Ile Arg Ile Asp Ala Lys Val Val Pro Lys Ser Lys Ile Asp
515 520 525

Thr Lys Ile Gln Glu Ala Gln Leu Asn Ile Asn Gln Glu Trp Asn Lys
530 535 540

Ala Leu Gly Leu Pro Lys Tyr Thr Lys Leu Ile Thr Phe Asn Val His
545 550 555 560

Asn Arg Tyr Ala Ser Asn Ile Val Glu Ser Ala Tyr Leu Ile Leu Asn
565 570 575

Glu Trp Lys Asn Asn Ile Gln Ser Asp Leu Ile Lys Lys Val Thr Asn
580 585 590

Tyr Leu Val Asp Gly Asn Gly Arg Phe Val Phe Thr Asp Ile Thr Leu

```
                 595              600              605
        Pro Asn Ile Ala Glu Gln Tyr Thr His Gln Asp Glu Ile Tyr Glu Gln
            610              615              620

        Val His Ser Lys Gly Leu Tyr Val Pro Glu Ser Arg Ser Ile Leu Leu
        625              630              635              640

        His Gly Pro Ser Lys Gly Val Glu Leu Arg Asn Asp Ser Glu Gly Phe
                    645              650              655

        Ile His Glu Phe Gly His Ala Val Asp Asp Tyr Ala Gly Tyr Leu Leu
                    660              665              670

        Asp Lys Asn Gln Ser Asp Leu Val Thr Asn Ser Lys Lys Phe Ile Asp
                    675              680              685

        Ile Phe Lys Glu Glu Gly Ser Asn Leu Thr Ser Tyr Gly Arg Thr Asn
            690              695              700

        Glu Ala Glu Phe Phe Ala Glu Ala Phe Arg Leu Met His Ser Thr Asp
        705              710              715              720

        His Ala Glu Arg Leu Lys Val Gln Lys Asn Ala Pro Lys Thr Phe Gln
                    725              730              735

        Phe Ile Asn Asp Gln Ile Lys Phe Ile Ile Asn Ser
            740              745
```

<210> 9
<211> 16
<212> PRT
<213> Bacillus anthracis

<400> 9

```
        Met Leu Ala Arg Arg Lys Pro Val Leu Pro Ala Leu Thr Ile Asn Pro
            1               5               10              15
```

<210> 10
<211> 16
<212> PRT
<213> Bacillus anthracis

<400> 10

```
        Met Phe Phe Arg Arg Lys Pro Val Leu Pro Ala Leu Thr Ile Asn Pro
            1               5               10              15
```

<210> 11

<211> 254
<212> PRT
<211> Homo sapiens

<400> 11

Ile Val Glu Gly Ser Asp Ala Glu Ile Gly Met Ser Pro Trp Gln Val
1               5                   10                  15

Met Leu Phe Arg Lys Ser Pro Gln Glu Leu Leu Cys Gly Ala Ser Leu
            20                  25                  30

Ile Ser Asp Arg Trp Val Leu Thr Ala Ala His Cys Leu Leu Tyr Pro
        35                  40                  45

Pro Trp Asp Lys Asn Phe Thr Glu Asn Asp Leu Leu Val Arg Ile Gly
        50                  55                  60

Lys His Ser Arg Thr Arg Tyr Glu Arg Asn Ile Glu Lys Ile Ser Met
65                  70                  75                  80

Leu Glu Lys Ile Tyr Ile His Pro Arg Tyr Asn Trp Arg Glu Asn Leu
                85                  90                  95

Asp Arg Asp Ile Ala Leu Met Lys Leu Lys Lys Pro Val Ala Phe Ser
            100                 105                 110

Asp Tyr Ile His Pro Val Cys Leu Pro Asp Arg Glu Thr Ala Ala Ser
        115                 120                 125

Leu Leu Gln Ala Gly Tyr Lys Gly Arg Val Thr Gly Trp Gly Asn Leu
130                 135                 140

Lys Glu Thr Trp Thr Thr Asn Val Gly Lys Gly Gln Pro Ser Val Leu
145                 150                 155                 160

Gln Val Val Asn Leu Pro Ile Val Glu Arg Pro Val Cys Lys Asp Ser
                165                 170                 175

Thr Arg Ile Arg Ile Thr Asp Asn Met Phe Cys Ala Gly Tyr Lys Pro
            180                 185                 190

Asp Glu Gly Lys Arg Gly Asp Ala Cys Gln Gly Asp Ser Gly Gly Pro
            195                 200                 205

Phe Val Met Lys Ser Pro Phe Asn Asn Arg Trp Tyr Gln Met Gly Ile
210                 215                 220

Val Ser Trp Gly Glu Gly Cys Asp Arg Asp Gly Lys Tyr Gly Phe Tyr
225                 230                 235                 240

Thr His Val Phe Arg Leu Lys Lys Trp Ile Gln Lys Val Ile
245 250

<210> 12
<211> 58
<212> PRT
<213> Bos taurus

<400> 12

Arg Pro Asp Phe Cys Leu Glu Pro Pro Tyr Thr Gly Pro Cys Lys Ala
1 5 10 15

Arg Ile Ile Arg Tyr Phe Tyr Asn Ala Lys Ala Gly Leu Cys Gln Thr
20 25 30

Phe Val Tyr Gly Gly Cys Arg Ala Lys Arg Asn Asn Phe Lys Ser Ala
35 40 45

Glu Asp Cys Met Arg Thr Cys Gly Gly Ala
50 55

<210> 13
<211> 223
<212> PRT
<213> Bos taurus

<400> 13

Ile Val Gly Gly Tyr Thr Cys Gly Ala Asn Thr Val Pro Tyr Gln Val
1 5 10 15

Ser Leu Asn Ser Gly Tyr His Phe Cys Gly Gly Ser Leu Ile Asn Ser
20 25 30

Gln Trp Val Val Ser Ala Ala His Cys Tyr Lys Ser Gly Ile Gln Val
35 40 45

Arg Leu Gly Glu Asp Asn Ile Asn Val Val Glu Gly Asn Glu Gln Phe
50 55 60

Ile Ser Ala Ser Lys Ser Ile Val His Pro Ser Tyr Asn Ser Asn Thr
65 70 75 80

Leu Asn Asn Asp Ile Met Leu Ile Lys Leu Lys Ser Ala Ala Ser Leu
85 90 95

Asn Ser Arg Val Ala Ser Ile Ser Leu Pro Thr Ser Cys Ala Ser Ala
100 105 110

```
Gly Thr Gln Cys Leu Ile Ser Gly Trp Gly Asn Thr Lys Ser Ser Gly
        115                 120             125

Thr Ser Tyr Pro Asp Val Leu Lys Cys Leu Lys Ala Pro Ile Leu Ser
        130                 135             140

Asp Ser Ser Cys Lys Ser Ala Tyr Pro Gly Gln Ile Thr Ser Asn Met
    145             150             155                 160

Phe Cys Ala Gly Tyr Leu Glu Gly Gly Lys Asp Ser Cys Gln Gly Asp
            165             170                 175

Ser Gly Gly Pro Val Val Cys Ser Gly Lys Leu Gln Gly Ile Val Ser
        180                 185                 190

Trp Gly Ser Gly Cys Ala Gln Lys Asn Lys Pro Gly Val Tyr Thr Lys
        195                 200                 205

Val Cys Asn Tyr Val Ser Trp Ile Lys Gln Thr Ile Ala Ser Asn
    210                 215                 220
```